# EUROPÄISCHE PATENTANMELDUNG

(11) **EP 2 857 008 A1**
(43) Veröffentlichungstag der Anmeldung: **08.04.2015**
(21) Anmeldenummer: 14003528.8
(22) Anmeldetag: 19.08.2012
(51) Int. Cl.: A61K 31/045, A61K 31/137, A61K 31/35, A61K 31/4174, A61K 31/4178, A61K 31/505, A61K 31/573, A61K 31/592, A61K 31/593, A61K 31/728, A61K 31/606, A61K 31/164, A61P 1/00

(54) **Kombinationstherapeutikum**

(30) Priorität: 19.08.2011 DE 102011111111
(62) Teilanmeldung aus: 12753908.8
(71) Anmelder: Joy Development UG, 53859 Niederkassel (DE)
(72) Erfinder: Juergens, Anna Mai Joy, D - 53859 Niederkassel (DE); Juergens, Heike, D - 53859 Niederkassel (DE); Juergens, Lisa Joy, D - 53859 Niederkassel (DE)
(74) Vertreter: Von Rohr Patentanwälte Partnerschaft mbB

(57) **Zusammenfassung**

Die Erfindung betrifft ein Kombinationstherapeutikum, vorzugsweise zur Verwendung bei der prophylaktischen und/oder therapeutischen Behandlung von insbesondere entzündlichen Erkrankungen des Verdauungstraktes, insbesondere des Darms. Das Kombinationstherapeutikum umfasst mindestens einen Vitamin D-Rezeptoragonisten (VDA) und mindestens einen weiteren Wirkstoff, jedoch mit der Maßgabe, dass das Kombinationstherapeutikum keinen Inhalts- und/oder Wirkstoff aus der Gruppe von Terpenen, Monoterpenen, Vasokonstriktoren, alpha-Sympathomimetika und/oder deren physiologisch unbedenklichen Salzen oder Derivaten enthält.

## Beschreibung

Die vorliegende Erfindung betrifft das medizinische Gebiet der prophylaktischen bzw. therapeutischen Behandlung von insbesondere entzündlichen Atemwegserkrankungen einerseits und entzündlichen Erkrankungen des Verdauungstrakts andererseits.

Insbesondere betrifft die vorliegende Erfindung ein Kombinationstherapeutikum, welches sich insbesondere zur Verwendung bei der prophylaktischen bzw. therapeutischen Behandlung von insbesondere entzündlichen Atemwegserkrankungen, insbesondere bronchopulmonalen Erkrankungen, einerseits oder von insbesondere entzündlichen Erkrankungen des Verdauungstraktes, insbesondere des Darms, andererseits eignet.

Darüber hinaus betrifft die vorliegende Erfindung die Verwendungen des erfindungsgemäßen Kombinationstherapeutikums bzw. von Vitamin D-Rezeptoragonisten (VDA) in Kombination mit speziellen weiteren Wirkstoffen, insbesondere für die vorgenannten Anwendungen.

Unter dem Begriff "Atemwegserkrankungen", wie er auch im Rahmen der vorliegenden Erfindung verwendet wird, ist eine allgemeine Bezeichnung zu verstehen, welche sämtliche, insbesondere entzündliche Erkrankungen der oberen wie unteren Atemwege bezeichnet, wobei hierunter akute wie chronische Krankheitszustände zu subsumieren sind. Beispiele für Atemwegserkrankungen der oberen Atemwege sind z. B. Entzündungen der Nebenhöhlen (z. B. Rhinosinusitis), während Beispiele für Atemwegserkrankungen der unteren Atemwege z. B. Asthma bronchiale, Bronchitis und COPD sind.

Der Begriff "bronchopulmonale Erkrankungen", wie er gleichermaßen auch im Rahmen der vorliegenden Erfindung verwendet wird, ist eine generische Bezeichnung insbesondere für sämtliche entzündliche wie nichtentzündliche Erkrankungen der unteren Atemwege (d. h. der bronchialen wie pulmonalen Atemwege), hierunter insbesondere Asthma bronchiale, Bronchitis sowie chronische obstruktive Lungenerkrankungen (sogenannte "COPD" bzw. "Chronic Obstructive Pulmonary Disease"), und wird im Rahmen der vorliegenden Erfindung synonym zu dem Begriff der sogenannten "Atemwegserkrankungen der unter Atemwege" verwendet.

Asthma bronchiale, oft vereinfachend auch nur als Asthma bezeichnet, ist eine chronische entzündliche Erkrankung der Atemwege mit dauerhaft bestehender bronchialer Überempfindlichkeit bzw. Hyperreaktivität, Entzündung der Bronchien sowie mangelnder bronchialer Reinigung (Clearance), wobei infolge der Bronchialobstruktion anfallsweise Atemnot auftreten kann, wobei grundsätzlich zwischen nichtallergischem (intrinsischem) und allergischem (extrinsischem) Asthma differenziert wird; daneben kennt man sowohl Mischtypen von allergischem und nichtallergischem Asthma als auch Mischtypen von Asthma und COPD. Je nach Schweregrad und den damit einhergehenden Krankheitssymptomen klassifiziert man gemäß den sogenannten Leitlinien nach GINA 2006 ("Global Initiative for Asthma") die verschiedenen Asthma-Krankheitsstadien von GINA I bis IV, wobei die Stufe 1 intermittierendes Asthma, die Stufe 2 leichtgradiges Asthma, die Stufe 3 mittelgradiges Asthma und schließlich die Stufe 4 schweres Asthma bezeichnet. In der neuen Klassifikation (GINA 2007) wird dagegen die Asthmakontrolle in den Vordergrund gestellt, und es wird somit unterschieden zwischen kontrolliertem, partiell kontrolliertem und unkontrolliertem Asthma.

Als Bronchitis wird dagegen allgemein die Entzündung der Bronchien, insbesondere der Bronchialschleimhaut, bezeichnet, wobei zwischen akuter Bronchitis einerseits und chronischer Bronchitis andererseits unterschieden wird. Die chronische Bronchitis ist gemäß Weltgesundheitsorganisation (WHO) definiert als Husten und Auswurf an den meisten Tagen während mindestens drei Monaten in zwei aufeinanderfolgenden Jahren und gehört zu den häufigsten chronischen Erkrankungen überhaupt (ca. 15 bis 25 %) mit infolgedessen großer Relevanz aus gesundheitsökonomischer Sicht. Im Fall der chronisch obstruktiven Bronchitis besteht eine dauerhafte Bronchialobstruktion, die sich meistens aus einer chronischen Bronchitis entwickelt.

Der Begriff der chronischen obstruktiven Lungenerkrankung bzw. COPD ("*Chronic Obstructive Pulmonary Disease*"), wie er auch im Rahmen der vorliegenden Erfindung verwendet wird, stellt einen Sammelbegriff für die chronisch obstruktive Bronchitis und das Lungenemphysem dar, wobei der Begriff "obstruktiv" das typische Merkmal der dauerhaften Bronchialverengung charakterisiert. Dabei klassifiziert man je nach Schweregrad und den damit einhergehenden Krankheitssymptomen gemäß den sogenannten Leitlinien nach GOLD ("*Global Initiative for Chronic Obstructive Lung Disease*") die verschiedenen COPD-Krankheitsstadien von GOLD I bis IV.

Bei der Behandlung von bronchopulmonalen Erkrankungen, insbesondere von Atemwegserkrankungen der vorgenannten Art, kommen bei mildem bis mittlerem Schweregrad oftmals topische bzw. lokale, insbesondere inhalative bzw. inhalierbare Atemwegstherapeutika zum Einsatz. Hierzu zählen beispielsweise inhalative Bronchodilatatoren und Bronchospasmolytika, wie inhalative beta-2-Sympathomimetika, inhalative Anticholinergika, inhalative Kortikosteroide oder dergleichen. Infolge ihrer nur topischen bzw. lokalen Wirkung können die vorgenannten inhalativen Atemwegstherapeutika oftmals nicht die gewünschte therapeutische Wirkung entfalten, so dass zumeist relativ hohe Dosismengen verabreicht werden müssen, um den gewünschten therapeutischen Erfolg zu erzielen, oder aber in schwerwiegenderen Fällen systemische Therapeutika, insbesondere auf Kortikosteroidbasis, bedarfsweise oder sogar dauerhaft hinzugezogen werden müssen. Infolge der hohen einzusetzenden und erforderlichen Dosismengen werden zudem vielfach unerwünschte Nebenwirkungen beobachtet. Bisweilen wird auch eine nicht ausreichende Sensitivität der vorgenannten inhalativen Atemwegstherapeutika in Bezug auf die zu behandelnden Atemwegserkrankungen beobachtet. Schließlich ist mit einer rein topischen, insbesondere inhalativen Therapierung der vorgenannten Atemwegserkrankungen insbesondere der Nachteil verbunden, dass die Anwendung inhalativer Atemwegstherapeutika nicht immer zu einer ausreichenden Deposition in den peripheren Atemwegen führt, da kleinste Atemwege, wie beispielsweise die terminalen und respiratorischen Bronchioli, im Allgemeinen durch eine inhalative Therapie nicht erreicht werden können, insbesondere bei Einschränkungen in der Lungenfunktion, wie z. B. bei schwerer COPD.

So kommen beispielsweise sowohl beim Asthma bronchiale als auch bei der COPD - neben einer Grundtherapie mit sogenannten Bronchodilatatoren - ab einem gewissen Schweregrad auch topische bzw. lokale, insbesondere inhalative bzw. inhalierbare Kortikosteroide zum Einsatz. Daneben kommen bei schwerem Asthma bronchiale entsprechend GINA IV zusätzlich auch systemische, insbesondere perorale Kortikosteroide zum Einsatz.

Inhalierbare bzw. inhalative Kortikosteroide, insbesondere inhalierbare bzw, inhalative Glukokortikoide, welche synonym auch als "Inhalative Corticosteroids" oder einfach nur mit dem Akronym "ICS" bezeichnet werden, gehören zu den wichtigsten Therapeutika zur topischen bzw. lokalen, insbesondere inhalativen Behandlung entzündlicher Atemwegserkrankungen, insbesondere bei Asthma bronchiale und COPD. Das Wirkprinzip besteht bei regelmäßiger Inhalation in einer primär topischen bzw. lokalen Deposition in den Atemwegen, verbunden mit einer zugleich effektiven Entzündungshemmung durch relativ kleine Steroidmengen.

Auf lokaler bzw. topischer Ebene reduzieren inhalierbare Kortikosteroide, insbesondere Glukokortikoide, die Atemwegsentzündung durch die Hemmung von Zytokinen und Arachidonsäure-Metaboliten (AA-Metaboliten), welche aus aktivierten Atemwegsepithelzellen und distalen, die Atemwege auskleidenden, sogenannten alveolaren Makrophagen freigesetzt werden. In Abhängigkeit von der inhalierten Noxe und der genetischen Disposition gelangen durch die verschiedenen, vorgenannten chemotaktisch und vasodilatierend wirkenden Mediatoren unterschiedliche weiße Blutzellen in die Atemwege und verursachen eine entweder eosinophile Zellinfiltration im Fall des Asthma bronchiale oder eine primär granulozytäre Zellinfiltration im Fall der COPD. Diese Zellinfiltration ist als wichtige Determinante zur Entwicklung der Atemwegsentzündung bei Asthma bronchiale und der Bronchitis bekannt.

Nach den aktuellen internationalen und nationalen Therapierichtlinien werden jedoch ICS nicht in den Frühstadien der Atemwegserkrankungen eingesetzt, sondern insbesondere erst bei mildem persistierendem Asthma (GINA II) und bei mittel- bis schwergradiger COPD (GOLD III und IV), wobei zumeist eine dauerhafte Therapie erforderlich ist. Mit dieser anerkannten Therapiestrategie werden die zunehmend bekannten Nebenwirkungen von ICS sowie eine nicht ausreichende Sensitivität von ICS bei COPD berücksichtigt. Aus diesem Grunde bleibt die Therapie mit ICS nur der mittel- und schwergradigen COPD vorbehalten, die jedoch nicht den Progress bei COPD, sondern nur die Abnahme von Exazerbationen beeinflussen kann.

Naturgemäß ist für den klinischen Erfolg einer topischen Therapie mit inhalativen Kortikosteroiden, insbesondere Glukokortikoiden, der Grad der Deposition und insbesondere auch die Steroidverteilung in den peripheren Atemwegen von direkter Bedeutung. Aber selbst bei optimalem Einsatz verschiedenster Atemhilfen für Dosieraerosole und Pulverpräparate ist der Therapieerfolg nicht nur durch die Fähigkeit des Patienten, optimal zu inhalieren, sondern vielmehr primär durch das Inhalationsprinzip aufgrund der nicht ausreichenden Behandlung peripherer Atemwege limitiert. Wesentliche Ursachen hierfür sind somit die nicht ausreichende Steroiddeposition in den kleinen Atemwegen (<10⁻⁸ mol/l) und die entsprechend höhere Steroiddeposition auf den Schleimhäuten des Mundes und der Luftröhre, Nach Resorption gelangen hierdurch regelmäßig kleinere Steroidmengen unerwünschtermaßen auch in die Blutbahn und verursachen typische Steroidnebenwirkungen, wie z- B. Hemmung der Kortisolproduktion, Entwicklung einer Osteoporose, Kataraktbildung etc. Diese zunehmend bekannt werdenden Nebenwirkungen limitieren daher bislang auch den therapeutischen Einsatz von ICS bei leichteren Formen der COPD, obgleich ICS, je nach Schweregrad der Atemwegserkrankung, gemäß den Therapierichtlinien noch höher dosiert und in Kombination mit weiteren Therapeutika bis hin zu oralen Glukokortikoiden über einen Zeitraum von zwei bis drei Wochen empfohlen werden. Die eigentliche Ursache hierfür ist auch auf eine nicht immer optimale Wirkeffizienz der eingesetzten Therapeutika zurückzuführen.

Neben den vorgenannten Therapieansätzen werden, mehr oder weniger traditionell, zur symptomatischen Behandlung beispielsweise bronchitischer Beschwerden und zur Erleichterung des Abhustens bei Hypersekretion, insbesondere bei Erkältungskrankheiten, auch inhalativ anzuwendende ätherische Öle bzw. Ölgemische für einen kürzeren Zeitraum eingesetzt. Hierbei handelt es sich jedoch um eine nichtkausale Therapie, welche insbesondere nur bei leichtgradigen, vor allem akuten Atemwegserkrankungen zum Einsatz kommt, jedoch bei chronischen und insbesondere schwergradigen Atemwegserkrankungen allenfalls unterstützend angewendet werden kann.

Darüber hinaus wird unter dem Begriff "Erkrankungen des Verdauungstraktes", wie er auch im Rahmen der vorliegenden Erfindung verwendet wird, eine allgemeine Bezeichnung verstanden, welche insbesondere entzündliche Erkrankungen der oberen wie unteren Abschnitte des Verdauungstraktes, insbesondere des Darms, bezeichnet, wobei hierunter akute wie chronische Krankheitszustände zu subsumieren sind. Beispiele für entzündliche Erkrankungen des Darms sind insbesondere chronisch entzündliche Darmerkrankungen (*Inflammatory Bowel Disease*, IBD), wie *Morbus Crohn* und *Colitis ulcerosa*.

Bei *Morbus Crohn* handelt es sich um chronisch-granulomatöse Entzündungen. Bevorzugt befallen sind der untere Dünndarm (*terminales Ileum*) und Dickdarm (*Co-lon*), seltener die Speiseröhre (*Ösophagus*). Charakterisierend für *Morbus Crohn* ist der diskontinuierliche, segmentale Befall der Darmschleimhaut, es können also gleichzeitig mehrere Darmabschnitte erkrankt sein, die durch gesunde Abschnitte voneinander getrennt sind. Typische Symptome des *Morbus Crohn* sind Bauchschmerzen und Durchfall. Die Schmerzen treten besonders oft im rechten Unter-bauch und oft nach dem Essen oder vor dem Stuhlgang auf. Die Durchfälle sind manchmal blutig. Die Beschwerden treten für gewöhnlich in sogenannten Schüben auf. Ein derartiger Schub dauert meist mehrere Wochen an.

Die *Colitis ulcerosa* (Cu, *ulcerative colitis*) ist durch einen entzündlichen Befall des Mastdarms und des Dickdarms gekennzeichnet. Anders als beim *Morbus Crohn* breitet sich die der Erkrankung zugrundeliegende Entzündung kontinuierlich vom Mastdarm beginnend aus, also von anal nach oral. Die Entzündung als solche ist in der Regel auf die Darmschleimhaut beschränkt.

Glukokortikoide sind die wichtigsten Medikamente bei der Behandlung von chronisch entzündlichen Darmerkrankungen und insbesondere zur Behandlung der akuten Schübe des *Morbus Crohn.* Die therapeutisch und vorrangig systemisch bzw. peroral applizierten Glukokortikoide führen selbst in schweren Fällen noch bei der Hälfte aller Patienten zu einer Remission. Bei einem leichten bis mittelgradigen Schub kann eine gewisse Verbesserung erreicht werden. Jedoch ist der Therapieerfolg nicht immer ausreichend, und zudem treten oftmals gravierende, insbesondere systemische Nebenwirkungen auf.

Der vorliegenden Erfindung liegt daher die Aufgabe zugrunde, die zuvor geschilderten Nachteile des Standes der Technik zumindest teilweise zu vermeiden oder aber wenigstens abzuschwächen.

Insbesondere soll im Rahmen der vorliegenden Erfindung eine verbesserte und/oder effizientere Therapie zur Behandlung von Atemwegserkrankungen, insbesondere bronchopulmonalen Erkrankungen, bzw. zur Behandlung von entzündlichen Darmerkrankungen bereitgestellt werden, welche neben einer Steigerung der Therapieeffizienz auch eine Dosisreduktion der eingesetzten Wirkkomponente ermöglichen sollen.

Insbesondere soll im Rahmen der vorliegenden Erfindung eine verbesserte Wirkeffizienz bzw. ein breiteres Anwendungsspektrum lokal bzw. topisch, insbesondere inhalativ applizierbarer Atemwegstherapeutika der vorgenannten Art (wie z. B. von inhalativen Kortikosteroiden, Bronchodilatatoren und/oder Bronchospasmolytika, einschließlich Sympathomimetika, Phosphodiesterasehemmern, Parasympatholytika und/oder Vagolytika, Anticholinergika etc.) insbesondere im Hinblick auf eine Therapie zur Behandlung von Atemwegserkrankungen ermöglicht bzw. erreicht werden.

Gleichermaßen soll im Rahmen der vorliegenden Erfindung eine verbesserte Wirkeffizienz bzw, ein breiteres Anwendungsspektrum von insbesondere systemisch bzw. peroral applizierbaren Therapeutika (wie z. B von systemischen Kortikosteroiden etc.) insbesondere im Hinblick auf eine Therapie zur Behandlung von Darmentzündungen ermöglicht bzw. erreicht werden.

Zur Lösung der zuvor angeführten Aufgabe schlägt die vorliegende Erfindung - gemäß einem ersten Aspekt der vorliegenden Erfindung - ein Kombinationstherapeutikum, insbesondere pharmazeutisches Kombinationstherapeutikum, gemäß Anspruch 1 bzw. gemäß den Ansprüchen 26 und 27 vor; weitere, insbesondere vorteilhafte Ausgestaltungen des erfindungsgemäßen Kombinationstherapeutikums sind Gegenstand der diesbezüglichen Unteransprüche.

Weiterer Gegenstand der vorliegenden Erfindung sind zudem die Verwendungen eines Vitamin D-Rezeptoragonisten (VDA), wie sie in den jeweiligen Patentansprüchen 28 bis 32 definiert sind. Weitere, insbesondere vorteilhafte Ausgestaltungen der erfindungsgemäßen Verwendungen gemäß diesem Aspekt sind Gegenstand der diesbezüglichen Unteransprüche.

Die vorliegende Erfindung ist sowohl im Hinblick auf das erfindungsgemäße Kombinationstherapeutikum als auch die erfindungsgemäßen Verwendungen dadurch gekennzeichnet, dass das zugrundeliegende Kombinationstherapeutikum nach der Erfindung keinen Inhalts- und/oder Wirkstoff aus der Gruppe von Terpenen, Monoterpenen, Vasokonstriktoren, alpha-Sympathomimetika und/oder deren physiologisch unbedenklichen Salzen oder Derivaten enthält. In diesem Zusammenhang gilt somit auch, dass im Rahmen der vorliegenden Erfindung auch solche Zusammensetzungen, welche im Rahmen einer Co-Medikation der zugrundeliegenden Indikationen bzw. Erkrankungen mit dem erfmdungsgemäßen Kombinationstherapeutikum eingesetzt werden, jeweils keinen Inhalts- und/oder Wirkstoff aus der Gruppe von Terpenen, Monoterpenen, Vasokonstriktoren, alpha-Sympathomimetika und/oder deren physiologisch unbedenklichen Salzen oder Derivaten enthalten.

Es versteht sich von selbst, dass im Folgenden besondere Ausgestaltungen, Ausführungsformen, Vorteile und dergleichen, welche nachfolgend zu Zwecken der Vermeidung von Wiederholungen nur zu einem Erfindungsaspekt beschrieben sind, auch in Bezug auf die übrigen Erfindungsaspekte entsprechend gelten, ohne dass dies einer ausdrücklichen Erwähnung bedarf.

Bei sämtlichen nachstehend genannten relativen bzw. prozentualen gewichtsbezogenen Angaben, insbesondere Mengenangaben, ist weiterhin zu beachten, dass diese im Rahmen der vorliegenden Erfindung vom Fachmann derart auszuwählen sind, dass in der Summe der jeweiligen Inhaltsstoffe, Wirkstoffe, Zusatz- bzw. Hilfsstoffe oder dergleichen stets 100 % bzw. 100 Gew.-% resultieren. Dies versteht sich für den Fachmann aber von selbst.

Im Übrigen gilt, dass der Fachmann anwendungsbezogen oder einzelfallbedingt von den nachfolgend aufgeführten Zahlen-, Bereichs- oder Mengenangaben abweichen kann, ohne dass er den Rahmen der vorliegenden Erfindung verlässt.

Zudem gilt, dass sämtliche im Folgenden genannten Werte- bzw. Parameterangaben oder dergleichen grundsätzlich mit genormten bzw. standardisierten oder explizit angegebenen Bestimmungsverfahren oder anderenfalls mit dem Fachmann auf diesem Gebiet an sich geläufigen Bestimmung- bzw. Messmethoden ermittelt bzw. bestimmt werden können.

Dies vorausgeschickt, wird die vorliegende Erfindung nunmehr nachfolgend im Detail erläutert.

Die Anmelderin hat nun überraschenderweise herausgefunden, dass das zuvor geschilderte Problem dadurch gelöst werden kann, dass im Rahmen der vorliegenden Erfindung ein spezielles Kombinationstherapeutikum, wie nachfolgend definiert, bereitgestellt wird. Das Kombinationstherapeutikum, insbesondere pharmazeutische Kombinationstherapeutikum, nach der Erfindung eignet sich vorzugsweise zur Verwendung bei der prophylaktischen und/oder therapeutischen Behandlung von (A) insbesondere entzündlichen Atemwegserkrankungen, insbesondere bronchopulmonalen Erkrankungen, oder von (B) insbesondere entzündlichen Erkrankungen des Verdauungstraktes, insbesondere des Darms. Das erfindungsgemäße Kombinationstherapeutikum zeichnet sich zudem dadurch aus, dass es insbesondere in jeweils wirksamen, vorzugsweise pharmazeutisch wirksamen Mengen mindestens einen Vitamin D-Rezeptoragonisten (VDA) umfasst. Zudem umfasst das Kombinationstherapeutikum nach der Erfindung mindestens einen weiteren Wirkstoff, wobei der weitere Wirkstoff im Fall der prophylaktischen und/oder therapeutischen Behandlung von (A) Atemwegserkrankungen mindestens ein topisches, insbesondere inhalatives Kortikosteroid ist. Im Fall der Behandlung von (B) entzündlichen Darmerkrankungen ist der weitere Wirkstoff 5-Aminosalicylsäure oder deren physiologisch unbedenkliche Salze oder Ester und gegebenenfalls mindestens ein insbesondere systemisches Kortikosteroid.

In diesem Zusammenhang ist für beide Fälle (A) und (B) eine zwingende Eigenschaft des erfindungsgemäßen Kombinationtherapeutikum darin zu sehen, dass das Kombinationstherapeutikum keinen Inhalts- und/oder Wirkstoff aus der Gruppe von Terpenen, Monoterpenen, Vasokonstriktoren, alpha-Sympathomimetika und/oder deren physiologisch unbedenklichen Salzen oder Derivaten enthält.

Denn die Anmelderin hat in völlig überraschender Weise gefunden, dass auf Basis der erfindungsgemäßen Kombination mit dem gezielten Einsatz eines Vitamin D-Rezeptoragonisten (VDA) eine synergistische Wirkverstärkung sowohl in Bezug auf das Steroid als auch auf 5-Acetylsalicylsäure vorliegt und dass auf dieser Basis ein wirkeffizientes Kombinationstherapeutikum für die angeführten Erkrankungen bzw. Indikationen bereitgestellt wird, welches zu einem verbesserten Therapieerfolg bzw, zu einer Dosisreduktion der eingesetzten pharmakologischen Wirkkomponenten sowie zu einem schnelleren Abklingen der Erkrankung führt. In diesem Zusammenhang kommt auch eine weiterführende Kombination, insbesondere im Sinne einer Co-Medikation, des erfindungsgemäßen Kombinationstherapeutikums insbesondere mit sogenannten Leitlinientherapeutika für die jeweils zugrundeliegende Erkrankung in Betracht, wobei es im Rahmen der vorliegenden Erfindung in völlig unerwarteter Weise gelungen ist, die Wirkeffizienz der jeweils eingesetzten Therapeutika signifikant zu verbessern. Hierauf wird im Nachfolgenden noch im Detail eingegangen. In diesem Zusammenhang kommt dem erfindungsgemäßen Kombinationstherapeutikum insbesondere auch eine antientzündliche Wirkweise zu, welche durch den Einsatz eines Vitamin D-Rezeptoragonisten (VDA) maßgeblich gesteigert wird.

Der im Rahmen der vorliegenden Erfindung völlig überraschend aufgefundene und in den nachfolgenden Ausführungsbeispiele belegte synergistische Effekt, welcher mit dem erfindungsgemäßen Kombinationstherapeutikums einhergeht, kann insbesondere - ohne sich auf diese Theorie beschränken zu wollen - darauf zurückgeführt werden, dass die Expression des Vitamin D-Rezeptors durch Vitamin D bzw. den Vitamin D-Rezeptoragonisten (VDA) selbst reguliert wird. Demnach ist der Vitamin D-Rezeptor bei Vitamin D-Mangelzuständen, welche oftmals bei den zugrundeliegenden Indikationen vorliegen, herunterreguliert bzw. kann durch Vitamin D hochreguliert werden. Die therapeutische Verabreichung von Vitamin D bzw. des in Rede stehenden Vitamin D-Rezeptoragonisten (VDA) bewirkt somit im Rahmen der vorliegenden Erfindung - ohne sich auf diese Theorie beschränken zu wollen - insbesondere in den therapeutisch angeführten Mengen eine Hochregulation des Vitamin D-Rezeptors, welche durch den weiteren Wirkstoff bzw. die weitere Wirkkomponente des erfindungsgemäßen Kombinationstherapeutikums noch weiterführend verstärkt wird. Denn der weitere Wirkstoff, insbesondere in Form von 5-Acetysalicylsäure, führt - gleichermaßen ohne sich auf diese Theorie beschränken zu wollen -insbesondere auch zu einer Hemmung von sogenannten COX II-Metaboliten, insbesondere Prostaglandinen, welche insbesondere bei Infekten sowie akuten und chronischen Entzündungen gebildet werden und eine Herabregulierung von Vitamin D-Rezeptoren induzieren können. Durch die Hemmung von COX II-Metaboliten kann somit auch auf diesem Wege der Vitamin D-Rezeptor hochreguliert werden, und zwar insbesondere insofern, als seine Herunterregulation verhindert wird. Zudem hemmt Vitamin D - gleichermaßen ohne sich auf diese Theorie beschränken zu wollen - sowohl das Protein MKP-1 als auch p-38-MAPK. Insofern führt Vitamin D auch zu einer Beeinflussung insbesondere des MAP-Kinase-Signaltransduktionswegs, welcher unter anderem entzündliche Prozesse im Körper induzieren kann. Die gezielte Wirkkombination der dem erfindungsgemäßen Kombinationstherapeutikum zugrundeliegenden Wirkkomponenten kann auch auf dieser Ebene zu dem in völlig unerwarteter Weise aufgefunden synergistischen Effekt führen.

Durch die erfindungsgemäß vorgesehene weiterführende Kombination mit Steroiden, insbesondere Kortikosteroiden, wird - gleichermaßen ohne sich auf diese Theorie beschränken zu wollen -, bedingt durch die Steroidgabe, eine Verminderung der Prostaglandinsynthese neben einer Hochregulation von Steroidrezeptoren, welche im Allgemeinen für immunmodulatorische Prozesse eine Rolle spielen, erzielt, was einen weiteren positiven Einfluss auf die Verringerung entzündlicher Prozesse hat.

Auf Basis der gezielten Wirkstoffkombination wird somit in völlig unerwarteter Weise ein synergistischer Effekt hinsichtlich der Wirkeffizienz des erfindungsgemäßen Kombinationstherapeutikums bereitgestellt, was folglich auch zu einer Dosisreduktion insbesondere der eingesetzten Kortikosteroide führen kann.

Üblicherweise sind gesunde Personen bei ausreichender Sonnenexposition grundsätzlich unabhängig von einer zusätzlichen Aufnahme von Vitamin D über die Nahrung, da unter Sonneneinfluss bzw. UV-Strahlung ausgehend von der Vorläufersubstanz von Vitamin D, nämlich 7-Dehydroxycholesterol, in der Haut Prävitamin D₃ entsteht. Aus dem instabilen Prävitamin D₃ entsteht nach etwa 48 Stunden Calcitriol, welches eine physiologisch aktive Form von Vitamin D₃ darstellt. Darüber hinaus können ausgehend von 7-Dehydroxycholesterol zum Schutz vor einer Vitamin D₃-Überproduktion Lumisterol und Tachysterol gebildet werden. Ein Mangel oder das Meiden von Sonne aber auch bestimmte Erkrankungen erfordern mitunter jedoch eine nahrungsabhängige Aufnahme von pflanzlichem Vitamin D₂ bzw. Ergocalciferol bzw. von tierischem Vitamin D₃ bzw. Cholecalciferol.

Da im Körper die aktive Form von Vitamin D erst durch enzymatische Prozesse bereitgestellt wird, stellt der körpereigene Vitamin D-Metabolismus ein komplexes Netzwerk auf Basis zahlreicher aufeinanderfolgender Stoffwechselschritte dar. Im Rahmen des Vitamin D-Metabolismus erreichen die verschiedenen Vitamin D-Metabolite, insbesondere gebunden an das Vitamin D-bindende Protein (VDB), die Leber oder werden im Fettgewebe aufgrund ihrer Lipophilie gespeichert. In der Leber werden Vitamin D-Metabolite durch die 25-Hydroxylase (CYP2R1) zu Calcidiol (25-Hydroxyvitamin D bzw. 25-OH D) mit höherer Bindungsaffinität zu dem Vitamin D-Rezeptor (synonym auch als Vitamin D₃-Rezeptor bezeichnet) und mit einer Halbwertszeit von etwa 2,5 Wochen metabolisiert. Die Konversionsrate kann dabei 0,1 bis 0,2 %/10⁶ Zellen/Stunde betragen. Der Spiegel von Calcidiol entspricht der sonnen- und/oder diätbedingten Vitamin D₃-Menge (100 bis 200 IU/Tag) und nimmt im Sommer im Mittel um 12 ng/ml zu.

Durch die 1-alpha-Hydroxylase entsteht im Rahmen des Vitamin D-Metabolismus schließlich der aktive Metabolit Calcitriol (1,25-Dihydroxyvitamin D bzw. 1,25-DOH D) in der Niere und anderen Zellen. Calcitriol besitzt im Vergleich zu Calcidiol mitunter ein stärkeres Potential zur Hypercalcämie. Die Aktivität der 1-alpha-Hydroxylase und somit die renale Bildung von Calcitriol wird durch verschiedene Hormone, insbesondere Parathormon, Oestrogene, Calcitonin, Wachstumshormone und Prolaktin, sowie durch ein Absinken des Phosphatspiegels, insbesondere Hypophosphatämie, stimuliert. Durch Calcium und Calcitriol selber hingegen wird die 1-alpha-Hydroxylase gehemmt. Neben den Zellen der Niere wird die 1-alpha-Hydroxylase auch in anderen Zellen, wie Makrophagen oder Granulomen, exprimiert. Im Gegensatz zu den Zellen der Nieren haben in den übrigen Zellen jedoch Parathormon, Calcium und Vitamin D-Metabolie keinen Einfluss auf deren Aktivität, Nebenwirkungen von Glukokortikoiden, Chloroquin oder Ketoconazol hingegen sind allgemein mit einer Reduktion von Calcitriol verbunden. Bei Adipositas liegt häufig ein Mangel an Calcidiol aufgrund seiner Lipophilie vor, wobei dennoch zwischen Ursache und Folge des Vitamin D-Mangels auch bei Adipositas nicht sicher differenziert werden kann.

Vitamin D-Rezeptoren (VDR) werden von verschiedenen Zellen exprimiert, beispielsweise von Monozyten, Makrophagen, Thrombozyten, Lymphozyten sowie auch Epithelzellen. Folglich kommen Vitamin D-Rezeptoren (VDR) unter anderem in den Atemwegen sowie im Darm vor. Die Expression des Vitamin D-Rezeptors (VDR) für Calcitriol wird dabei durch Calcitriol selbst kontrolliert. Insbesondere ist ein übermäßiger Anstieg der Calcitriolkonzentration mitunter mit einer Hemmung bzw. Verringerung der Expression von Vitamin D-Rezeptoren (VDR) verbunden. Liegt Calcitriol in einer Konzentration von 10⁻⁷ mol/l vor, erfolgt eine 9-fache Expression, wohingegen die Anwesenheit einer höheren Calcitriolkonzentration von 10⁻⁶mol/l lediglich zu einer 1,6-fachen Expression von VDR führt,

Im Gegensatz dazu werden Vitamin D-Rezeptoren (VDR) für Calcidiol weder durch Calcidiol selbst noch durch Calcitriol herunterreguliert, was insbesondere im Hinblick auf den therapeutischen Einsatz von Calcitriol bzw. Calcidiol zu berücksichtigen ist.

Insgesamt ist die Rolle dieser Rezeptoren weitgehend unerforscht. Unter anderem wird von einer Involvierung in die Zunahme der intrazellulären Calcium-Konzentration, die Phospolipase C-Aktivität, die Regulation der DNA-Transkription und die RNA-Synthese mit einer höheren Bindungsaffinität für Calcitriol im Vergleich zu Calcidiol vermutet. In verschiedenen Zellmodellen wurde zudem eine zunehmende Zelldifferenzierung und eine Hemmung der Zellproliferation durch Calcitriol beschrieben und es existieren Hinweise, dass auch Calcidiol hierbei eine Rolle spielt.

Als ungefährer Tagesbedarf von Vitamin D bzw. Vitamin D₃ werden 600 IU empfohlen und bei älteren Patienten oder bei erhöhtem Osteoporoserisiko 800 IU bzw. 4.000 IU als maximale tägliche Aufnahme, um einen Calcidiol-Spiegel von mindestens 20 ng/ml zu erreichen. Andere Empfehlungen zur Behandlung der Osteoporose gehen von einer täglichen Aufnahme von 2.000 IU aus, um einen Plasmaspiegel von > 30 ng/ml sicherzustellen. Hierbei mangelt es jedoch an gesicherten Erfahrungen, um die Entwicklung verschiedener Erkrankungen bei Überdosierung bis hin zur Arteriosklerose und Bauchspeicheldrüsenkarzinomen zu vermeiden. Das *US-Institute of Medicine* empfiehlt in diesem Zusammenhang eine orale Calcitriol-Aufnahme von 50.000 IU/Woche über einen Zeitraum von acht Wochen. Anschließend erfolgt eine Senkung der Calcitriol-Zufuhr auf 50.000 IU alle zwei bis vier Wochen. Alternativ wird die Gabe von 100.000 IU über einen Zeitraum von drei Monaten angeführt.

Insgesamt existiert eine Vielzahl von Erkrankungen, welche mit einem Vitamin D-Mangel assoziiert sind. Dazu gehören überwiegend chronische Erkrankungen, wie verschiedene Tumorerkrankungen, Autoimmunerkrankungen, sowie Infektionserkrankungen, beispielsweise Tuberkulose, kardiovaskuläre Erkrankungen, wie die koronare Herzerkrankung und Bluthochdruck, *Morbus Crohn, Colitis ulcerosa*, Arthritis, Psoriasis und Nierenerkrankungen. Auch Behandlungen mit bestimmten Therapeutika, insbesondere Kortikosteroiden und Antiepileptika, führen zu einem Vitamin D bzw. Vitamin D₃-Mangel als Nebenwirkung. Weiterhin können auch psychische Erkrankungen, wie Depressionen oder Schizophrenie, mit einem Vitamin D bzw. Vitamin D₃-Mangel assoziiert sein. Bei allen vorgenannten Erkrankungen kann im Allgemeinen also ein Mangel an Calcitriol nachgewiesen werden, wobei bislang nicht geklärt ist, ob der Mangel an Vitamin D bzw. Vitamin D₃ die Ursache oder die Folge der Erkrankung ist.

Ein Mangelzustand an Calcitriol wird üblicherweise durch gleichzeitige Bestimmung des Präkusors oder Metaboliten Calcidiol bestimmt.

Es wird angenommen, dass - ohne sich auf diese Theorie beschränken zu wollen-der Wirkmechanismus von Vitamin D auf einer Bindung von Calcidiol bzw. Calcitriol an den Vitamin D-Rezeptor (VDR) basiert. Dieser Komplex bindet an das Vitamin D-Response-Element in der Promotorregion des Vitamin D-Reponsegenes, so dass es zu einer Zunahme der durch die RNA-Polymerase vermittelten Transkription des Vitamin D-Reponsegenes kommt. In Folge der verstärkten Transkription des Vitamin D-Reponsegenes werden verschiedene immunrelevante Vorgänge vermittelt bzw. induziert, unter anderem die Aktivierung der CD4-Rezeptoren von T-Helferzellen, die Hemmung der von T-Helferzellen produzierten Zytokinen (Interferon-gamma, Interleukin-2, Interleukin-5), welche insbesondere zu Entzündungsreaktionen führen, und eine Stimulation von Interleukin-4. Darüber hinaus kann auch eine Hemmung von Interferon-gamma bei gleichzeitiger Stimulation von Interleukin-4, Interleukin-5 und Interleukin-10 auftreten. Auch kann eine Hemmung der Interleukin-12 stimulierten Produktion von Interferon-gamma sowie eine Hemmung von Interleukin-4 und Interleukin-13 durch Calcitriol erfolgen. Aus den vorstehenden Ausführungen ist ersichtlich, dass Vitamin D, insbesondere Calcitriol, an verschiedenen Immunantworten beteiligt ist.

Darüber hinaus wird auch angenommen, dass Calcidiol eine Rolle für Prozesse des Immunsystems haben könnte. Im Rahmen der vorliegenden Erfindung wurde gefunden, dass Calcidiol und Calcitriol die Produktion des im Zusammenhang mit Entzündungsreaktionen gebildeten Tumornekrosefaktor-alpha (TNF-alpha) hemmen, wobei Calcidiol die TNF-alpha-Bildung bis zu dreimal stärker hemmt als Calcitriol und somit überraschenderweise eine nochmals stärkere Entzündungshemmung bewirkt. Darüber hinaus wurde überraschenderweise gefunden, dass auch Calcidiol in synergistischer Weise mit verschiedenen Leitlinientherapeutika interagiert und diese in ihrer Wirkung verstärkt. Für Calcitriol, ist in diesem Zusammenhang beachtlich, dass Calcitriol in höheren Konzentrationen mitunter eine Downregulation von Vitamin D-Rezeptoren (VDR) induziert, so dass die Wirkung von Calcitriol insbesondere bei höheren Konzentrationen von Calcitriol abgeschwächt sein kann. Dennoch kommt auch Calcitriol im Rahmen der vorliegenden Erfindung eine hohe Bedeutung zur Behandlung der zugrundeliegenden Erkrankungen zu.

Auf dieser Grundlage wird im Rahmen der vorliegenden Erfindung auch eine Co-Medikation von Vitamin D bzw. Vitamin D-Metaboliten, insbesondere Calcidiol und/oder Calcitriol, vorzugsweise Calcidiol, in Kombination mit verschiedenen entzündungshemmenden, bronchodilatatorischen und/oder antiallergischen Leitlinientherapeutika, wie nachfolgend definiert, vorgeschlagen, jedoch jeweils mit der Maßgabe, dass das hierzu eingesetzte Kombinationstherapeutikum bzw. die eingesetzte Zusammensetzung keinen Inhalts- und/oder Wirkstoff aus der Gruppe von Terpenen, Monoterpenen, Vasokonstriktoren, alpha-Sympathomimetika und/oder deren physiologisch unbedenklichen Salzen oder Derivaten enthält. Insbesondere werden Co-Medikationen auf Basis einer Kombination von Vitamin D einerseits und topischen oder systemischen Kortikosteroiden bzw. 5-Aminosalicylsäure andererseits, wie in Anspruch 1 definiert, mit Immunsuppressiva, langwirksamen beta-2-Sympatho-mimetika (LABA) oder deren Kombination mit insbesondere inhalativen Kortikosteroiden, langwirksamen Vagolytika (LAMA) vorgeschlagen.

Im Rahmen der vorliegenden Erfindung steht vorrangig die entzündungshemmende Wirkung von Vitamin D im Vordergrund. Erfindungsgemäß von Bedeutung ist dabei einerseits die entzündungshemmende Wirkung von Vitamin D an sich, welche in völlig überraschender Weise im Rahmen eines synergistischen, antientzündlichen Effektes durch eine Kombination mit weiteren Wirkstoffen, insbesondere einem Monoterpen, gesteigert werden kann. Im Rahmen der vorliegenden Erfindung wird in diesem Zusammenhang insbesondere eine Art Zusatztherapie bzw. Co-Medikation zur Verbesserung der klinischen Effekte bzw. eine Reduktion des Medikamentenbedarfs auch im Rahmen von Leitlinientherapien bereitgestellt.

Bei Patienten mit chronisch obstruktiver Atemwegserkrankung (COPD) ist oftmals ein Vitamin D-Mangel vorhanden. In diesem Zusammenhang hat sich gezeigt, dass der Calcidiol-Spiegel im Blut mit dem Schweregrad der Lungenfunktionseinschränkung (FEV1) und somit auch mit dem Schweregrad der COPD (GOLD-Stadien) korreliert.

Was im Speziellen den Zusammenhang zwischen Autoimmunerkrankungen und Vitamin D-Mangel anbelangt, wird vermutet, dass bei verschiedenen Autoimmunerkrankungen ein niedriger Vitamin D-Spiegel durch eine chronische, intrazelluläre Infektion verursacht wird, welche zu einer Fehlfunktion des Vitamin D-Rezeptors führt und auf diese Weise wiederum die Empfänglichkeit für sekundär auftretende Infektionen erhöht. Dies wird insbesondere für die Krankheiten *Psoriasis vulgaris, Lupus Erythematosis*, rheumatoide Arthritis, Sarkoidose, Uveitis und weitere vermutet. Weiterhin sind Patienten mit chronisch polypoider Rhinosinusitis und einer Pilzallergischen Rhinosinusitis teilweise von Vitamin D-Mangelzuständen betroffen, welche auch mit Nasenpolypen, einer vermehrten ossären Erosion und einer Verminderung von dendritischen Zellen assoziiert sind. Häufig tritt ein Vitamin D-Mangel auch bei verschiedenen Formen chronisch-entzündlichen Darmerkrankungen (IBD), insbesondere bei *Morbus Crohn* (CD) und *Colitis ulcerosa* (UC), auf.

Erfindungsgemäß wurde in völlig überraschender Weise gefunden, dass auf Basis des erfindungsgemäßen Kombinationstherapeutikums nicht nur eine effiziente Wirksteigerung bzw. Dosisreduzierung von weiteren Wirkstoffen, wie sie beispielsweise im Rahmen von Leitlinientherapien eingesetzt werden, erreicht werden kann, sondern dass auch den zuvor mit den zugrundeliegenden Krankheiten aufgefundenen Vitamin D-Mangelzuständen in effektiver Weise entgegengetreten werden kann, was einen weiteren zentralen Vorteil der vorliegenden Erfindung darstellt.

Im Stand der Technik wird jedoch im Zusammenhang mit Vitamin D-Mangelerkrankungen, welche vorwiegend aus Beobachtungsstudien und teilweise aus Interventionsstudien bei malignen Erkrankungen, wie Colon-Karzinomen, stammen, zunehmend Empfehlungen nationaler und internationaler Fachverbände ausgesprochen, den Vitamin D-Spiegel nicht routinemäßig zu bestimmen. Insbesondere wird von unnötigen Vitamin D-Supplementierungen abgeraten. So empfiehlt das *U.S Institute of Medicine* (IOM) im Januar 2011, dass für die Knochengesundheit eine Vitamin D-Konzentration von 20 ng/ml für 97,5 % der Bevölkerung völlig auseichend ist. Bislang wurde eine Vitamin D-Konzentration von 30 ng/ml empfohlen. Darüber hinaus wird weiterhin argumentiert, dass "Nahrungsergänzungsmittel und Vitamin D-Zusätze zu Lebensmitteln aus medizinischer Sicht nur dann sinnvoll sind, wenn weitere Risikofaktoren für eine Osteoporose vorliegen - beispielsweise bei älteren Menschen oder bei verminderter Knochendichte". In gleicher Weise warnt auch die Deutsche Gesellschaft für Endokrinologie, dass "eine Überversorgung Gesundheitsrisiken berge und die Einnahme von Vitamin D-Präparaten nur in ärztlich begründeten Fällen notwendig ist", insbesondere vor dem Hintergrund, dass der Organismus ungefähr 80 % des benötigten Vitamin D selbst bildet. Auch ausgehend von den obigen Ausführungen ist die erfindungsgemäß vorgeschlagene gezielte Therapie für die angeführten Erkrankungen auf Basis von Vitamin D dem Fachmann gerade nicht nahegelegt.

Denn erfindungsgemäß wurden nunmehr in völlig überraschender Weise spezifische Wirkeffekte von Vitamin D, insbesondere Calcidiol und/oder Calcitriol, gefunden, welche die erfindungsgemäßen speziellen Verwendungen im Rahmen von Wirkeffizienzsteigerungen und Dosisreduktionen begleitender Therapeutika bzw. pharmakologischer Wirksubstanzen ermöglichen.

In diesem Zusammenhang wurde überraschenderweise auch gefunden, dass Calcidiol, der Präkusor und Metabolit von Calcitriol, eine primäre und stärker ausgebildete sowie eigenständige steroidartige und entzündungshemmende Wirkung besitzt als Calcitriol bzw. Vitamin D in seiner physiologisch aktiven Form selbst, so dass Cacidiol eine erfindungsgemäß besonders bevorzugte Wirkkomponente in Form des Vitamin D-Rezeptoragonisten (VDA) darstellt.

Insbesondere übersteigt die entzündungshemmende Wirkung von Calcidiol die Wirkung von Calcitriol etwa um das dreifache, so dass Calcidiol, wie zuvor angeführt, im Rahmen des erfindungsgemäßen therapeutischen Einsatzes, insbesondere als Kombinationstherapeutikum zur Wirkungssteigerung von Leitlinientherapeutika, als eine der bevorzugten Substanzen anzuführen ist. Dessen ungeachtet kommt jedoch auch Calcitriol im Hinblick auf die vorliegende Erfindung eine große Rolle als Vitamin D-Rezeptoragonist (VDA) zu.

Für den therapeutischen Einsatz von Calcitriol und/oder Calcidiol bzw. einer Mischung aus beiden vorgenannten Substanzen zur Erhöhung insbesondere lokaler, antientzündlicher Vitamin D-Konzentrationen und somit der Wirksamkeit, wird erfindungsgemäß zur Gewährleistung einer optimalen Verfügbarkeit ein spezielles Therapiekonzept im Allgemeinen zur primären Lokaltherapie einerseits zur Behandlung von Atemwegserkrankungen, insbesondere in Form eines Nasensprays; zur inhalativen Therapie, insbesondere in Form eines Pulvers und/oder Dosieraerosols; und andererseits zur Behandlung entzündlicher Darmerkrankungen (*Morbus Crohn, Colitis ulcerosa*), insbesondere in Form von Pellets oder gelöst bzw, suspendiert in Öl in dünndarmlöslichen Kapseln, zur lokalen Darmtherapie- und der Systemtherapie bei assoziierter Systementzündung bereitgestellt.

Erfindungsgemäß kann das Kombinationstherapeutikum für die Therapie mit Vitamin D somit in nichtbeschränkender Weise in Form einer insbesondere inhalierbaren Lösung oder Suspension auf Basis eines Pulvers oder Sprays bereitgestellt werden, insbesondere verbunden mit dem Vorteil, unabhängig vom Körpergewicht und der Lipophilie des Wirkstoffs, auf Basis von Vitamin D eine lokale Schleimhautentzündung, z. B. der Atemwege, direkt zu behandeln. Durch diesen Ansatz werden auch gegebenenfalls vorliegende Vitamin D-Nebenwirkungen der oralen Therapie, insbesondere die etwaige Ausbildung einer Hypercalcämie, vermieden.

Gegenstand der vorliegenden Erfindung - gemäß einem ersten Aspekt der vorliegenden Erfindung - ist somit ein Kombinationstherapeutikum, insbesondere pharmazeutisches Kombinationstherapeutikum, vorzugsweise zur Verwendung bei der prophylaktischen und/oder therapeutischen Behandlung von (A) insbesondere entzündlichen Atemwegserkrankungen, insbesondere bronchopulmonalen Erkrankungen, oder von (B) insbesondere entzündlichen Erkrankungen des Verdauungstraktes, insbesondere des Darms,
wobei das Kombinationstherapeutikum - insbesondere in jeweils wirksamen, vorzugsweise pharmazeutisch wirksamen Mengen - umfasst:
(a) mindestens einen Vitamin D-Rezeptoragonisten (VDA) und
(b) mindestens einen weiteren Wirkstoff,
   (b1) wobei der weitere Wirkstoff mindestens ein topisches, insbesondere inhalatives Kortikosteroid im Fall der prophylaktischen und/oder therapeutischen Behandlung von (A) Atemwegserkrankungen ist oder
   (b2) wobei der weitere Wirkstoff 5-Aminosalicylsäure oder deren physiologisch unbedenkliche Salze oder Ester und gegebenenfalls mindestens ein insbesondere systemisches Kortikosteroid im Fall der prophylaktischen und/oder therapeutischen Behandlung von (B) entzündlichen Darmerkrankungen ist;
jedoch jeweils mit der Maßgabe, dass das Kombinationstherapeutikum keinen Inhalts- und/oder Wirkstoff aus der Gruppe von Terpenen, Monoterpenen, Vasokonstriktoren, alpha-Sympathomimetika und/oder deren physiologisch unbedenklichen Salzen oder Derivaten enthält.

Die Begriffe "Kombinationstherapeutikum" bzw. "pharmazeutisches Kombinations-therapeutikum" oder dergleichen, wie sie im Rahmen der vorliegenden Erfindung verwendet werden, sind sehr umfassend zu verstehen und bezeichnen nicht nur pharmazeutische Präparate bzw. Pharmazeutika, sondern auch sogenannte Medizinprodukte, homöopathische Mittel, Nahrungsergänzungsmittel oder dergleichen.

Weiterhin werden unter den Begriffen "Vitamin D-Rezeptoragonist" bzw. "Vitamin D-Rezeptoragonist (VDA)", wie sie im Rahmen der vorliegenden Erfindung verwendet werden, insbesondere solche Substanzen verstanden, welche imstande sind, mit den zugrundeliegenden Vitamin D-Rezeptoren insbesondere nach Art eines Liganden in Wechselwirkung zu treten bzw. mit den zugrundeliegenden Vitamin D-Rezeptoren zu interagieren, so dass infolge der zugrundeliegenden Wechselwirkung bzw. Interaktion eine Aktivierung der in Rede stehenden Vitamin D-Rezeptoren erfolgt. Insbesondere kann die Wechselwirkung bzw. Interaktion des erfindungsgemäß eingesetzten Vitamin D-Rezeptoragonisten mit den Vitamin D-Rezeptoren auf Basis einer Bindung, insbesondere auf Basis des sogenannten "Schlüssel/Schloss-Prinzips" erfolgen, infolgedessen eine Aktivierung der zugrundeliegenden Vitamin D-Rezeptoren mit nachfolgender Induktion stoffwechselspezifischer Vorgänge erfolgen kann. Hierbei kann es insbesondere vorgesehen sein, dass die erfindungsgemäß eingesetzte Substanz als solche, d. h. ohne weiterführende Modifizierung bzw. Verstoffwechselung, mit den Vitamin D-Rezeptoren zu interagieren imstande ist. Gleichermaßen umfassen die Begriffe "Vitamin D-Rezeptoragonist" bzw. "Vitamin D-Rezeptoragonist (VDA)", auch solche Substanzen, welche selbst nicht unmittelbar zur Wechselwirkung, insbesondere Bindung, mit bzw. an den Vitamin D-Rezeptor imstande sind, sondern gewissermaßen als Vorläufersubstanzen bzw. Präkursoren zur Bereitstellung eines Liganden für den Vitamin D-Rezeptor fungieren. Dabei kann die Modifizierung bzw. Umwandlung der eingesetzten Vorläufer insbesondere auf Basis stoffwechselspezifischer Vorgänge im Körper erfolgen.

Im Rahmen der vorliegenden Erfindung können als Vitamin D-Rezeptoragonisten insbesondere den natürlichen bzw. körpereigenen Metaboliten des körpereigenen Vitamin D-Stoffwechsels entsprechende bzw. hierzu chemisch zumindest im Wesentlichen identische bzw. wirkidentische Substanzen auf Basis synthetischer Verbindungen eingesetzt werden, wobei es sich diesbezüglich, wie nachfolgend noch definiert, insbesondere um 25-Hydroxy-Vitamin D₃ bzw. 25-Hydroxy-Vitamin D (Calcidiol) und/oder 1,25-Dihydroxy-Vitamin D₃ bzw. 1,25-Dihydroxy-Vitamm D (Calcitriol) handelt. In diesem Zusammenhang ist es im Rahmen der vorliegenden Erfindung gleichermaßen in nicht beschränkender Weise möglich, die entsprechenden Vorläufersubstanzen bzw. Präkursoren bzw, Speicherformen der in Rede stehenden Substanzen, wie beispielsweise 7-Dehydrocholesterol, Vitamin D₃ (Cholecalciferol) bzw. entsprechende inaktive Speicherprodukte, wie Lumisterol bzw. Tachysterol, einzusetzen, auch wenn dies erfindungsgemäß weniger bevorzugt ist. Insbesondere kann der Vitamin D-Rezeptoragonist auch in Form von Vitamin D₃ bzw. Cholecalciferol als solchem eingesetzt werden, welches nach entsprechender Verabreichung bzw. Applikation - ohne sich auf diese Theorie beschränken zu wollen - im Körper zu den entsprechend aktiven Liganden, insbesondere Calcidiol und/oder Calcitriol, metabolisiert bzw. verstoffwechselt wird.

Im Rahmen der vorliegenden Erfindung ist es, wie zuvor ausgeführt, gleichermaßen möglich, synthetische Analoga von Vitamin D₃ sowie dessen Vorläufern bzw. Präkursoren als Vitamin D-Rezeptoragonisten einzusetzen. Insbesondere kommt im Rahmen der vorliegenden Erfindung auch die Verwendung von Vitamin D₃-Analoga, wie Doxercalciferol, Alphacalciferol, Calcipotriol bzw. Dihydrotachystyrol, als Vitamin D-Rezeptoragonist in Betracht.

Gleichermaßen kommt im Rahmen der vorliegenden Erfindung auch die Verwendung von Vitamin D₂ bzw. Ergocalciferol sowie dessen Analoga in Betracht.

Bei den erfindungsgemäß eingesetzten Vitamin D-Rezeptoragonisten handelt es sich insbesondere um Substanzen, welche in Form von sogenannten Secosteroiden vorliegen können.

Weiterhin beziehen sich die im Rahmen der vorliegenden Erfindung verwendeten Begriffe "Vitamin D-Rezeptor" bzw. "Vitamin D-Rezeptor (VDR)" auf insbesondere zur Familie der Steroidrezeptoren vom Typ II gehörenden ligandenaktivierte Transkriptionsfaktoren bzw. Rezeptoren, welche insbesondere eine Affinität zu Liganden aus der Vitamin D-Gruppe aufweisen, wobei die in Rede stehenden Rezeptoren insbesondere eine hohe (Bindungs-)Affinität zu 25-Hydroxy-Vitamin D (Calcidiol) und/oder 1,25-Dihydroxy-Vitamin D (Calcitriol) aufweisen. Die in Rede stehenden Rezeptoren, welche erfindungsgemäß sozusagen als Zielstruktur bzw. *Target* für die eingesetzten Liganden fungieren, stellen somit im Allgemeinen insbesondere zu der Familie der Steroidrezeptoren gehörende Rezeptoren in Form von ligandenaktivierten Transkriptionsfaktoren dar, die infolge ihrer Aktivierung bestimmte Zielgene zu aktivieren oder zu hemmen im Stande sind, und so den Stoffwechsel insbesondere auch im Hinblick auf die Steuerung entzündlicher Prozesse beeinflussen können. Ausgehend von der hohen Bindungsaffinität des Vitamin D-Rezeptors zu Calcidiol bzw, Calcitritol resultiert auch dessen Funktion, nämlich die Vermittlung der physiologischen Funktionen von Vitamin D in den zugrundeliegenden biologischen Strukturen, insbesondere Zielzellen. Ohne sich auf diese Theorie beschränken zu wollen, bilden Vitamin D-Rezeptoren nach Aktivierung durch Anbindung des Liganden ein Heterodimer mit dem sogenannten Retinoid-X-Rezeptor, wobei das resultierende Heterodimer als regulatives Element für die Expression von Zielgenen des Vitamin D-Rezeptors dient. Insbesondere kommt Vitamin D-Rezeptoren eine wichtige Funktion in der Regulation von Genen bzw. Genprodukten zu, welche für Immunantworten, insbesondere entzündliche Prozesse unter Prostaglandinausschüttung sowie für proliferative Prozesse von Bedeutung sind.

Im Rahmen der vorliegenden Erfindung kann es vorgesehen sein, dass (a) der Vitamin D-Rezeptoragonist (VDA) ausgewählt ist aus der Gruppe von Verbidungen der Vitamin D-Gruppe, insbesondere Vitamin D₃ (Cholecalciferol) und/oder Vitamin D₂ (Ergocaliferol), vorzugsweise Vitamin D₃ (Cholecalciferol). Gemäß einer erfindungsgemäß besonders bevorzugten Ausführungsform ist (a) der Vitamin D-Rezeptoragonist (VDA) die Substanz 25-Hydroxy-Vitamin D₃ (Calcidiol).

Gleichermaßen kann es im Rahmen der vorliegenden Erfindung vorgesehen sein, dass (a) der Vitamin D-Rezeptoragonist (VDA) ausgewählt ist aus der Gruppe von insbesondere physiologischen Vitamin D₃-Metaboliten und/oder insbesondere physiologischen Vitamin D₃-Präkursoren, vorzugsweise aus der Gruppe von insbesondere physiologischen Vitamin D₃-Metaboliten. Insbesondere kann (a) der Vitamin D-Rezeptoragonist (VDA) ausgewählt sein aus der Gruppe von insbesondere synthetischen Vitamin D₃-Analoga, insbesondere Tacalcitol (1,24-Dihydroxy-Vitamin D₃).

Erfindungsgemäß werden im Hinblick auf die Behandlung von insbesondere entzündlichen Erkrankungen der oberen Atemwege bzw. von insbesondere entzündlichen Erkrankungen des Darms besonders gute Ergebnisse erhalten, wenn der Vitamin D-Rezeptoragonist (VDA) ausgewählt ist aus der Gruppe von 25-Hydroxy-Vitamin D₃ (Calcidiol) und/oder 1,25-Dihydroxy-Vitamin D₃ (Calcitriol), vorzugsweise 25-Hydroxy-Vitamin D₃ (Calcidiol).

Bei 25-Hydroxy-Vitamin D₃, welches synonym auch als 25-OH-Vitamin D₃ bzw. 25-OH D₃ bzw, 25-Hydroxycholecalciferol bezeichnet wird, handelt es sich insbesondere um eine Substanz des Vitamin D-Stoffwechsels mit Ligandenfunktion in Bezug auf Vitamin D-Rezeptoren. Im menschlichen Körper kann Calcidiol ausgehend von Vitamin D₃ durch Hydroxylierung von Vitamin D₃ insbesondere in der Leber gebildet werden.

Weiterhin handelt es sich bei 1,25-Dihydroxy-Vitamin D₃ bzw. Calcitriol, synonym auch als 1,25-(OH)₂-Vitamin D₃, 1.25-DOH D₃ bzw. 1α,25(OH)₂-Cholecalciferol bezeichnet, um eine weitere Substanz des Vitamin D₃-Stoffwechsels, wobei Calcitriol aus einer weiterführenden Hydroxylierung von Calcidiol resultieren kann.

Erfindungsgemäß ist es gleichermaßen möglich, dass (a) der Vitamin D-Rezeptoragonist (VDA) eine Kombination von 25-Hydroxy-Vitamin D₃ (Calcidiol) und 1,25-Dihydroxy-Vitamin D₃ (Calcitriol) ist.

In diesem Zusammenhang hat es sich als besonders vorteilhaft erwiesen, wenn das Kombinationstherapeutikum (i) 25-Hydroxy-Vitamin D₃ und (ii) 1,25-Dihydroxy-Vitamin D₃ in einem gewichtsbezogenen Mengenverhältnis von [(i) : (ii)] im Bereich von 1.000 : 1 bis 1 : 100, insbesondere im Bereich von 500 : 1 bis 1 : 10, vorzugsweise im Bereich von 100 : 1 bis 1 : 5, bevorzugt im Bereich von 50 : 1 bis 1 : 1, besonders bevorzugt im Bereich von 10 : 1 bis 1 : 1, aufweist.

Gemäß einer erfindungsgemäß weiter bevorzugten Ausführungsform kann der Vitamin D-Rezeptoragonist (VDA) 25-Hydroxy-Vitamin D₃ (Calcidiol) sein.

Im Allgemeinen kann die Menge an Vitamin D-Rezeptoragonist (VDA) in dem erfindungsgemäßen Kombinationstherapeutikum in weiten Bereichen variieren. Insbesondere kann es im Rahmen der vorliegenden Erfindung vorgesehen sein, insbesondere um eine ausreichende Wirksamkeit zu gewährleisten, dass das Kombinationstherapeutikum nach der Erfindung (a) den Vitamin D-Rezeptoragonisten (VDA), insbesondere 25-Hydroxy-Vitamin D₃ (Calcidiol) in einer Menge im Bereich von 0,0001 Gew.-% bis 5 Gew.-%, insbesondere im Bereich von 0,0005 Gew.-% bis 2 Gew.-%, vorzugsweise im Bereich von 0,001 Gew.-% bis 1,5 Gew.-%, bevorzugt im Bereich von 0,005 Gew.-% bis 1 Gew.-%, besonders bevorzugt im Bereich von 0,01 Gew.-% bis 0,75 Gew.-%, ganz besonders bevorzugt im Bereich von 0,05 Gew.-% bis 0,5 Gew.-%, bezogen auf das Kombinationstherapeutikum, enthält.

Zur Gewährleistung einer hohen Wirksamkeit des erfindungsgemäßen Kombinationstherapeutikums kann es im Rahmen der vorliegenden Erfindung zudem vorgesehen sein, dass das Kombinationstherapeutikum (a) den Vitamin D-Rezeptoragonisten (VDA), insbesondere 25-Hydroxy-Vitamin D₃ (Calcidiol) und/oder 1,25-Dihydroxy-Vitamin D₃ (Calcitriol), in einer Menge im Bereich von 0,025 µg 500 µg, insbesondere im Bereich von 0,05 µg bis 400 µg, vorzugsweise im Bereich von 0,1 µg bis 300 µg, bevorzugt im Bereich von 1 µg bis 200 µg, besonders bevorzugt im Bereich von 10 µg bis 100 µg, bezogen auf eine Dosier- und/oder Applikationseinheit des Kombinationstherapeutikums, enthält.

Im Rahmen der vorliegenden Erfindung kann es zudem vorgesehen sein, dass das Kombinationtherapeutikum nach der Erfindung mindestens einen mit (a) dem Vitamin D-Rezeptoragonisten (VDA) mischbaren und/oder hierin löslichen, insbesondere bei 20°C und Atmosphärendruck flüssigen, physiologisch unbedenklichen Träger (Exzipienten) enthält. Der in Rede stehende Träger bzw. Exzipient dient insbesondere zur Aufnahme der Wirksubstanzen, vorzugsweise auf Basis des Vitamin D-Rezeptoragonisten (VDA) bzw. gegebenenfalls eingesetzter Kortikoide oder dergleichen.

Insbesondere im Hinblick auf die Behandlung von (A) insbesondere entzündlichen Atemwegserkrankungen kann es erfindungsgemäß vorgesehen sein, dass (b1) das topische, insbesondere inhalative Kortikosteroid ein Glukokortikoid ist, vorzugsweise ausgewählt aus der Gruppe von Beclometason, Mometason, Budesonid, Flunisolid, Fluticason, Triamcinolon und deren physiologisch verträglichen Derivaten, insbesondere Salzen und Estern, sowie Mischungen und Kombinationen der vorgenannten Verbindungen.

Die Kortikosteroide sind im Allgemeinen eine Gruppe von ca. 50 in der Nebennierenrinde gebildeten Steroidhormonen sowie chemisch vergleichbaren synthetischen Stoffen, wobei sämtliche Kortikosteroide aus dem Ausgangsstoff Cholesterin entstehen und als gemeinsames Grundgerüst das Progesteron (Delta-4-Pregnen-3,20-Dion) aufweisen. Die Kortikosteroide lassen sich nach ihrer biologischen Wirkung bzw. ihrem Bildungsort in drei Gruppen einteilen, nämlich Mineralkortikoide, Glukokortikoide und Androgene. Die erfindungsgemäß bevorzugt eingesetzten Glukukortikoide zählen somit zu den Kortikosteroiden.

Um eine ausreichende Wirksamkeit des erfindungsgemäßen Kombinationstherapeutikums zu gewährleisten, sollte das Kombinationstherapeutikum (b1) das topische, insbesondere inhalative Kortikosteroid insbesondere in einer Menge im Bereich von 0,001 Gew.-% bis 10 Gew.-%, insbesondere im Bereich von 0,01 Gew.-% bis 5 Gew.-%) vorzugsweise im Bereich von 0,1 Gew.-% bis 2 Gew,-%, bezogen auf das Kombinationstherapeutikum, enthalten.

Auch dem mengenbezogenen Verhältnis von (a) Vitamin D-Rezeptoragonist (VDA) einerseits und (b1) topisches, insbesondere inhalatives Kortikosteroid andererseits kommt erfindungsgemäß eine hohe Bedeutung zu: So hat es sich als besonders vorteilhaft erwiesen, wenn das Kombinationstherapeutikum (a) den Vitamin D-Rezeptoragonisten (VDA) und (b1) das topische, insbesondere inhalative Kortikosteroid in einem gewichtsbezogenen Mengenverhältnis von [(a): (b1)] im Bereich von 100 : 1 bis 1 : 1000, insbesondere im Bereich von 10 : 1 bis 1 : 500, vorzugsweise im Bereich von 5 : 1 bis 1 : 100, bevorzugt im Bereich von 1 : 1 bis 1 : 50, besonders bevorzugt im Bereich von 1 : 1 bis 1 : 10, aufweist.

Was darüber hinaus die im Fall der Behandlung von (B) entzündlichen Damerkrankungen eingesetzte Wirkkomponente in Form von (b2) 5-Aminosalicylsäure (5-ASA) anbelangt, so kann diese in Form ihrer unbedenklichen Alkali- und/oder Erdalkalisalze, vorzugsweise in Form des Natriumsalzes von 5-Aminosalicylsäure und/oder Natrium-5-Aminosalicylat, eingesetzt werden.

Bei 5-Aminosalicylsäure (synonym auch als 5-ASA bzw. Mesalazin bezeichnet) handelt es sich um ein Aminderivat der Salicylsäure mit entzündungshemmender Wirkung.

Erfindungsgemäß ist es bevorzugt, wenn das Kombinationstherapeutikum nach der Erfindung (b2) 5-Aminosalicylsäure in einer Menge im Bereich von 0,001 Gew.-% bis 50 Gew.-%, insbesondere im Bereich von 0,01 Gew.-% bis 20 Gew.%, vorzugsweise im Bereich von 0,05 Gew.-% bis 10 Gew.-%, bevorzugt im Bereich von 0,1 Gew.-% bis 5 Gew.-%, bezogen auf das Kombinationstherapeutikum, enthält.

Hinsichtlich einer weiterführend optimierten Wirkeffizienz kommt dabei auch dem Verhältnis von (a) Vitamin D-Rezeptoragonist (VDA) einerseits zu (b2) 5-Aminosalicylsäure andererseits in dem erfindungsgemäßen Kombinationstherapeutikum eine große Bedeutung zu: So hat es sich als vorteilhaft erwiesen, wenn das Kombinationstherapeutikum nach der Erfindung (a) den Vitamin D-Rezeptoragonisten (VDA) und (b2) 5-Aminosalicylsäure in einem gewichtsbezogenen Mengenverhältnis von [(a): (b2)] im Bereich von 100 : 1 bis 1 : 10.000, insbesondere im Bereich von 10 : 1 bis 1 : 5.000, vorzugsweise im Bereich von 5 : 1 bis 1 : 1.000, bevorzugt im Bereich von 1 : 1 bis 1 : 500, bevorzugt im Bereich von 1 : 2 bis 1 : 100, aufweist.

Zur weiterführenden Steigerung bzw. individuellen Anpassung bzw. Maßschneiderung der Wirksamkeit des erfindungsgemäßen Kombinationstherapeutikums im Hinblick auf die zugrundeliegende Indikation kann es zudem vorgesehen sein, dass das Kombinationstherapeutikum nach der Erfindung mindestens einen weiteren pharmakologisch wirksamen Inhaltsstoff und/oder Wirkstoff umfasst. Diesbezüglich kann der pharmakologisch wirksame Inhaltsstoff und/oder Wirkstoff ausgewählt sein aus der Gruppe von Antiphlogistika, insbesondere nichtsteroidalen Antiphlogistika, vorzugsweise Acetylsalicylsäure und/oder Paracetamol; beta-Sympathomimetika, vorzugsweise beta-2-Sympathomimetika; Parasympatholytika und/oder Vagolytika; und Anticholinergika; sowie Mischungen und Kombinationen der vorgenannten Verbindungen. In diesem Zusammenhang kommt insbesondere auch den nichtsteroidalen Antiphlogistika eine zusätzliche Rolle - ohne sich auf diese Theorie beschränken zu wollen - in der Hemmung von COX II-Metaboliten, insbesondere Prostaglandinen, zu, was zu einer weiteren Steigerung der antientzündlichen Wirksamkeit führt.

Der Fachmann ist jederzeit in der Lage, den weiteren pharmakologisch wirksamen Inhalts- bzw. Wirkstoff sowohl hinsichtlich seiner Art als auch seiner Menge in Bezug auf die zugrundeliegende Indikation bzw. Erkrankung auszuwählen. So kommt beispielsweise insbesondere im Hinblick auf die Behandlung von (A) Atemwegserkrankungen der Einsatz von beta-Sympathomimetika, Parasympathomimetika und/oder Vagolytika sowie Anticholinergika in Betracht, wohingegen in Bezug auf (B) entzündliche Darmerkrankungen insbesondere die Verwendung von Antiphlogistika in Betracht kommt. Die zuvor angeführte Einteilung ist jedoch in keiner Weise abschließend zu verstehen.

Was weiterhin die Indikation der (B) entzündlichen Darmerkrankungen anbelangt, so kann es im Rahmen der vorliegenden Erfindung vorgesehen sein, dass (b2) das systemische Kortikosteroid ein Glukokortikoid, insbesondere Glukokortikosteroid, ist. In diesem Zusammenhang kann das Kortikosteroid ausgewählt sein aus der Gruppe von Dexamethason, Prednison, Prednisolon, Betamethason, Rimexolon, Paramethason, Hydrocortison und deren physiologisch verträglichen Derivaten, insbesondere Salzen und Estern, sowie Mischungen und Kombinationen der vorgenannten Verbindungen.

In diesem Zusammenhang ist es erfindungsgemäß von Vorteil, wenn - insbesondere zur Gewährleistung einer ausreichenden Wirkeffizienz - das Kombinationstherapeutikum nach der Erfindung (b2) das systemische Kortikosteroid in einer Menge im Bereich von 0,001 Gew.-% bis 10 Gew.-%, insbesondere im Bereich von 0,01 Gew.-% bis 5 Gew.-%, vorzugsweise im Bereich von 0,1 Gew.-% bis 2 Gew.-%, bezogen auf das Kombinationstherapeutikum, enthält.

Erfindungsgemäß kommt auch im Fall der Verwendung eines systemischen Kortikosteroids dem mengenbezogenen Verhältnis von (a) Vitamin D-Rezeptoragonist (VDA) einerseits und (b2) systemisches Kortikosteroid andererseits eine vorteilhafte Bedeutung zu: So kann es erfindungsgemäß vorgesehen sein, dass das Verhältnis [(a) : (b2)] im Bereich von 100 : 1 bis 1 : 1.000, insbesondere im Bereich von 10 : 1 bis 1 : 500, vorzugsweise im Bereich von 5 : 1 bis 1 : 100, bevorzugt im Bereich von 1 : 1 bis 1 : 50, besonders bevorzugt im Bereich von 1 : 1 bis 1 : 10, liegt.

Erfindungsgemäß kann es gleichermaßen vorgesehen sein, dass das Kombinationstherapeutikum nach der Erfindung mindestens ein weiteres Vitamin, insbesondere ausgewählt aus Gruppe von Vitamin A, Vitamin C und Vitamin E, insbesondere Vitamin C und Vitamin E, enthält.

Zudem kann das erfindungsgemäße Kombinationstherapeutikum mindestens einen weiteren Inhaltsstoff aufweisen, insbesondere ausgewählt aus der Gruppe von Verarbeitungshilfsstoffen, Stabilisatoren, insbesondere organischen Lösemitteln, Emulgatoren, Antioxidantien, Feuchthaltemitteln, Verdickungsmitteln, Antiseptika, Farbstoffen, Aromastoffen, Riechstoffen, Duftstoffen, Streckmitteln, Bindemitteln, Netzmitteln, Vitaminen, Spurenelementen, Mineralstoffen, Mikronährstoffen und/oder ätherischen Ölen sowie deren Kombinationen.

Was das erfindungsgemäße Kombinationstherapeutikum weiterhin anbelangt, so kann dieses topisch applizierbar sein und/oder appliziert werden. Gleichermaßen kann das Kombinationstherapeutikum nach der Erfindung systemisch, insbesondere peroral applizierbar sein und/oder appliziert werden.

In diesem Zusammenhang kommt in nicht beschränkender Weise die topische Applizierung des Kombinationstherapeutikums nach der Erfindung, insbesondere in Bezug auf die diesbezügliche Verwendung zur Behandlung von (A) Atemwegserkrankungen in Betracht. In diesem Zusammenhang ist der Begriff des topisch, insbesondere inhalativ zu applizierenden Kombinationstherapeutikums erfindungsgemäß sehr breit zu verstehen, was insbesondere sämtliche dem Fachmann zur Behandlung von Atemwegserkrankungen bekannten Darreichungsformen umfasst, welche sich für die topische, insbesondere inhalative Anwendung eignen, wie z. B. pulverbasierte und/oder wässrig basierte Sprühformulierungen oder dergleichen, insbesondere Inhalations- und/oder Rachensprayformulierungen bzw. Aerosolsprays zur Inhalation und/oder Applikation im Rachenraum.

Weiterhin kommt die systemische Verabreichung und/oder Applikation, insbesondere in peroraler Form, insbesondere und in nicht beschränkender Weise im Hinblick auf die Behandlung von (B) entzündlichen Erkrankungen des Verdauungstrakts, insbesondere des Darms, in Betracht. In diesem Zusammenhang kann das Kombinationstherapeutikum nach der Erfindung insbesondere in Form von dünndarmlöslichen Tabletten bzw. Kapseln oder aber in Form von sogenannten Pellets vorliegen bzw. verabreicht werden, was insbesondere im Hinblick auf die Behandlung von entzündlichen Darmerkrankungen zu hohen Wirkstoffkonzentrationen am Wirkort führt.

Der Fachmann ist jederzeit in der Lage, die entsprechende Darreichungs- bzw. Applikationsform vor dem Hintergrund der jeweils zugrundeliegenden Erkrankung auszuwählen und anzupassen. So kommt eine systemische Applikation grundsätzlich auch für die Behandlung von insbesondere entzündlichen Atemwegserkrankungen in Betracht, insbesondere im Hinblick auf eine systemische Aufnahme der entsprechenden Wirkkomponenten mit entsprechend positiver Beeinflussung bzw. hoher Wirkeffizienz sowohl in Bezug auf lokale als auch systemische (Krankheits-)-Prozesse.

Im Allgemeinen kann das Kombinationstherapeutikum nach der Erfindung in Tagesdosen von (a) im Bereich von 10 µg bis 1.000 µg, insbesondere 20 µg bis 800 µg, vorzugsweise 30 µg bis 500 µg, Vitamin D-Rezeptoragonist (VDA) verabreicht werden. Insbesondere kann das Kombinationstherapeutikum zur Verabreichung in einer Tagesdosis von (a) 10 µg bis 1.000 µg, insbesondere 20 µg bis 800 µg, vorzugsweise 30 µg bis 500 µg, Vitamin D-Rezeptoragonist (VDA) hergerichtet sein.

Gleichermaßen kann - insbesondere im Hinblick auf die Behandlung von (A) Atemwegserkrankungen - das Kombinationstherapeutikum in Tagesdosen von (b1) im Bereich von 50 bis 1.000 µg, insbesondere 75 bis 800 µg, besonders bevorzugt 100 bis 600 µg, topisches, insbesondere inhalatives Kortikosteroid verabreicht werden. Insbesondere kann das Kombinationstherapeutikum zur Verabreichung in einer Tagesdosis von (b1) 50 bis 1.000 µg, insbesondere 75 bis 800 µg, besonders bevorzugt 100 bis 600 µg, topisches, insbesondere inhalatives Kortikosteroid hergerichtet sein.

Zudem kann - insbesondere im Hinblick auf die Behandlung von (B) entzündlichen Darmerkrankungen - das Kombinationstherapeutikum in Tagesdosen von (b2) im Bereich von 50 bis 10.000 mg, insbesondere 75 bis 6.000 mg, besonders bevorzugt 100 bis 2.000 mg, 5-Aminosalicylsäure verabreicht werden. Insbesondere kann das Kombinationstherapeutikum zur Verabreichung einer Tagesdosis von (b2) 50 bis 10.000 mg, insbesondere 75 bis 6.000 mg, besonders bevorzugt 100 bis 2.000 mg, 5-Aminosalicylsäure hergerichtet sein.

Zudem kann - insbesondere im Hinblick auf die Behandlung von (B) entzündlichen Darmerkrankungen - das Kombinationstherapeutikum in Tagesdosen von (b2) 50 bis 1.500 µg, insbesondere 75 bis 1.000 µg, besonders bevorzugt 100 bis 800 µg, insbesondere systemisches Kortikosteroid verabreicht werden. Insbesondere kann das Kombinationstherapeutikum zur Verabreichung in einer Tagesdosis von (b2) 50 bis 1.500 µg, insbesondere 75 bis 1.000 µg, besonders bevorzugt 100 bis 800 µg, insbesondere systemisches Kortikosteroid hergerichtet sein.

Die vorgenannten Tagesdosen können sich in vorteilhafter Weise auf eine, zwei oder mehrere Einzelgaben verteilen. Zudem liegt es im Ermessen des Fachmanns, von den oben genannten Tagesdosen abzuweichen, insbesondere im Hinblick auf die zugrundeliegende Erkrankung und ihrer Schwere. Dies versteht sich für den Fachmann aber von selbst.

Was das erfindungsgemäße Kombinationstherapeutikum weiterhin anbelangt, so kann dieses, wie zuvor ausgeführt - insbesondere im Hinblick auf die Behandlung von (A) Atemwegserkrankungen - in einer peroral, insbesondere inhalativ applizierbaren Form, vorzugsweise als Suspension und/oder in Pulverform, beispielsweise als inhalatives Atemwegsspray oder Rachenspray vorliegen. Alternativ kann es erfindungsgemäß auch vorgesehen sein, dass wie zuvor angeführt, das Kombinationstherapeutikum nach der Erfindung in einer peroral applizierbaren Darreichungsform, insbesondere in Form von Kapseln, Pellets und/oder Tabletten, vorzugsweise in Form von Kapseln oder Pellets, bevorzugt Kapseln, vorliegt. Insbesondere kann das Kombinationstherapeutikum in Form einer peroralen, magensaftresistenten, aber dünndarmlöslichen Darreichungsform, insbesondere Kapseln und/oder Pellets, bevorzugt Kapseln, vorliegen. Die perorale Applizierung kommt insbesondere sowohl bei der Behandlung von (A) Atemwegserkrankungen als auch bei der Behandlung von (B) entzündlichen Darmerkrankungen, vorzugsweise jedoch bei (B) entzündlichen Darmerkrankungen, in Betracht, insbesondere vor dem Hintergrund, eine hohe Wirkstoffkonzentration am Wirkort zu erhalten.

Im Rahmen der vorliegenden Erfindung kann das Kombinationstherapeutikum nach der Erfindung zur prophylaktischen und/oder therapeutischen Behandlung, insbesondere Kombinationstherapie und/oder Co-Medikation, von (A) Atemwegserkran-kungen, insbesondere bronchopulmonalen Erkrankungen, und/oder zur prophylaktischen und/oder therapeutischen Behandlung, insbesondere Kombinationstherapie und/oder Co-Medikation, von (B) insbesondere entzündlichen Erkrankungen des Verdauungstrakts, insbesondere des Darms, eingesetzt werden.

In diesem Zusammenhang kann es erfindungsgemäß vorgesehen sein, dass das Kombinationstherapeutikum nach der Erfindung (a) den Vitamin D-Rezeptoragonisten (VDA) einerseits und (b) den weiteren Wirkstoff, insbesondere (b1) das topische, insbesondere inhalative Kortikosteroid bzw. (b2) 5-Aminosalicylsäure und/oder das systemische Kortikosteroid andererseits, gegebenenfalls gemeinsam mit dem weiteren pharmakologisch wirksamen Inhaltsstoff und/oder Wirkstoff, insbesondere wie zuvor definiert, und/oder dem weiteren Vitamin und/oder dem weiteren Inhaltsstoff, in einer gemeinsamen bzw. einzigen Zusammensetzung aufweist. Im Rahmen der vorliegenden Erfindung ist es in diesem Zusammenhang möglich, dass die in Rede stehende Zusammensetzung wiederum im Rahmen einer Co-Medikation mit weiteren Wirkstoffen verabreicht wird, insbesondere wie vorliegend angeführt.

Alternativ kann es gemäß einem weiteren Aspekt der vorliegenden Erfindung auch vorgesehen sein, dass das erfindungsgemäße Kombinationstherapeutikum, insbesondere wie zuvor definiert, bevorzugt zur Verwendung bei der prophylaktischen und/oder therapeutischen Behandlung von (A) Atemwegserkrankungen, umfassend - insbesondere in jeweils wirksamen, vorzugsweise pharmazeutisch wirksamen Mengen - mindestens einen Vitamin D-Rezeptoragonisten (VDA) einerseits und mindestens einen weiteren Wirkstoff andererseits, wobei der weitere Wirkstoff mindestens ein topisches, insbesondere inhalatives Kortikosteroid ist, in Form eines Kits (*"Kit of Parts"*) vorliegt bzw. (a) den Vitamin D-Rezeptoragonisten (VDA) einerseits und (b) den weiteren Wirkstoff, insbesondere (b1) das topische, insbesondere inhalative Kortikosteroid, andererseits räumlich voneinander getrennt, insbesondere in voneinander verschiedenen Zusammensetzungen, aufweist, jedoch mit der Maßgabe, dass das Kombinationstherapeutikum als solches keinen Inhalts- und/oder Wirkstoff aus der Gruppe von Terpenen, Monoterpenen, Vasokonstriktoren, alpha-Sympathomimetika und/oder deren physiologisch unbedenklichen Salzen oder Derivaten enthält.

Auch diesbezüglich kommen die jeweils zuvor beschriebenen Applikationsformen für die jeweiligen Komponenten, unabhängig voneinander, in Betracht. Beispielsweise kann es erfindungsgemäß vorgesehen sein, dass in nichtbeschränkender Weise der Vitamin D-Rezeptoragonist (VDA) in Form einer insbesondere dünndarmlöslichen Kapsel appliziert wird, während der (b) weitere Wirkstoff in Form eines inhalativen Sprühzusammensetzung oder dergleichen verabreicht wird.

Alternativ kann es zudem gemäß einem noch weiteren Aspekt der vorliegenden Erfindung vorgesehen sein, dass das erfindungsgemäße Kombinationstherapeutikum, insbesondere wie zuvor definiert, bevorzugt zur Verwendung bei der prophylaktischen und/oder therapeutischen Behandlung von (B) entzündlichen Darmerkrankungen, welches - insbesondere in jeweils wirksamen, vorzugsweise pharmazeutisch wirksamen Mengen - mindestens einen Vitamin D-Rezeptoragonisten (VDA) einerseits und mindestens einen weiteren Wirkstoff andererseits umfasst, wobei der weitere Wirkstoff 5-Aminosalicylsäure oder deren physiologisch unbedenkliche Salze oder Ester und gegebenenfalls mindestens ein insbesondere systemisches Kortikosteroid ist, in Form eines Kits (*"Kit of Parts"*) vorliegt und/oder (a) den Vitamin D-Rezeptoragonisten (VDA) einerseits und (b) den weiteren Wirkstoff, insbesondere (b2) 5-Aminosalicylsäure oder deren physiologisch unbedenkliche Salze oder Ester und gegebenenfalls das systemische Kortikosteroid andererseits, räumlich voneinander getrennt, insbesondere in voneinander verschiedenen Zusammensetzungen, aufweist, jedoch mit der Maßgabe, dass das Kombinationstherapeutikum keinen Inhalts- und/oder Wirkstoff aus der Gruppe von Terpenen, Monoterpenen, Vasokonstriktoren, alpha-Sympathomimetika und/oder deren physiologisch unbedenklichen Salzen oder Derivaten enthält.

Auch diesbezüglich kommen die jeweils zuvor beschriebenen Applikationsformen für die jeweiligen Komponenten, unabhängig voneinander, in Betracht. Beispielsweise kann der Vitamin D-Rezeptoragonist (VDA) einerseits, gegebenenfalls in Kombination mit dem insbesondere systemischen Kortikosteroid, in Form einer dünndarmlöslichen Tablette oder Kapsel eingesetzt werden, wohingegen der weitere Wirkstoff in Form von 5-Aminosalicylsäure in Form einer hiervon getrennten bzw. separaten Tablette bzw. Kapsel eingesetzt werden kann.

Im Rahmen einer Co-Medikation, beispielsweise mit Kortikosteroiden, kann es erfindungsgemäß in nicht beschränkender Weise vorgesehen sein, dass insbesondere für den Fall (A) der Atemwegserkrankungen einerseits der Vitamin D-Rezeptoragonist (VDA), insbesondere gemeinsam mit dem Kortikosteroid, lokal, insbesondere inhalativ (= *lokal applizierbare Komponenten),* beispielsweise in Form eines Sprays, verabreicht wird und andererseits - gemeinsam bzw. in Kombination hiermit oder aber zeitlich versetzt hierzu - mindestens ein weiteres systemisch applizierbares Atemwegstherapeutikum (= *topisch applizierbare Komponente)* insbesondere zu Zwecken der prophylaktischen und/oder therapeutischen Behandlung von Atemwegserkrankungen, insbesondere bronchopulmonalen Erkrankungen, vorabreicht wird.

Erfindungsgemäß kann es insbesondere für den Fall (D) der entzündlichen Darmerkrankung vorgesehen sein, dass einerseits der Vitamin D-Rezeptoragonist (VDA), insbesondere gemeinsam mit dem Kortikosteroid, systemisch, insbesondere peroral *(= erste systemisch applizierbare Komponenten),* beispielsweise in Form von dünndarmlöslichen Kapseln, verabreicht wird und andererseits - gemeinsam bzw. in Kombination hiermit oder aber zeitlich versetzt hierzu - mindestens ein systemisch applizierbares, insbesondere peroral appliziertes Darmtherapeutikum bzw. 5-Aminosalicylsäure (= *zweite systemisch applizierbare Komponente)* insbesondere zu Zwecken der prophylaktischen und/oder therapeutischen Behandlung von insbesondere entzündlichen Darmerkrankungen, verabreicht wird. Die ersten und zweiten systemisch applizierbaren Komponenten können auch in einer gemeinsamen bzw. einzigen Zusammensetzung vorliegen bzw. verabreicht werden.

Die erfindungsgemäßen Ausführungsformen sind jedoch in keiner Weise beschränkend und der Fachmann ist jederzeit in der Lage, die jeweils geeignete Applikationsform der jeweiligen Wirkkomponente auszuwählen.

Weiterhin betrifft die vorliegende Erfindung - gemäß einem noch weiteren Aspekt der vorliegenden Erfindung - die Verwendung eines Vitamin D-Rezeptoragonisten (VDA) zur Herstellung eines Arzneimittels zur prophylaktischen und/oder therapeutischen Behandlung von insbesondere entzündlichen Atemwegserkrankungen, insbesondere bronchopulmonalen Erkrankungen, wobei der Vitamin D-Rezeptoragonist (VDA) zusammen mit mindestens einem topischen, insbesondere inhalativen Kortikosteroid verabreicht und/oder eingesetzt wird, jedoch mit der Maßgabe, dass das Kombinationstherapeutikum keinen Inhalts- und/oder Wirkstoff aus der Gruppe von Terpenen, Monoterpenen, Vasokonstriktoren, alpha-Sympathomimetika und/oder deren physiologisch unbedenklichen Salzen oder Derivaten enthält.

Darüber hinaus betrifft die vorliegende Erfindung gleichermaßen - gemäß einem noch weiteren Aspekt der vorliegenden Erfindung - die Verwendung eines Vitamin D-Rezeptoragonisten (VDA) zur Herstellung eines Arzneimittels zur prophylaktischen und/oder therapeutischen Behandlung von insbesondere entzündlichen Erkrankungen des Verdauungstraktes, insbesondere des Darms, insbesondere wobei der Vitamin D-Rezeptoragonist (VDA) zusammen mit mindestens einem weiteren Wirkstoff eingesetzt wird, wobei der weitere Wirkstoff 5-Aminosalicylsäure oder deren physiologisch unbedenkliche Salze oder Ester und gegebenenfalls mindestens ein insbesondere systemisches Kortikosteroid im Fall der prophylaktischen und/oder therapeutischen Behandlung von (B) entzündlichen Dannerkrankungen ist, jedoch mit der Maßgabe, dass das Kombinationstherapeutikum keinen Inhalts- und/oder Wirkstoff aus der Gruppe von Terpenen, Monoterpenen, Vasokonstriktoren, alpha-Sympathomimetika und/oder deren physiologisch unbedenklichen Salzen oder Derivaten enthält.

Weiterhin betrifft die vorliegende Erfindung - gemäß einem wiederum weiteren Aspekt der vorliegenden Erfindung - die Verwendung eines Kombinationstherapeutikums, insbesondere wie zuvor definiert, und mindestens eines weiteren Therapeutikums zur prophylaktischen und/oder therapeutischen Behandlung, insbesondere Kombinationstherapie und/oder Co-Medikation, von insbesondere entzündlichen Atemwegserkrankungen, insbesondere bronchopulmonalen Erkrankungen und/oder zur prophylaktischen und/oder therapeutischen Behandlung, insbesondere Kombinationstherapie und/oder Co-Medikation, von insbesondere entzündlichen Erkrankungen des Verdauungstraktes, insbesondere des Darms,
wobei das Kombinationstherapeutikum - insbesondere in jeweils wirksamen, vorzugsweise pharmazeutisch wirksamen Mengen - umfasst:
(a) mindestens einen Vitamin D-Rezeptoragonisten (VDA) und
(b) mindestens einen weiteren Wirkstoff,
   (b1) wobei der weitere Wirkstoff mindestens ein topisches, insbesondere inhalatives Kortikosteroid im Fall der prophylaktischen und/oder therapeutischen Behandlung von (A) Atemwegserkrankungen ist oder
   (b2) wobei der weitere Wirkstoff 5-Aminosalicylsäure oder deren physiologisch unbedenkliche Salze oder Ester und gegebenenfalls mindestens ein insbesondere systemisches Kortikosteroid im Fall der prophylaktischen und/oder therapeutischen Behandlung von (B) entzündlichen Darmerkrankungen ist;
jedoch jeweils mit der Maßgabe, dass das Kombinationstherapeutikum keinen Inhalts- und/oder Wirkstoff aus der Gruppe von Terpenen, Monoterpenen, Vasokonstriktoren, alpha-Sympathomimetika und/oder deren physiologisch unbedenklichen Salzen oder Derivaten enthält.

Erfindungsgemäß können, wie zuvor ausgeführt, die jeweiligen Wirkstoffkomponenten zumindest teilweise voneinander räumlich getrennt vorliegen bzw. verabreicht werden bzw. räumlich getrennt von dem weiteren Therapeutikum vorliegen bzw. getrennt verabreicht werden. In diesem Zusammenhang kann insbesondere auch eine zeitlich voneinander unabhängige Verabreichung der jeweiligen Wirkstoffe bzw. Therapeutika vorgenommen werden, wobei die diesbezügliche konkrete Ausgestaltung im Hinblick auf die zugrundeliegende Indikation und Schwere der Erkrankung vom Fachmann ohne Weiteres bestimmt werden kann.

Erfindungsgemäß kann das weitere Therapeutikum, insbesondere im Fall der prophylaktischen und/oder therapeutischen Behandlung von (A) Atemwegserkrankungen, ein topisch applizierbares Atemwegstherapeutikum, insbesondere ein inhalatives Atemwegstherapeutikum, sein.

Insbesondere kann das topisch applizierbare, insbesondere inhalative Atemwegstherapeutikum ausgewählt sein aus der Gruppe von Bronchodilatatoren und Bronchospasmolytika. Insbesondere kann das topisch applizierbare, insbesondere inhalative Atemwegstherapeutikum ausgewählt sein aus der Gruppe von (i) Kortikosteroiden, insbesondere Glukokortikoiden; (ii) Sympathomimetika, insbesondere beta-Sympathomimetika, vorzugsweise beta-2-Sympathomimetika; (iii) Phosphodiesterasehemmern; (iv) Parasympatholytika und/oder Vagolytika; (v) Anticholinergika; sowie Mischungen und Kombinationen der vorgenannten Verbindungen, und besonders bevorzugt ausgewählt sein aus der Gruppe von Kortikosteroiden, insbesondere Glukokortikoiden, beta-2-Sympathomimetika und Anticholinergika sowie deren Mischungen und Kombinationen.

Gemäß einer erfindungsgemäß bevorzugten Ausführungsform kann das topisch applizierbare, insbesondere inhalative Atemwegstherapeutikum ein Kortikosteroid, insbesondere Glukokortikoid, sein, vorzugsweise ausgewählt aus der Gruppe von Beclometason, Mometason, Budesonid, Flunisolid, Fluticason, Triamcinolon und deren physiologisch verträglichen Derivaten, insbesondere Salzen und Estern, sowie Mischungen und Kombinationen der vorgenannten Verbindungen. In diesem Zusammenhang kann das Kortikosteroid in Tagesdosen im Bereich von 50 bis 1.000 µg, insbesondere 75 bis 800 µg, besonders bevorzugt 100 bis 600 µg, verabreicht werden. Insbesondere kann das Kombinationstherapeutikum zur Verabreichung des Kortikosteroids in einer Tagesdosis von 50 bis 1.000 µg, insbesondere 75 bis 800 µ*g, besonders bevorzugt 100 bis 600 µg, hergerichtet sein.

Gemäß einer weiteren erfindungsgemäßen Ausführungsform kann das topisch applizierbare, insbesondere inhalative Atemwegstherapeutikum ein beta-Sympathomimetikum, vorzugsweise beta-2-Sympathomimetikum, sein, insbesondere ausgewählt aus der Gruppe von kurzwirkenden Betamimetika (SABA), insbesondere Albuterol, Fenoterol, Hexoprenalin, Levalbuterol, Metaproterenol, Orciprenalin, Pirbuterol, Reproterol, Salbutamol und/oder Terbutalin, und langwirkenden Betamimetika (LABA), insbesondere Salmeterol und/oder Formoterol.

Der Begriff der Sympathomimetika kennzeichnet solche Substanzen, welche die Wirkung des Sympathikus nachahmen. Speziell die beta-Sympathomimetika (synonym auch als "Betamimetika" bezeichnet), besitzen überwiegend Wirkung auf beta-Rezeptoren. Ganz speziell die erfindungsgemäß eingesetzten beta-2-Sympathomimetika führen an der glatten Muskulatur (beta-2-Rezeptoren) zur Erschlaffung und besitzen bronchospasmolytische Wirkungen. Bei den erfindungsgemäß bevorzugt eingesetzten inhalativen beta-2-Sympathomimetika kann es sich entweder um kurzwirkende Betamimetika (SABA = Short Acting Beta-2 Agonist) oder um langwirkende Betamimetika (LABA = Long Acting Beta-2 Agonist) handeln. Beispiele für kurzwirkende Betamimetika (SABA) sind insbesondere Albuterol, Fenoterol, Hexoprenalin, Levalbuterol, Metaproterenol, Orciprenalin, Pirbuterol, Reproterol, Salbutamol und/oder Terbutalin. Beispiele für langwirkende Betamimetika (LABA) sind insbesondere Salmeterol und/oder Formoterol.

Gemäß einer weiteren erfindungsgemäßen Ausführungsform kann es vorgesehen sein, dass das topisch applizierbare, insbesondere inhalative Atemwegstherapeutikum ein Anticholinergikum ist, insbesondere aus der Gruppe von Ipratropium, Tiotropium und/oder deren physiologisch verträglichen Derivaten, vorzugsweise Salzen, besonders bevorzugt Ipratropiumbromid und/oder Tiotropiumbromid, sowie Mischungen und Kombinationen der vorgenannten Verbindungen.

Anticholinergika sind solche Substanzen, welche die Wirkung von Acetylcholin unterdrücken, wobei die in Rede stehenden Substanzen gleichermaßen bronchospasmolytische Wirkungen aufweisen.

Gleichermaßen kann es erfindungsgemäß vorgesehen sein, dass mindestens ein weiterer, systemischer, insbesondere peroraler Wirkstoff appliziert wird, insbesondere ausgewählt aus der Gruppe von systemischen Phosphodiesterasehemmern, insbesondere Theophyllin; systemischen Leukotrienrezeptorantagonisten, insbesondere Montelukast, Zaforlukast und Pranlukast; systemischen Kortikosteroiden; Antiphlogistika, insbesondere nichtsteroidalen Antiphlogistika, vorzugsweise Acetylsalicylsäure und/oder Paracetamol; sowie deren Mischungen und Kombinationen.

Im Rahmen der vorliegenden Erfindung kann es darüber hinaus vorgesehen sein, dass das weitere Therapeutikum, insbesondere im Fall der prophylaktischen und/oder therapeutischen Behandlung von (B) entzündlichen Darmerkrankungen, ein systemisch applizierbares Therapeutikum, insbesondere ein peroral applizierbares Therapeutikum, ist, insbesondere wobei das Therapeutikum mindestens ein systenisch applizierbares, vorzugsweise peroral applizierbares Kortikosteroid, insbesondere Glukokortikosteroid, aufweist, insbesondere wobei das Glukokortikosteroid ausgewählt ist aus der Gruppe von Dexamethason, Prednison, Prednisolon, Betamethason, Rimexolon, Paramethason, Hydrocortison und deren physiologisch verträglichen Derivaten, insbesondere Salzen und Estern, sowie Mischungen und Kombinationen der vorgenannten Verbindungen.

Die Verabreichung derartiger systemischer Kortikoide kommt insbesondere im Rahmen der Behandlung von entzündlichen Erkrankungen des Verdauungstraktes, insbesondere des Darms, in Betracht, ist jedoch nicht hierauf beschränkt, da die Verabreichung systemischer Kortikoide grundsätzlich auch bei der Behandlung von bronchopulmonalen Erkrankungen in Betracht kommt.

Im Rahmen der erfindungsgemäßen Konzeption können beliebige bronchopulmonale Erkrankungen bzw. Atemwegserkrankungen behandelt werden. Insbesondere ist die Atemwegserkrankung, insbesondere die bronchopulmonalen Erkrankung eine entzündliche oder nichtentzündliche, insbesondere entzündliche Erkrankung der oberen oder unteren Atemwege. Insbesondere kann es sich bei der bronchopulmonalen Erkrankung bzw. Atemwegserkrankung um eine entzündliche, insbesondere infektexazerbierte bzw. steroidpflichtige Atemwegserkrankung handeln.

Beispielsweise kann es sich bei der Atemwegserkrankung, insbesondere bronchopulmonalen Erkrankung, um Asthma bronchiale oder Bronchitis handeln.

Weiterhin kann die Atemwegserkrankung, insbesondere die bronchopulmonale Erkrankung, eine chronische obstruktive Lungenerkrankung (COPD) sein, insbesondere eine chronische obstruktive Bronchitis oder ein Lungenemphysem.

Des Weiteren kann die Atemwegserkrankung, insbesondere die bronchopulmonale Erkrankung, eine tabakrauchinduzierte, insbesondere nikotininduzierte akute oder chronische Atemwegserkrankung, insbesondere entzündliche Atemwegserkrankung, sein.

Im Rahmen der erfindungsgemäßen Konzeption können auch Frühformen von COPD, insbesondere im Stadium nach GOLD 0 oder I, oder eine Frühform des Asthma bronchiale, insbesondere im Stadium 0 oder I nach GINA 0 oder I, sein. Insbesondere können im Rahmen der vorliegenden Erfindung Frühformen von COPD, insbesondere im Stadium 0 oder I nach GOLD, oder Frühformen des Asthma bronchiale, insbesondere im Stadium nach GINA 0 oder I behandelt werden, und auf diese Weise kann eine Exazerbationprophylaxe vor und nach erfolgter Exazerbation bzw. eine Verhinderung und Verlangsamung des Krankheitsprogresses vor oder nach erfolgter Exazerbation erreicht werden.

Darüber hinaus kann die Atemwegserkrankung eine Erkältungserkrankung oder ein grippaler Infekt sein. Gleichermaßen kann die Atemwegserkrankung Rhinitis und/oder Sinusitis sein.

Gemäß einer alternativen erfindungsgemäßen Ausführungsform kann die entzündliche Erkrankung des Verdauungstrakes, insbesondere des Darms, *Morbus Crohn* und/oder *Colitis ulcerosa* und/oder eine chronisch entzündliche Darmerkrankung (*Inflammatory Bowel Disease,* IBD) sein.

Das erfindungsgemäßen Kombinationstherapeutikum eignet sich darüber hinaus auch zur insbesondere synergistischen Wirkungssteigerung mindestens eines topisch applizierbaren, insbesondere inhalativen Atemwegstherapeutikums bei der prophylaktischen und/oder therapeutischen Behandlung von Atemwegserkrankungen, insbesondere bronchopulmonalen Erkrankungen.

Darüber hinaus eignet sich das erfindungsgemäße Kombinationstherapeutikum auch zur insbesondere synergistischen Steigerung der antiinflammatorischen und/oder antioxidativen Wirkung topischer, insbesondere inhalativer Kortikosteroide, insbesondere Glukokortikoide.

Gleichermaßen eignet sich das Kombinationstherapeutikum nach der Erfindung zur Dosisreduktion von topisch applizierbaren, insbesondere inhalativen Atemwegstherapeutika, vorzugsweise inhalativen Kortikosteroiden, bei der prophylaktischen und/oder therapeutischen Behandlung von Atemwegserkrankungen, insbesondere bronchopulmonalen Erkrankungen.

Gemäß einer weiteren erfindungsgemäßen Ausführungsform eignet sich das Kombinationstherapeutikum nach der Erfindung auch zur Induktion und/oder Verstärkung des steroidpermissiven Effektes von topisch applizierbaren, insbesondere inhalativen Atemwegstherapeutika, vorzugsweise inhalativen Kortikosteroiden, bei der prophylaktischen und/oder therapeutischen Behandlung von Atemwegserkrankungen, insbesondere bronchopulmonalen Erkrankungen.

Zudem eignet sich das erfindungsgemäße Kombinationstherapeutikum auch zur Vermeidung oder Reduktion eines Gewöhnungseffektes von beta-Sympathomimetika bei der prophylaktischen und/oder therapeutischen Behandlung von Atemwegserkrankungen, insbesondere bronchopulmonalen Erkrankungen, insbesondere unter Dauertherapie bei allen Schweregraden von COPD und Asthma bronchiale.

Zudem eignet sich das erfindungsgemäße Kombinationstherapeutikum nach der Erfindung auch in Kombination mit mindestens einem topisch applizierbaren, insbesondere inhalativen Atemwegstherapeutikum, insbesondere in Kombination mit einem inhalativen Kortikosteroid, zur Reduktion des Bedarfs oder zum Ersatz systemischer Kortikosteroide oder anderer antiinflammatorisch und/oder immunsuppressiv wirkender systemischer Substanzen bei der prophylaktischen und/oder therapeutischen Behandlung von Atemwegserkrankungen, insbesondere bronchopulmonalen Erkrankungen.

Gemäß einer noch weiterführenden erfindungsgemäßen Ausführungsform eignet sich das Kombinationstherapeutikum nach der Erfindung auch in Kombination mit mindestens einem topisch applizierbaren, insbesondere inhalativen Atemwegstherapeutikum, insbesondere in Kombination mit einem inhalativen Kortikosteroid und gegebenenfalls einem inhalativen beta-2-Sympathomimetikum, zur Optimierung der Basistherapie bei Asthma bronchiale und COPD.

Insbesondere betrifft die vorliegende Erfindung auch die Behandlung der systemischen Organbeteiligung bei sämtlichen Schwereformen von COPD.

Insbesondere eignet sich das erfindungsgemäße Kombinationstherapeutikum auch zur Modulation und Hemmung COPD-abhängiger und/oder COPD-unabhängiger Alterungsprozesse, insbesondere mit dem Ziel der Reduktion von Morbidität, der Erhöhung der Lebensqualität und/oder Lebenserwartung.

Insbesondere eignet sich das erfindungsgemäße Kombinationstherapeutikum auch zur kombinierten antiinflammatorischen und/oder antioxidativen Therapie von persistierenden und/oder progredienten Atemwegserkrankungen, insbesondere entzündlichen Atemwegserkrankungen, nach Aufgabe des Rauschens, gegebenenfalls unter Co-Medikation mit anderen Atemwegstherapeutika, insbesondere mit dem Ziel der Retardierung der Emphysementwicklung, der respiratorischen Insuffizienz und/oder der Entwicklung peripherer Atemwegsobstruktionen.

Darüber hinaus eignet sich das erfindungsgemäße Kombinationstherapeutikum nach der Erfindung auch zur Co-Medikation mit insbesondere nasal-topischen Glukokortikosteroiden, insbesondere zur Intensivierung und/oder Verbesserung der Wirksamkeit und/oder zur Wirkeffizienzsteigerung von Leitlinientherapien für obere Atemwegserkrankungen, insbesondere der akuten, allergischen, chronischen und/oder chronisch rezidivierenden polypoiden Rhinitis und/oder Rhinosinusitis.

Zudem eignet sich das Kombinationstherapeutikum zur Verwendung bei der Co-Medikation zur Intensivierung und/oder Verbesserung der Wirksamkeit und/oder zur Wirkeffizienzsteigerung von Leitlinientherapien, insbesondere auf Basis topischnasaler Steroide und/oder alpha-1-Sympathomimetika, für Erkrankungen der oberen Atemwege, insbesondere akuter, allergischer und/oder chronischer Rhinitis und/oder Rhinosinusitis, und/oder zur Verwendung bei der zur Co-Medikation zur Intensivierung und/oder Verbesserung der Wirksamkeit und/oder zur Wirkeffizienzsteigerung von Leitlinientherapien, insbesondere auf Basis von LABA, ICS (inhalatives Kortikosteroid), LAMA (long acting muscarinic antagonist), LABA in Kombination mit ICS, LABA und LAMA, LABA in Kombination mit ICS und LAMA), für Erkrankungen, der unteren Atemwege, insbesondere Asthma, COPD und/oder chronische Lungenemphyseme, durch Zigarettenrauch oder Feinpartikel oder andere Umweltnoxen induzierte und/oder assoziierte Atemwegserkrankungen, insbesondere mit dem Ziel einer Induktion und/oder Verstärkung von steroidpermissiven Effekten, insbesondere um den progressiven Verlauf von Obstruktionen und/oder Emphysemen, Exazerbationen und/oder den Steroidbedarf zu reduzieren. Insbesondere kann das Kombinationstherapeutikum in diesem Fall als dünndarmlösliche Kapsel vorliegen.

Gleichermaßen eignet sich das erfindungsgemäße Kombinationstherapeutikum zur Verwendung bei der Co-Medikation und/oder als Zusatztherapie insbesondere in Form eines Kits, mit mindestens einem weiteren Wirkstoff, wobei der Wirkstoff ausgewählt ist aus der Gruppe von systemischen Glukokortikosteroiden (SCS), intravenösen Substitutionstherapien mit alpha-1-Antitrypsin, IgG-Immunglobulinen, insbesondere bei AK-Mangelsyndrom, Phosphodiesteraseinhibitoren, insbesondere Roflumilast und/oder Theophyllin, zur prophylaktischen und/oder therapeutischen Behandlung von Erkrankungen der oberen Atemwege, insbesondere akuter, allergischer und/oder chronischer Rhinitis und/oder Rhinosinusitis, und/oder zur prophylaktischen und/oder therapeutischen Behandlung von Erkrankungen der unteren Atemwege, insbesondere Asthma, COPD und/oder chronischen Lungenemphysemen, und/oder zur prophylaktischen und/oder therapeutischen Behandlung von durch Zigarettenrauch, Feinpartikel und/oder durch andere Umweltnoxen induzierten und/oder assoziierten Atemwegserkrankungen, insbesondere mit dem Ziel, die Therapieeffekte von Leitlinientherapien, wie zuvor definiert, zu intensivieren und/oder zu verbessern, und/oder mit dem Ziel einer Induktion und/oder Verstärkung von steroidpermissiven Effekten, insbesondere um den progressiven Verlauf von Obstruktionen und/oder Emphysemen, Exazerbationen und/oder den Steroidbedarf zu reduzieren. Diesbezüglich kann auch eine Substitutionstherapie mit alpha-1-Antitrypsin bei einer Applikation des Vitamin D-Rezeptoragonisten (VDA) von etwa 250 mg bis 375 mg pro Woche in Betracht kommen.

Weiterhin eignet sich das erfindungsgemäße Kombinationstherapeutikum zur Kompensation niedriger Wirkstoffspiegel von Vitamin D, insbesondere wobei das Kombinationstherapeutikum als Kit vorliegt, für die Prophylaxe, Therapie und/oder Retardierung des progressiven Verlaufs bei insbesondere schweren entzündlichen Darmerkrankungen, insbesondere chronisch-aktiven entzündlichen Darmerkrankungen, und/oder für die Reduktion von Exazerbationen, schwerer COPD, insbesondere des Lungenemphysems gegebenenfalls mit oder ohne respiratorischer Insuffizienz und/oder schwerem Asthma.

In diesem Zusammenhang eignet sich das erfindungsgemäße Kombinationstherapeutikum auch zur Erhöhung der körpereigenen Vitamin D-Synthese bzw. zu einer Erhöhung des körpereigenen Vitamin D-Spiegels, insbesondere durch orale Dosierung von dünndarmlöslichen Kapseln.

Darüber hinaus eignet sich das erfindungsgemäße Kombinationstherapeutikum auch zur Verwendung bei der prophylaktischen oder therapeutischen Behandlung von oberen Atemwegsobstruktionen sowie von profibrotischen Entzündungsreaktionen, insbesondere bei schlafbezogenen Atmungsstörungen, insbesondere des obstruktiven und zentralen Schlafapnoesyndroms. Auch diesbezüglich kommen insbesondere dünndarmlösliche Kapseln des erfindungsgemäßen Kombinationstherapeutikums in Betracht.

Gleichermaßen eignet sich das erfindungsgemäße das Kombinationstherapeutikum zur Intensivierung und/oder Verbesserung der Wirksamkeit und/oder zur Wirkeffizienzsteigerung von Leitlinientherapien von chronisch entzündlichen Darmerkrankungen, insbesondere des Reizdarmsyndroms, *Morbus Crohn* und *Colitis ulcerosa,* insbesondere mit dem Ziel der Prophylaxe, insbesondere Exazerbationsprophylaxe, Remissionserhaltung und Erhöhung der Entzündungshemmung bei Kindern und Erwachsenen, insbesondere wobei das Kombinationstherapeutikum als Kit vorliegt.

Insbesondere besteht ein Fokus des erfindungsgemäßen Kombinationstherapeutikums in der Reduktion akuter, infektiöser, allergisch oder chronisch bedingter Rezidivhäufungen mit Besserung der Therapierefraktärität durch eine kombinierte Behandlung von Lokal- und/oder Systementzündung insbesondere bei Reizdarmsyndromen, insbesondere bei entzündlichen Darmerkrankungen, wie *Inflammatory Bowel Disease.*

Weiterhin eignet sich das erfindungsgemäße Kombinationstherapeutikum zur Inhalation, insbesondere in Verbindung mit ICS, LABA, LAMA, LABA in Kombination mit ICS sowie LABA in Kombination mit ICS und LAMA, insbesondere mit dem Ziel der Verbesserung des Therapieeffekts von insbesondere topisch eingesetzten Leitlinientherapien für Erkrankungen der unteren Atemwege. In diesem Zusammenhang kann das Kombinationstherapeutikum nach der Erfindung als Kit vorliegen, insbesondere in Form eines Kits zur lokalen Applikation.

Weiterhin eignet sich das erfindungsgemäße Kombinationstherapeutikum auch zur lokalen, systemischen und/oder synergistischen Verstärkung von Leitlinientherapien von entzündlichen Erkrankung des Verdauungstraktes, insbesondere des Darms, vorzugsweise mit Befall des Dick- und/oder Dünndarms, wie chronisch entzündlichen Darmerkrankungen (*Inflammatory Bowel Disease,* IBD), vorzugsweise *Morbus Crohn* und/oder *Colitis ulcerosa,* insbesondere mit dem Ziel, akute, infektiöse, allergische und/oder chronisch bedingte Rezidivhäufungen zu reduzieren und/oder insbesondere mit dem Ziel der Besserung der Therapierefraktärität, vorzugsweise durch kombinierte Behandlung von Lokal- und Systementzündungen insbesondere bei IBD. In diesem Zusammenhang sollte das Kombinationstherapeutikum nach der Erfindung als Kit vorliegen, insbesondere in Form von dünndarmlöslichen Kapseln. Das erfindungsgemäße Kombinationstherapeutikum eignet sich insbesondere zur Dosisreduktion des inhalativen Kortikosteroids im Fall der (A) Atemwegserkrankungen, und/oder zur Dosisreduktion von 5-Aminosalicylsäure und/oder des insbesondere systemischen Kortikosteroids im Fall der (B) entzündlichen Darmerkrankungen und/oder zur Dosisreduktion mindestens eines weiteren pharmakologischen Wirk- und/oder Inhaltsstoffs, insbesondere wie zuvor definiert, und/oder zur Dosisreduktion mindestens eines systemisch oder topisch applizierbaren pharmakologischen Wirkstoffs, insbesondere wie zuvor definiert, vorzugsweise eines Kortikosteroids. In diesem Zusammenhang kann die zu verabreichende Menge und/oder Dosis des jeweiligen Wirkstoffs, unabhängig voneinander, insbesondere nach einem definierten Anwendungszeitraum des erfindungsgemäßen Kombinationstheraputikums, z. B. nach drei Wochen, insbesondere nach zwei Wochen, vorzugsweise nach einer Woche, um mindestens 5 %, insbesondere mindestens 10 %, vorzugsweise mindestens 20 %, bevorzugt mindestens 40 %, bezogen auf die ein entsprechendes Therapeutikum ohne Vitamin D-Rezeptoragonist (VDA), verringert werden.

Im Rahmen der erfindungsgemäßen Konzeption kann Vitamin D, insbesondere Calcidiol und/oder Calcitriol, vorzugsweise Calcidiol, als spezielle Medikation in Form des erfindungsgemäß definierten Kombinationstherapeutikums eingesetzt werden, insbesondere auch in Form einer Co-Medikation zusammen mit einer Leitlinientherapie in verschiedenen Kombinationen, insbesondere mit dem Ziel, die eigenständige antiinflammatorische Wirkung von Vitamin D in Kombination mit verschiedenen Leitlinientherapeutika auch unabhängig von einem Vitamin D-Mangel zur Verstärkung der Steroidwirkung von Leitlinientherapeutika, wie SABA, LABA, ICS oder LAMA, einzusetzen. Insbesondere kann auf dieser Basis auch der Bedarf an Steroiden gesenkt werden bzw. kann eine Alternative zur Therapie mit Steroiden bereitgestellt werden.

Erfingdungsgemäß können zudem insbesondere folgende Behandlungskonzepte bereitgestellt werden: Behandlung der unteren Atemwege durch Inhalation von Vitamin D und topisches Kortikosteroid mit LABA, insbesondere Formoterol; Vitamin D und topisches Kortikosteroid, insbesondere Budesonid; Vitamin D und topisches Kortikosteroid mit LABA und ICS; Vitamin D und topisches Kortikosteroid mit LAMA; Vitamin D und topisches Kortikosteroid mit LABA und/oder LAMA; Vitamin D und topisches Kortikosteroid mit weiterem ICS und/oder LABA und/oder LAMA; insbesondere auch zur Verstärkung einer steroidartigen entzündungshemmenden Wirkung, mit dem Ziel, Steroide zu reduzieren, steroidale Nebenwirkungen und eine gewisse krankheitsassoziierte Steroidresistenz zu überwinden.

Erfindungsgemäß können zudem insbesondere folgende Behandlungskonzepte bereitgestellt werden: Systemische Behandlung der oberen und unteren Atemwege mit Vitamin D und topischem Kortikosteroid mit Montelukast oder einem anderen Leukotrien-Rezeptorantagonisten oder -Inhibitor; Vitamin D mit topischem Kortikosteroid mit Prednsiolon, Vitamin D mit topischem Kortikosteroid mit anti-Immunglobulin E (z. B. Omalizumab); Vitamin D mit topischem Kortikosteroid mit Immunsuppressiva; Vitamin D und topischem Kortikosteroid mit Antirheumatika, insbesondere zur Verstärkung einer steroidartigen entzündungshemmenden Wirkung, mit dem Ziel, Steroide zu reduzieren, steroidale Nebenwirkungen und eine gewisse krankheitsassoziierte Steroidresistenz zu überwinden.

Erfindungsgemäß können zudem insbesondere noch folgende Behandlungskonzepte bereitgestellt werden: Erzielung einer antibiotischen und entzündungshemmmenden Wirkung durch die Kombination von Vitamin D und Kortikosteroid mit Antibiotika.

Erfindungsgemäß können zudem insbesondere auch folgende Behandlungskonzepte bereitgestellt werden: Intensivierung der antientzündlichen Leitlinientherapie bei entzündlichen Darmerkrankungen auf Basis einer Kombination von Vitamin D mit 5-ASA; Vitamin D mit 5-ASA und Budesonid.

Weitere Ausgestaltungen, Abwandlungen und Variationen sowie Vorteile der vorliegenden Erfindung sind für den Fachmann beim Lesen der Beschreibung ohne Weiteres erkennbar und realisierbar, ohne dass er dabei den Rahmen der vorliegenden Erfindung verlässt.

Die folgenden Ausführungsbeispiele dienen lediglich der Veranschaulichung der vorliegenden Erfindung, ohne sie jedoch hierauf zu beschränken.

### Ausführungsbeispiele und

### weitere Ausführungen zur vorliegenden Erfindung:

### Methodik

Im Rahmen der vorliegenden Erfindung wurde der Einfluss der entzündungshemmenden Wirkung von Calcidiol und Calcitriol sowie gegebenenfalls verschiedenen weiteren Testsubstanzen in Kulturen von normalen humanen Monozyten *in vitro* untersucht. Als Maß zur Bestimmung der entzündungshemmenden Wirkung wurde die Stärke der Hemmung der Produktion des Tumornekrosefaktor-alpha (TNF-alpha), repräsentativ für die mit entzündlichen Prozessen assoziierte Zytokinproduktion, in humanen Monozyten bestimmt, Die Produktion von TNF-alpha kann in humanen Monozyten gezielt durch die Zugabe von Lipopolysacchariden (LPS) stimuliert werden. Je stärker die entzündungshemmende Wirkung einer Testsubstanz ist, desto weniger TNF-algha wird durch die mit LPS stimulierten Monozyten produziert. Im Rahmen der erfindungsgemäßen Versuche wurde neben Calcidiol und Calcitriol als weitere Testsubstanz 5-Aminosalicylsäure bzw. ein Kortikosteroid eingesetzt.

Im Rahmen der Untersuchungen wurden zunächst humane Monozyten durch standardisierte und dem Fachmann grundsätzlich bekannte Methoden (Dichtegradientenzentrifugation oder magnetische Zellseparation unter Einsatz von *MicroBeads^{®}*) aus venösem Blut von Spendern isoliert. Anschließend wurden die gereinigten und hochvitalen Monozyten (95 %) in 48-Loch Kulturplatten (10⁵/ml) in Gegenwart der Testsubstanzen inkubiert. Als Kontrollen bzw. Vergleichsversuche wurden Inkubationen der Monozyten in Gegenwart von Calcidiol bzw. Calcitriol sowie der weiteren Testsubstanzen jeweils alleine durchgeführt. Die Inkubation der Monozyten mit Calcidiol bzw. Calcitriol diente darüber hinaus dem generellen Nachweis von deren entzündungshemmender Wirkung. Darüber hinaus wurden Co-Inkubationen von Calcitriol bzw. Calcidiol in Kombination mit weiteren Testsubstanz durchgeführt.

Während der Inkubation mit den Testsubstanzen erfolgte die Stimulation der Produktion von TNP-alpha (Tumornekrosefaktor-alpha) über einen Zeitraum von 20 Stunden mit Lipopolysacchariden (10 µg/ml LPS). Anschließend wurden die Überstände gewonnen und bis zur weiteren Analyse bei -80 °C gelagert. Zu Analysezwecken wurden die jeweiligen Kulturüberstände einem ELISA *(Enzyme-Linked Immunosorbent Assay)* unterzogen, dessen Durchführung dem Fachmann an sich bekannt ist, wobei die durch die Monozyten produzierte Menge an TNF-alpha gemessen wurde.

Bei den nachfolgend detailliert dargestellten Ergebnissen bzw. Untersuchungen sind die prozentualen Hemmwirkungen von Calcidiol bzw. Calcitriol, gegebenenfalls mit weiteren Testsubstanzen, auf die Produktion von TNF-alpha durch humane, mit LPS stimulierte Monozyten, im Vergleich zur Produktion von TNF-alpha durch Monozyten, welche durch LPS stimuliert, allerdings nicht in Gegenwart von Testsubstanzen inkubiert wurden, angegeben. Die Ergebnisse sind dargestellt als Hemmung in Prozent der LPS-induzierten Kontrolle ohne Wirkstoff aus gepoolten Messungen von mindestens zwei bis vier Experimenten (n = 8 bis 12). Für statistische Analysen wurde der nichtparametrische Mann & Whitney Test herangezogen, insbesondere vor dem Hintergrund der Ermittlung einer Signifikanz im Vergleich zur LPS-Kontrolle, dem alleinigen Einsatz von Vitamin D₃ bzw. von Calcidiol bzw. Calcitriol, sowie dem alleinigen Einsatz der Testsubstanz. Ab einem p-Wert von < 0,05 gegenüber der jeweiligen Kontrolle bzw. dem jeweiligen Vergleichsansatz wird im Rahmen der vorliegenden Erfindung die Hemmwirkung als signifikant angesehen.

### a) Calcidiol bzw. Calcitriol als Entzündungshemmer

Zum Nachweis der entzündungshemmenden Wirkung von Calcidiol bzw. Calcitriol wurde der Einfluss auf die LPS-stimulierte Produktion von TNF-alpha durch humane Monozyten untersucht. Dazu wurde Calcidiol bzw. Calcitriol in jeweils separaten Experimentreihen in Konzentrationen von jeweils 0,1 ng/ml, 1 ng/ml, 10 ng/ml, 30 ng/ml, 50 ng/ml und 100 ng/ml eingesetzt. Die in Prozent angegebene Hemmung der TNF-alpha Produktion durch Calcitriol bzw. Calcidiol bezieht sich dabei auf die Menge an TNF-alpha, welche durch mit LPS stimulierte Monozyten ohne den Einsatz von Hemmstoffen produziert wurde.

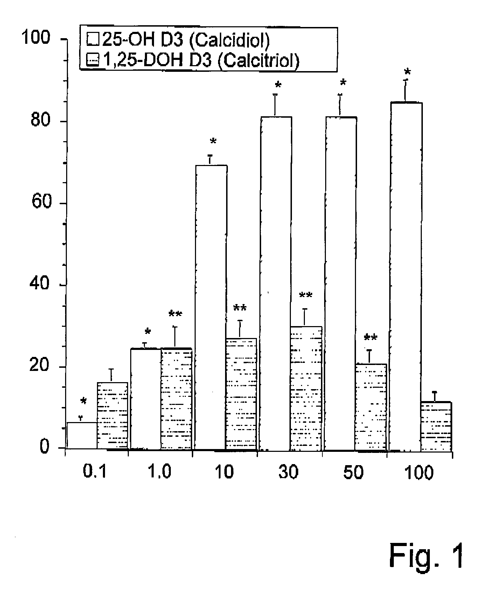

Die Ergebnisse zeigen überraschenderweise sowohl für Calcidiol als auch Calcitriol eine signifikante Hemmung der Produktion von TNF-alpha, wobei die durch Calcidiol induzierte Hemmung diejenige von Calcitriol noch übersteigt. Die durch Calcidiol induzierte Hemmung von TNF-alpha variierte im Bereich von - 6,5 ± 1 % bei 0,1 ng/ml bis - 85,7 ± 5 % bei 100 ng/ml bei einem p-Wert von ≤ 0,0117. Somit kann die Hemmung der Produktion von TNF-alpha als signifikant angesehen werden. Mit Calcitriol konnte ebenfalls eine Hemmung von TNF-alpha erzielt werden, wobei diese mit Werten im Bereich von - 24,8 ± 5 % für 1 ng/ml und - 21,4 ± 3 % für 50 ng/ml mit einem Maximalwert der Hemmung von - 30,5 ± 4 % bei Einsatz von 30 ng/ml Calcitriol geringer ausfiel als die durch Calcidiol induzierte Hemmung. Die entsprechenden Ergebnisse sind Fig. 1 dargestellt, wobei die x-Achse die Konzentration an eingesetztem Calcidiol bzw. Calcitriol und die y-Achse den jeweiligen Mittelwert mit Angabe der Standardabweichung der prozentualen Hemmung im Vergleich zu der TNF-alpha-Kontrolle darstellt (mit *p = 0,0008 und **p < 0,017). Fig. 1 zeigt somit, dass im therapeutischen Konzentrationsbereich von Calcidiol (n = 8, 2 Experimente) und von Calcitriol (n = 8, 4 Experimente) die LPS-stimulierte Produktion von TNF-alpha in normalen Monozyten im Vergleich zur LPS-Kontrolle signifikant gehemmt wird.

Insgesamt konnte gezeigt werden, dass Vitamin D in seiner aktiven Form, insbesondere in Form von Calcidiol, aber auch in Form von Calcitriol, über eine eigenständige entzündungshemmende Wirkung verfügt. Im Gegensatz zu Calcidiol fällt die durch Calcitriol induzierte Hemmung von TNF-alpha bei Konzentrationen im Bereich von 20 bis 55 ng/ml wieder leicht ab. Steigende Calcitriol-Konzentrationen sind also insgesamt mit einem gewissen Rückgang der entzündungshemmenden Wirkung verbunden. Ohne sich hierbei auf diese Theorie beschränken zu wollen, spricht die abnehmende Entzündungshemmung bei höheren Konzentrationen von Calcitriol für eine Downregulation bzw. Herunterregulation des Vitamin D-Rezeptors durch höhere Konzentrationnen an Calcitriol, so dass Calcitriol gewissermaßen imstande ist, seinen eigenen Rezeptor herunterzuregulieren.

Im Unterschied hierzu ist die Expression des Vitamin D-Rezeptors bei Vitamin D-Mangelsituationen induzierbar und wird durch eine Supplementierung oder Therapie mit Calcidiol nicht negativ beeinflusst.

Die obigen Ergebnisse erlauben die Schlussfolgerung, dass Vitamin D, insbesondere in seiner aktiven Form, für einen Einsatz im Rahmen der Behandlung von nicht-steroidpflichtigen, allergischen und entzündlichen Erkrankungen oder zur Steroidreduktion geeignet ist, wobei aufgrund der höheren antientzündlichen Wirkung Calcidiol bevorzugt wird. Calcidiol ist nämlich auch in höheren Konzentrationen in gesunden humanen Monozyten wirksam, was bislang im Stand der Technik bislang so nicht bekannt war.

### b) Vitamin D zur Intensivierung der Wirkung von Leitlinientherapien bei unteren Atemwegserkrankungen

Hintergrund: Bisherige Studien bei chronisch obstruktiver Atemwegserkrankung (COPD) belegen, dass die kombinierte inhalative Therapie auf Basis von inhalativen Kortikosteroiden (ICS), wie beispielsweise Budesonid, in Kombination mit LABA, wie beispielsweise Formoterol, sowie die Therapie mit LABA und LAMA oder auch mit einem selektiven Phosphodiesterase-4-Inhibitor (PDE-4-Inhibitor) nur einen partiellen, retardierenden Therapieerfolg bezüglich des progredienten Verlusts der Lungenfunktion bewirkt. Diese überwiegend inhalativ zur Verfügung stehenden Substanzen oder Substanzkombinationen reduzieren Exazerbationen im Bereich von 14 % (LAMA, z. B. Tiotropium) bis 25 % (ICS, z. B. Budesonid, in Kombination mit LABA, z. B. Formoterol) und führen nur zu einer Retardation des Progresses des Lungenfunktionsverlusts (FEV1) und können diesen nicht vollständig verhindern bzw. stoppen.

Ein wichtiger Grund hierfür könnte darin liegen, dass die überwiegend inhalativ und somit topisch eingesetzte Medikation den zunehmend bedeutenden Effekt einer Systementzündung bei COPD nicht ausreichend kontrolliert. COPD tritt insbesondere in Assoziation mit typischen Vitamin D-Mangelerkrankungen, wie Osteoporose, Diabetes und kardio-vaskulären Erkrankungen, auf. Insgesamt ist COPD verhältnismäßig schwer zu behandeln.

Im Vergleich dazu lässt sich Asthma deutlich besser behandeln, mit dem unstrittig hoch angesetzten Ziel, eine vollständige Beschwerdefreiheit zu erreichen. Ein wichtiger Unterschied bei Asthma ist das relativ gute Ansprechen auf ICS und der bisher fehlende Hinweis auf eine Assoziation mit Systemerkrankungen. Jedoch gibt es gehäuft, zumeist auch mit Übergewichtigkeit einhergehende schwere allergische und nicht-allergische Begleiterscheinungen, die eine systemische Therapie mit Kortikosteroiden erfordern, was auf eine Assoziierung mit Vitamin D-Mangelzuständen schließen lässt.

Insgesamt ist davon auszugehen, dass sowohl bei COPD und als auch bei Asthma häufig eine Assoziation mit Vitamin D-Mangelzuständen vorliegt.

Erfindungsgemäßer Ansatz: Erfindungsgemäß ist insbesondere davon auszugehen, dass - ohne sich auf diese Theorie beschränken zu wollen -der Vitamin D-Mangel bei COPD und Asthma die primäre Ursache für das unterschiedliche Ansprechen auf inhalative Leitlinientherapien und somit die Erkrankungsschwere ist. Insgesamt ist es auf Basis der synergistischen Interaktion von Vitamin D im Rahmen von COPD bzw. Asthma erstrebenswert, eine Erhöhung der Vitamin D-Atemwegskonzentration, insbesondere von Calcidiol, zu erreichen, um die antiinflammatorische und bronchodilatatorische Wirkung, insbesondere von ICS in Kombination mit LABA sowie LAMA, zu verbessern. Dagegen haben - ohne sich auf diese Theorie beschränken zu wollen - etwaige genregulatorische Wirkungen von Vitamin D, wie eine mögliche Downregulation von krankheitsspezifischen Genen, allenfalls einen grundsätzlichen Bedeutungsgrad, der bestimmend für den Zeitpunkt des Beginns der Erkrankung ist, aber jedoch nicht für den Schweregrad der Atemwegserkrankung im weiteren Verlauf.

Erfindungsgemäß kann somit die zusätzliche insbesondere inhalative Therapie mit Vitamin D, z. B. kombiniert mit einer Leitlinientherapie oder alleine, den Verlauf von Atemwegserkrankungen drastisch verbessern und insbesondere bei COPD und konstriktiver Bronchiolitis den Progress und die Exazerbationshäufigkeit reduzieren. Bei Asthma hingegen kann im Rahmen des erfindunggemäßen Therapiekonzepts eine bronchodilatatorische Wirkung erzielt werden, welche die Folgen von allergischen Sensibilisierungen, Atemwegsinfekten sowie Reizungen der Atemwege durch Dämpfe oder Stäube signifikant verringert. Darüber hinaus kann auf Basis des erfindungsgemäßen Konzepts der Therapiebedarf an Steroiden, insbesondere an hochdosierten ICS und systemischen Kortikosteroiden, reduziert werden.

In diesem Zusammenhang wurde im Rahmen der vorliegenden Erfindung zunächst die direkte entzündungshemmende Wirkung von Vitamin D an isolierten Monozyten untersucht, welche den Vitamin D-Rezeptor exprimieren und Vorläufer von alveolären Makrophagen darstellen, die insbesondere auch die unteren Atemwege auskleiden (Fig. 1).

Anschließend wurde die entzündungshemmende Wirkung einer Kombination auf Basis von Vitamin D, insbesondere Calcitriol, und Budesonid untersucht. Dazu wurde der hemmende Effekt von Calcitriol und Budesonid sowohl alleine als auch in Kombination auf die LPS-stimulierte Produktion von TNF-alpha in humanen Monozyten bestimmt. Die diesbezüglich erhaltenen Ergebnisse sind der nachfolgenden Tabelle 1 zu entnehmen.

**Tabelle 1: Effekt von Budesonid und Calcitriol auf die LPS-stimulierte Produktion von TNF-alpha in humanen Monozyten**

| **Konz.** | **n** | **TNF-alpha (pg/10⁵ Monozyten)** | **Vergleich zur Kontrolle (%)** | **p-Wert** |
|---|---|---|---|---|
| ohne Stimulation | 8 | 756,1 ± 192 | - | - |
| 10 µg/ml LPS | 8 | 889,1 ± 52 | - | - |

| **Calcitriol alleine** | | | | |
|---|---|---|---|---|
| 1 ng/ml | 8 | 712 ± 31 | -20,0 | 0,0117 |
| 10 ng/ml | 8 | 687,5 ± 33 | -22,7 | 0,0008 |
| 30 ng/ml | 8 | 588,8 ± 14 | -33,8 | 0,0008 |
| 50 ng/ml | 8 | 605,6 ± 21 | -31,9 | 0,0008 |

| **Budesonid alleine** | | | | |
|---|---|---|---|---|
| 10⁻¹⁰ mol | 8 | 606,4 ± 110 | -31,8 | 0,0460 |
| 10⁻⁹ mol | 8 | 412,2 ± 22 | -53,6 | 0.0008 |

| **Budesonid in Kombination mit Calcitriol** | | | | |
|---|---|---|---|---|
| Bud. 10⁻¹⁰) mol und Vit. D 1 ng/ml | 8 | 439,4 ± 53 | -50,6 | 0,0008 |
| Bud. 10⁻¹⁰ mol und Vit. D 10 ng/ml | 8 | 430,0 ± 73 | -51,6 | 0,0008 |
| Bud.10⁻¹⁰ mol und Vit. D 30 ng/ml | 8 | 354,4 ± 57 | -60,1 | 0,0008 |
| Bud.10⁻¹⁰ mol und Vit. D 50 ng/ml | 8 | 333,4 ± 34 | -62,5 | 0,0008 |
| Bud. 10⁻⁹ mol und Vit. D 1 ng/ml | 8 | 303,7 ± 12 | -65,5 | 0,0008 |
| Bud. 10⁻⁹ mol und Vit. D 10 ng/ml | 8 | 266,8 ± 22 | -70,0 | 0,0008 |
| Bud. 10⁻⁹ mol und Vit. D 30 ng/ml | 8 | 223.1 ± 18 | -73,8 | 0,0008 |
| Bud. 10⁻⁹ mol und Vit. D 50 ng/ml | 8 | 218,6 ± 27 | -75,5 | 0,0008 |

Die in Tabelle 1 aufgeführten Ergebnisse zeigen, dass sich überraschenderweise auf Basis einer Kombination von Budesonid und Calcitriol eine deutlich stärkere Hemmung der Produktion von TNF-alpha in humanen Monozyten erzielen lässt als mit Calcitriol bzw. Budesonid jeweils alleine, und zwar über die Wirkung der Einzelsubstanzen hinausgehend Somit wurde im Rahmen der vorliegenden Erfindung eine synergistische Wirkung zwischen dem inhalativen Kortikosteroid Budesonid und Calcitriol gefunden, d. h. eine Zunahme der entzündungshemmenden Wirkung von Budesonid in Gegenwart von relevanten Atemwegskonzentrationen von Calcitriol.

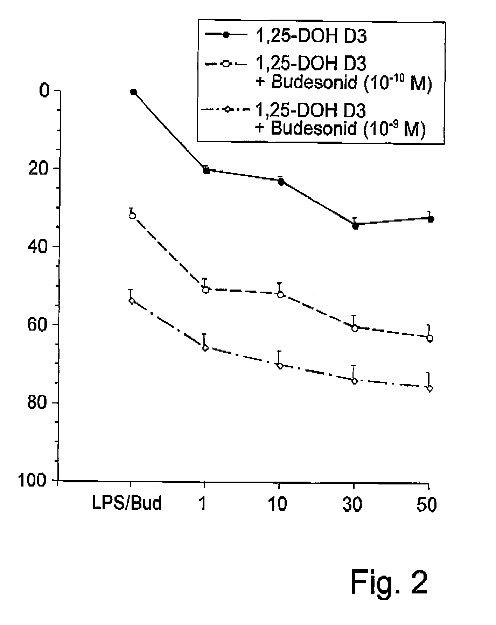

Fig. 2 zeigt, dass die LPS-stimulierte TNF-alpha-Produktion durch ansteigende Konzentrationen von Calcitriol signifikant gehemmt wird (durchgezogene Linie). Co-Inkubation von Calcitriol mit einer niedrigeren Atemwegskonzentration von Budesonid (10⁻¹⁰mol/l, gestrichelte Linie) führt zu einer signifikanten Zunahme der TNF-alpha-Hemmung im Vergleich zur Kontrolle (LPS + Budesonid, Calcitriol oder Budesonid alleine). Diese Hemmeffekte nehmen weiter synergistisch zu nach Co-Inkubation von Calcitriol mit einer höheren Konzentration von Budesonid (10⁻⁹mol/l, strichpunktierte Linie). In

Fig. 2 zeigt die x-Achse die Konzentration an eingesetztem Calcitriol in ng/ml, und die y-Achse zeigt den jeweiligen Mittelwert mit Angabe der Standardabweichung der prozentualen Hemmung im Vergleich zu der TNF-alpha-Kontrolle (mit p < 0,045 für Calcitriol, Budesonid, Budesonid + Calcitriol vs. LPS-Kontrolle; p < 0.016 für Budesonid 10⁻¹⁰ mol/l + Calcitriol vs. Calcitriol alleine; p = 0.0008 für Budesonid 10⁻⁹ mol/l + Calcitriol vs. Calcitriol alleine; p < 0.0275 für Budesonid 10⁻¹⁰ mol/l + Calcitriol vs. Budesonid 10⁻¹⁰ mol/l alleine; p < 0.0035 für Budesonid 10⁻⁹ mol/l + Calcitriol vs. Budesonid alleine).

Weiterhin wurde der Effekt von Formoterol auf die LPS-stimulierte TNF-alpha-Produktion in normalen humanen Monozyten *in vitro* untersucht. Die diesbezüglich erhaltenen Ergebnisse sind in Tabelle 2 aufgeführt.

**Tabelle 2: Effekt von Formoterol auf die LPS-stimulierte Produktion von TNF-alpha in humanen Monozyten**

| **Konz. Formoterol [mol/l]** | **TNF-alpha (pg/10⁵ Monozyten)** | **Vergleich zur Kontrolle (%)** | **p-Wert** |
|---|---|---|---|
| ohne Stimulation | 110,9 ± 72 | - | - |
| 10 µg/ml LPS | 701,6 ± 80 | - | - |
| 10⁻¹² | 693,7 ± 74 | -1,1 ± 11 | 0,8728 |
| 10⁻¹¹ | 653,1 ± 64 | -6,9 ± 10 | 0,3367 |
| 10⁻¹⁰ | 550,9 ± 42 | -21,5 ± 8 | 0,1495 |
| 10⁻⁹ | 491,7 ± 37 | -29,9 ± 7 | 0,1495 |
| 10⁻⁸ | 481,1 ± 21 | -31.4 ± 4 | 0,0163 |
| 10⁻⁷ | 452,9 ± 24 | -35,4 ± 5 | 0,0163 |
| 10⁻⁶ | 487,8 ± 22 | -30,5 ± 4 | 0,0250 |
| 10⁻⁵ | 679,7 ± 34 | -3,1 ± 5 | >0,9999 |

Die in Tabelle 2 aufgeführten Ergebnisse zeigen, dass Formoterol in Alleinstellung bei variablen Konzentrationen im Bereich von 10⁻⁹ bis 10⁻⁵ mol/l keine ausreichende hemmende Wirkung auf die LPS-stimulierte Produktion von TNF-alpha in humanen Monozyten besitzt. Die mittels des *Mann* & *Whitney-Tests* ermittelten p-Werte lagen oberhalb von 0,05. Aufgrund der p-Werte kann in Bezug auf die Kontrolle - also die TNF-alpha-Produktion in ausschließlich mit LPS stimulierten humanen Monozyten - keine Signifikanz angenommen werden.

Weiterhin wurde im Rahmen der vorliegenden Erfindung untersucht, inwieweit eine Kombination auf Basis von Calcitriol und Formoterol die LPS-stimulierte Produktion von TNF-alpha in humanen Monozyten hemmen kann und somit eine antiinflammatorische Wirkung besitzt. Die diesbezüglich erhaltenen Ergebnisse sind in Tabelle 3 aufgeführt.

**Tabelle 3: Effekt von Calcitriol (Vit. D) und Formoterol (F) auf die LPS-stimulierte Produktion von TNF-alpha in humanen Monozyten**

| **Konz.** | **TNF-alpha (pg/10⁵ Monozyten)** | **Vergleich zur Kontrolle (%)** | **p-Wert** |
|---|---|---|---|
| ohne Stimulation | 511,75 ± 198 | - | - |
| 10 µg/ml LPS | 3,219 ± 195 | - | - |

| **Vitamin D3 alleine** | | | |
|---|---|---|---|
| Vit. D: 1 ng/ml | 2.779 ± 254 | -13,9 ± 9 | 0,1333 |
| Vit. D: 10 ng/ml | 2.209 ± 245 | -31,4 ± 11 | 0,0179 |
| Vit. D: 30 ng/ml | 2.202 ± 263 | -31,6 ± 12 | 0,0209 |
| Vit. D: 50 ng/ml | 2.048 ± 256 | -36,4 ± 12 | 0,0079 |

| **Formoterol alleine** | | | |
|---|---|---|---|
| F: 10⁻⁸ mol/l | 1.945 ± 237 | -39,6 ± 12 | 0,0067 |

| **Calcitriol und Formoterol** | | | |
|---|---|---|---|
| F: 10⁻⁸ mol/l Vit. D: 1 ng/ml | 1.484 ± 196 | -53,9 ± 13 | < 0,0001 |
| F: 10⁻⁸ mol/l Vit. D: 10 ng/ml | 1.224 ± 176 | -62 ± 14 | < 0,0001 |
| F: 10⁻⁸ mol/l Vit. D: 30 ng/ml | 1.232 ± 177 | -61,7 ± 14 | < 0,0001 |
| F: 10⁻⁸ mol/l Vit. D: 50 ng/ml | 1,370 ± 223 | -57,4 ± 16 | < 0,0001 |

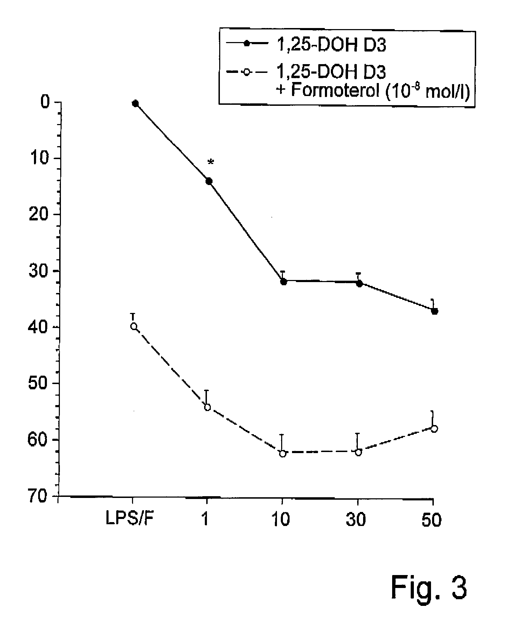

Anhand der obigen Ergebnisse gemäß Tabelle 3 ist ersichtlich, dass sich Calcitriol und Formoterol in ihrer Wirkung in Bezug auf die Hemmung der LPSstimulierten Produktion von TNF-alpha in humanen Monozyten synergistisch ergänzen. Durch die Co-Inkubation der humanen Monozyten in Calcitriol und Formoterol kann sowohl im Vergleich zur Kontrolle (TNF-alpha-Produktion in Monozyten durch Stimulation mit LPS) als auch im Vergleich zum alleinigen Einsatz von Calcitriol bzw. Formoterol eine signifikant stärkere Hemmung der TNF-alpha-Produktion erzielt werden. Somit ist auch bei einem therapeutischen Einsatz einer Kombination auf Basis von Calcitriol und Formoterol ein starker entzündungshemmender Effekt zu erwarten. Die in Tabelle 3 aufgeführten Ergebnisse sind zudem in Fig. 3 graphisch dargestellt.

Fig. 3 zeigt, dass die LPS-stimulierte TNF-alpha-Produktion durch ansteigende Konzentrationen von Calcitriol signifikant gehemmt wird (durchgezogene Linie). Co-Inkubation von Calcitriol mit Formoterol (10⁻⁸ mok/l, gestrichelte Linie) führt zu einer signifikanten Zunahme der TNF-alpha-Hemmung im Vergleich zur Kontrolle. In Fig. 3 zeigt die x-Achse die Konzentration an eingesetztem Calcitriol in ng/ml, und die y-Achse zeigt den jeweiligen Mittelwert mit Angabe der Standardabweichung der prozentualen Hemmung im Vergleich zu der TNF-alpha-Kontrolle (mit *p > 0,1, sonst p < 0,02 für Calcitriol, Formoterol, Formoterol ÷ Calcitriol vs. LPS-Kontrolle; p = 0.0001 für Formoterol vs. Caleitriol; p = 0.0001 für Calcitriol + Formoterol vs. Calcitriol alleine).

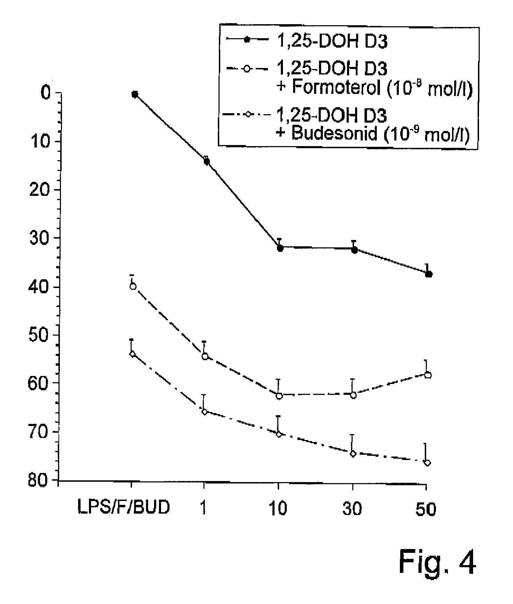

In Fig. 4 wird im Rahmen einer Zusammenschau der in Tabelle 1 und 3 dargestellten Ergebnisse die Hemmwirkung von Budesonid und Formoterol jeweils in Kombination mit Calcitriol gegenüber dem alleinigen Einsatz von Calcitriol dargestellt. In Fig. 4 zeigt die x-Achse die Konzentration an eingesetztem Calcitriol in ng/ml, und die y-Achse zeigt den jeweiligen Mittelwert mit Angabe der Standardabweichung der prozentualen Hemmung im Vergleich zu der TNF-alpha-Kontrolle.

Insgesamt sind die oben aufgeführten Ergebnisse für die Atemwegstherapie bei Asthma und COPD, vor allem für die Behandlung von Patienten mit höherer Erkrankungsschwere, insbesondere adipösen Patienten, von großer Bedeutung, insbesondere da in Abhängigkeit vom Körpergewicht auch eine höheigradige Systementzündung vorliegt. Im Rahmen der vorliegenden Erfindung wird davon ausgegangen, dass - ohne sich auf diese Theorie beschränken zu wollen - die Systementzündung durch einen Vitamin D-Mangelzustand bei Adipositas verursacht wird. Erfindungsgemäß werden somit insbesondere für diese schwierige Patientengruppe durch die kombinierte Inhalation mit Vitamin D wirkeffiziente Therapiekonzepte nach der Erfindung bereitgestellt.

Diese Ergebnisse sind auch insofern richtungweisend, als - ohne sich auf diese Theorie beschränken zu wollen - die zunehmende Stimulierung des Vitamin D-Rezeptors zu einer vermehrten Expression und somit zu einer Zunahme entzündungshemmender, bronchodilatatorischer und exazerbationsschützender Wirkungen führt. LABA alleine hingegen führt zu einer Downregulation von beta-2-Rezeptoren, so dass eine Co-Medikation von Vitamin D und LABA oder auch von Vitamin D und LAMA zu einer Wirkverstärkung der Leitlinientherapien führt. In gleicher Weise hat sich im Rahmen der vorliegenden Erfindung eine Dreifachtherapie auf Basis von Vitamin D, ICS und LABA im Vergleich zur gegenwärtigen kombinierten Therapie mit ICS und LABA als vorteilhaft erwiesen.

Insgesamt wurde im Rahmen der vorliegenden Erfindung überraschenderweise herausgefunden, dass Vitamin D insbesondere die Wirkung kleiner topischer inhalativer Konzentrationen von Kortikosteroiden in ihrer antientzündlichen Wirksamkeit verstärkt. Insbesondere kommt es auch zu einer Wirkverstärkung in den kleinen Atemwegen der Lungenperipherie, in welchen üblicherweise die Wirkung nur unzureichend ist.

Im Rahmen der vorliegenden Erfindung wurde somit in überraschender Weise ein therapeutischer Nutzen von Vitamin D in Kombination mit ICS und/oder beta-Sympathomimetika insbesondere bei COPD, Bronchiolitis und Asthma gefunden.

Aufgrund der Tatsache, dass Monozyten Vitamin D produzieren, kann im Rahmen der vorliegenden Erfindung - ohne sich auf diese Theorie beschränken zu wollen - davon ausgegangen werden, dass die Co-Medikation von Vitamin D auch unabhängig von einer vorliegenden Vitamin D-Defizienz in Ergänzung zur Leitlinientherapie aufgrund der eigenen und mit ICS kombiniert verstärkt nachgewiesenen Hemmung von TNF-alpha effektiv entzündungshemmend wirkt und den Leitlinieneffekt intensiviert. Zusätzlich kann davon ausgegangen werden, dass die Co-Medikation von Vitamin D zur Leitlinientherapie den zunehmenden Schweregrad von Atemwegserkrankungen reduziert und remissionserhaltend wirksam ist. Als bevorzugtes Vitamin D wird die Vorläufersubstanz Calcidiol als Co-Medikation mit ICS, LABA und LAMA oder mit einem Anti-Immunglobulin E (z. B. Omalizumab) eingesetzt, da die mit ihr erzielte Entzündungshemmung im Vergleich zum Einsatz von Calcitriol stärker ist (siehe Fig.1).

### c) Vitamin D zur Intensivierung der Wirkung der Leitlinientherapie bei entzündlichen Darmerkrankungen

Hintergrund: Die lokale Therapie bei entzündlichen Darmerkrankungen auf Basis einer Kombination von 5-Aminosalicylsäure und Budesonid ist oftmals alleine nicht ausreichend und erfordert eine zusätzliche Immunsuppression. Üblicherweise wird zur Erzielung der notwendigen Immunsuppression systemisch wirksames Azathioprim eingesetzt. Der Krankheitsverlauf dieser Patienten mit zusätzlichem, systemisch wirksamem Therapiebedarf zur Lokaltherapie ist aufgrund der gesteigerten Entzündungsaktivität mit einem gehäuften Auftreten von Rezidiven von ca. 50 % nach 3 Jahren assoziiert und erfordert daher im Allgemeinen eine Langzeittherapie.

Es ist bekannt, dass Budesonid aufgrund eines schnellen Lebermetabolismus nur wenig systemische Nebenwirkungen verursacht, jedoch mitunter keine ausreichende systemische Entzündungshemmung vermittelt, aber zur Behandlung autoimmuner Bauchspeicheldrüsenerkrankungen geeignet ist. Insbesondere hat sich der Einsatz von 6 mg Budesonid bei der Behandlung von *Morbus Crohn* im Vergleich zum Placebo bei der Vermittlung einer Remission von drei Monaten als nicht effektiver erwiesen. Der Einsatz von 6 mg Budesonid hat sich allerdings effektiver als die Gabe von 3 mg Mesalazin (5-Aminosalicylsäure) erwiesen. Darüber hinaus hat sich gezeigt, dass der Einsatz von 6 mg Budesonid nicht effektiver ist als der Einsatz einer reduzierten Menge von 3 mg. Vielmehr traten häufige systemische Nebenwirkungen mit einer Kortisolsuppression bei Verabreichung von 6 mg Budesonid auf. Budesonid hat sich daher insgesamt zur Langzeittherapie bei *Morbus Crohn* als nicht empfehlenswert erwiesen.

Was die Wirksamkeit anbelangt, so ist die immunsuppressive Systemtherapie zur Remissionserhaltung mit Azathioprim (83 % Remission) im Vergleich zur Lokaltherapie mit Budesonid (24 % Remission) effektiver, jedoch gleichzeitig auch mit größeren Nebenwirkungen verbunden. Neben der Entwicklung einer primär sklerosierenden Cholangitis und von Gelenksschmerzen gilt auch die Rolle der Systementzündung für die Entstehung und Progression einer Darmentzündung als nicht geklärt.

Bei leichteren Formen und zur Rezidivprophylaxe wird diese bislang in einer lokalen Therapie des Darms mit verschiedenen dünndarmlöslichen Substanzen, wie Salazosulfpyridin, 5-Aminosalicylsäure, Paraaminosalicylsäure und lokal resorbierbaren Glukokortikosteroiden, wie z. B. Budesonid, mit unterschiedlicher, primär lokaler, antientzündlicher Wirkung behandelt. Bei schweren Formen ist dagegen der Einsatz systemisch applizierbarer entzündungshemmender Substanzen in Form von im Allgemeinen nebenwirkungsreichen Immunsuppressiva, wie Glukokortikosteroiden, Azathioprim und dergleichen, erforderlich. Bei Kindern, insbesondere Kindern mit einem Alter unter 12 Jahren, ist die Manifestation der Krankheit unterschiedlich und tritt gehäuft als Pancolitis auf. Bei ca. 25 % der jungen Patienten ist bereits in frühen Jahren eine Colektomie notwendig. Zudem führen die starken Nebenwirkungen der Medikamente sowie Systementzündungen häufig zu Wachstumsstörungen.

Dem Fachmann sind zur Behandlung milder bis mittelgradiger und insbesondere linksseitiger *Colitis ulcerosa* sowie zur Remissionserhaltung 5-Aminosalicylate (5-ASA) als Haupttherapeutika bekannt. Im Vergleich zu Budesonid sprechen diese im Rahmen der Therapie deutlich besser an. Bei schweren Formen der *Colitis ulcerosa,* bei Befall des Dünndarms *(Ileum)* und des rechtseitigen Colons, bei milden bis mittelgradigen Krankheitsaktivitäten sowie bei *Morbus Crohn* hingegen ist die Therapie mit Steroiden, insbesondere Budesonid, nur über einen begrenzten Zeitraum empfehlenswert. Auch der systemische Einsatz von Steroiden zur Behandlung akuter Exazerbationen ist nur über einen begrenzten Zeitraum empfehlenswert.

Somit besteht im Stand der Technik weiterhin ein Bedarf für ein gut verträgliches und effektives, entzündungshemmendes Therapiekonzept zur prophylaktischen Remissionserhaltung chronisch entzündlicher Darmerkrankungen. Grundsätzlich ist ein früher Therapiebeginn anzustreben, um der Eskalation der Entzündung und einem hohen Steroidäquivalenzbedarf mit nebenwirkungsreichen Immunsuppressiva vorzubeugen, um den Krankheitsverlauf positiv zu beeinflussen. Um dieses Ziel, insbesondere mit Blick auf einen Therapiebeginn zu einem möglichst frühen Zeitpunkt der Erkrankung zu erreichen, wird erfindungsgemäß ein gut verträgliches und effektives Therapiekonzept bereitgestellt, welches auf eine Verstärkung der lokalen Wirkung bisher eingesetzter Therapeutika, insbesondere von 5-Aminosalicylsäure (5-ASA) und Budesonid, abzielt und darüber hinaus eine gut verträgliche systemische Wirksamkeit besitzt.

Erfindungsgemäßer Ansatz: Der Tumornekrosefaktor (TNF-alpha) spielt für die Vermittlung der Zytokininduktion und somit der Entzündungsreaktion bei verschiedenen Erkrankungen eine zentrale Rolle. Daher wurde in den vorliegenden *in vitro* durchgeführten Untersuchungen TNF-alpha als Maß für die entzündungshemmende Wirkung einer Substanz oder Substanzkombination eingesetzt. Das Zytokin TNF-alpha wird bei *Colitis ulcerosa* und *Morbus Crohn* vermehrt von sogenannten Schwann-Zellen gebildet. Im Vergleich zu nicht-aktiver *Colitis ulcerosa* oder normalen Zellen ist bei aktiver *Colitis ulcerosa* sowohl die Produktion als auch die Rezeptorexpression von TNF-alpha erhöht. Bei Patienten mit chronisch-entzündlichen Darmerkrankungen, insbesondere *Morbus Crohn,* ist die Expression von relevanten Typ 1-T-Helferzellen charakteristisch, was insbesondere zu einer vermehrten Ausschüttung von Zytokinen, insbesondere Interferon-gamma, TNF-alpha und Interleukin-2, führt. Bei *Colitis ulcerosa* kann allerdings Interleukin-10 als Antagonist der Zytokinproduktion eine kompensatorische, entzündungshemmende Wirkung besitzen.

Zur Frage einer immunmodulatorischen Bedeutung der aktiven Form von Vitamin D, insbesondere Calcidiol und/oder Calcitriol, sind durch den Einsatz von 100 pg/ml Calcitriol Effekte auf die Produktion von Typ 1 und Typ 2 T-Helferzellen bei 8 Patienten mit *Morbus Crohn* und 9 Patienten mit *Colitis ulcerosa* mit 6 gesunden Probanden im Rahmen einer *ex vivo*-Studie verglichen worden. Dazu wurden aus dem Blut der Probanden isolierte periphere mononukleäre Zellen nach einer Inkubation mit 100 pg/ml Calcitriol untersucht.

Die diesbezüglichen Ergebnisse zeigen, dass bei den aus Patienten mit chronisch-entzündlichen Darmerkrankungen isolierten peripheren mononukleären Zellen im Vergleich zu denen der gesunden Kontrollgruppe die Produktion von TNF-alpha signifikant erhöht war, allerdings durch Inkubation der Zellen mit Calcitriol gesenkt werden konnte. Zudem ist gezeigt, dass Calcitriol die Produktion von Interferon-gamma reduziert und die Produktion von Interleukin-10 stimuliert. Der Effekt von Calcitriol auf die Interleukin-10 Produktion war bei Patienten mit *Colitis ulcerosa* nicht signifikant größer als bei Patienten mit *Morbus Crohn.* Die Produktion von TNF-alpha wurde nur bei Zellen aus Patienten mit *Morbus Crohn* und nicht bei Zellen aus gesunden Patienten durch Calcitriol gehemmt. Für *Morbus Crohn* und *Colitis ulcerosa* wird somit insgesamt ein unterschiedliches Ansprechen auf Vitamin D bzw. dessen Präkursoren bzw. Vorläufersubstanzen postuliert.

Um die Funktion von Interleukin-10 produzierenden dendritischen Zellen bzw. die Folgen eines Mangels dieser Zellen bei chronisch-entzündlichen Darmerkrankungen zu untersuchen, wurden Kurzzeit- und Langzeitinkubationen von Interleukin-10 und dentritischen Zellen mit Dexamethason und Calcitriol durchgeführt. Dabei wurde insbesondere die Produktion des CD4-Rezeptors und Lymphozyten im Vergleich zu einer Kontrolle untersucht. Aus den diesbezüglich erhaltenen Ergebnissen geht hervor, dass immunsuppressiv wirkende dendritische Zellen vor einer experimentell vermittelten chronisch-entzündlichen Darmerkrankung schützen können. Im Zusammenhang mit dem neuen Vitamin D-Rezeptoragonisten Calcitriol hat sich zudem herausgestellt, dass die mRNA für TNF-alpha und andere Zytokine einen stärkeren hemmenden Effekt hat als Calcitriol und weiterhin weniger hypercalcämisch wirkt.

Zusammenfassend zeigt der aktuelle Stand, dass Calcitriol bislang maßgeblich nur zur klinischen Diagnose eines Vitamin D-Mangelzustands bekannt ist. Die Ursachen für Vitamin D-Mangelzustände sind grundsätzlich noch nicht vollständig verstanden, es existieren allerdings Vermutungen, dass auch die Lebensführung eine Rolle bei *Morbus Crohn* spielen könnte, insbesondere im Hinblick auf ernährungsphysiologische Aspekte. Dabei wird vor allem die Abhängigkeit des Vitamin D-Spiegels von der Zufuhr von Fischöl mit hohem Anteil an Omega-3-Fettsäuren, Prebiotika und von Oxidantien als relevant in Bezug auf *Morbus Crohn* angesehen.

Im grundlegenden Unterschied zum Stand der Technik wurde im Rahmen der vorliegenden Erfindung überraschenderweise eine starke entzündungshemmende Wirkung für Calcidiol gefunden, welche die von Calcitriol um mehr als das dreifache übersteigt und darüber hinaus bei steigenden Konzentrationen nicht wieder abnimmt. Im Rahmen der vorliegenden Erfindung wurde somit überraschenderweise gefunden, dass neben Calcitriol insbesondere Calcidiol zur Langzeittherapie verschiedener entzündlicher Erkrankungen, insbesondere von chronisch-entzündlichen Darmerkrankungen, geeignet ist.

Aufgrund der zuvor geschilderten Probleme des Standes der Technik besteht ein wichtiger Bedarf, die klinisch nicht ausreichende entzündungshemmende Wirkung von 5-ASA für die Therapie auch schwerer Formen der *Colitis ulcerosa* sowie die Resistenzentwicklung, unter anderem gegen Kortikosteroide, insbesondere bei *Morbus Crohn,* zu verbessern.

Im Rahmen der vorliegenden Erfindung wurde daher untersucht, ob durch den Einsatz einer Kombination von Vitamin D sowie 5-ASA der entzündungshemmende Effekt von 5-ASA durch Vitamin D verstärkt werden kann und eventuelle proentzündliche Effekte von 5-ASA durch eine Kombination mit Vitamin D reduziert werden können. Auf dieser Basis könnte die Wirksamkeit und Verträglichkeit der Standardtherapie mit 5-ASA erhöht werden.

Zu diesem Zweck wurden die entzündungshemmenden Effekte einer Kombination von Vitamin D und 5-ASA erstmalig im Rahmen der vorliegenden Er*findung in vitro* in normalen humanen Monozyten untersucht. Die erfindungsgemäß erhaltenen Ergebnisse zeigen, dass 5-ASA in therapeutischen Konzentrationen von 0,040 bis 0,12 mg/mg im Gewebe und 0,1 bis 1,5 mg/ml im Plasma die LPS-stimulierte Produktion von TNF-alpha bei einer geweberelevanten Konzentration von 0,1 mg/ml und einer plasmarelevanten Konzentraton von 1 mg/ml signifikant hemmt. Darüber hinaus hat sich gezeigt, dass mit - 7 bis - 8 % im Vergleich zur LPS-Kontrolle die Hemmung durch 5-ASA jedoch nur schwach ausfiel. Darüber hinaus nahmen die Hemmeffekte bei Einsatz höherer Konzentrationen von 5-ASA von 10 mg/ml wieder ab. Insgesamt lässt dies den Schluss zu, dass 5-ASA nur einen relativ schwachen entzündungshemmenden Effekt, insbesondere in Bezug auf eine Hemmung der Produktion von TNF-alpha, besitzt.

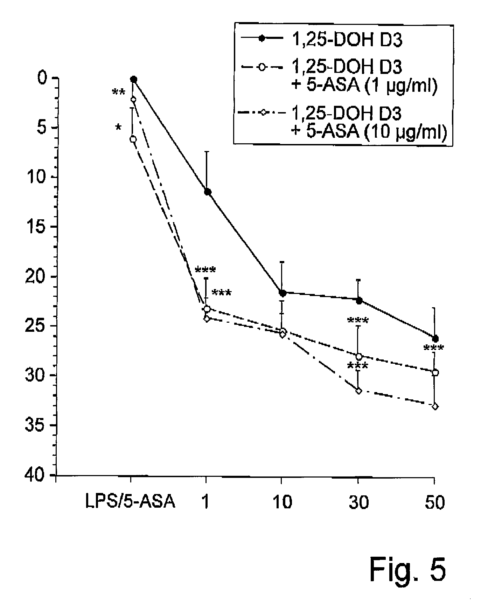

Überraschenderweise wurde im Rahmen der vorliegenden Erfindung herausgefunden, dass die Hemmeffekte von TNF-alpha bei Co-Inkubation relevanter Plasmaspiegel von 5-ASA von 1 mg/ml und Vitamin D in aufsteigenden, therapeutisch relevanten Vitamin D-Konzentrationen zunehmen (Fig. 5).

Fig. 5 zeigt, dass die LPS-stimulierte TNF-alpha-Produktion durch ansteigende Konzentrationen von Calcitriol signifikant gehemmt wird (durchgezogene Linie). Co-Inkubation von Calcitriol mit einer plasmarelevanten Konzentration von 5-ASA (1µg/ml, gestrichelte Linie) führt zu einer signifikanten und auch synergistischen Zunahme der TNF-alpha-Hemmung im Vergleich zur Kontrolle (LPS/5-ASA), 5-ASA oder Vitamin D alleine. Die Hemmeffekte sind nicht signifikant für eine höhere Konzentration von 5-ASA (10 µg/ml) alleine und nehmen dosisabhängig nach Co-Inkubation von Calcitriol mit 5-ASA (10µg/ml, strichpunktierte Linie) weiter zu. In Fig. 5 zeigt die x-Achse die Konzentration an eingesetztem Calcitriol in ng/ml, und die y-Achse zeigt den jeweiligen Mittelwert mit Angabe der Standardabweichung der prozentualen Hemmung im Vergleich zu der TNF-alpha-Kontrolle (mit p < 0,003 vs. LPS-Kontrolle; *p<0.0352 vs. LPS-Kontrolle; **p>0.2 vs. LPS-Kontrolle; p<0.0001 für Calcitriol + 5-ASA vs. 5-ASA alleine; ***p < 0.022 für Calcitriol + 5-ASA vs. 5-ASA alleine).

Anhand von Fig. 5 wird somit deutlich, dass die LPS-stimulierte Produktion von TNF-alpha durch ansteigende Konzentrationen von Calcitriol signifikant gehemmt wird. Die Co-Inkubation von Calcitriol mit einer plasmarelevanten Konzentration von 5-ASA (1 mg/ml) führt darüber hinaus im Vergleich zu den Kontrollen auf Basis von 5-ASA bzw. Calcitriol allein sowie 5-ASA in Kombination mit LPS zu einer signifikanten und synergistischen Zunahme der Hemmung der Produktion von TNF-alpha. Die Hemmeffekte sind darüber hinaus nicht signifikant für eine höhere 5-ASA-Konzentration von 10 mg/ml alleine. Weiterhin nehmen die Hemmeffekte dosisabhängig nach Co-Inkubation von Calcitriol mit 10 mg/ml 5-ASA weiter zu.

Die Versuchsreihe auf Basis einer Co-Inkubation von Calcitriol und 5-ASA mit einer Konzentration von 1 mg/ml zeigt, dass der kombinierte Einsatz zu einer synergistischen Hemmwirkung der Produktion von TNF-alpha führt. Im Vergleich zum alleinigen Einsatz von 5-ASA konnte die Hemmwirkung über den gesamten therapeutischen Bereich mit aufsteigenden Vitamin D- bzw. Calcitriol-Konzentrationen von 1 ng/ml (Hemmwirkung - 23,1 ± 3 %), 10 ng/ml (Hemmwirkung -25,3 ± 3%), 30 ng/ml (Hemmwirkung - 27,8 ± 3 %) und 50 ng/ml (- 29,4 ± 2 %) beobachtet werden. Insgesamt zeigen die Ergebnisse zudem, dass mit steigender Calcitriol-Konzentration auch die Hemmung der Produktion von TNF-alpha signifikant ansteigt. Im Rahmen der vorliegenden Erfindung wurde somit für die Kombination von Calcitriol und 5-ASA überraschenderweise eine Zunahme der entzündungshemmenden Wirkung im Vergleich zu 5-ASA alleine im Bereich von 400 bis 500 % gefunden.

Aufgrund der erfindungsgemäß beschriebenen stärkeren, entzündungshemmenden Wirkung von Calcidiol im Vergleich zu Calcitriol konnte darüber hinaus im Rahmen der vorliegenden Erfindung eine noch weitaus stärkere Internsivierung der 5-ASA-Wirkung für die Kombination von Calcidiol und 5-ASA nachgewiesen werden. Dies bedeutet, dass für eine Verstärkung der relativ schwachen entzündungshemmenden Wirkung von 5-ASA die Kombination auf Basis von Calcidiol und 5-ASA noch stärker ist und somit eine bevorzugte Ausführungsform darstellt. Darüber hinaus ist dies auch dahingehend von Relevanz, dass die Wahrscheinlichkeit bei bestehender Vitamin D-Mangelsituation eine Hypercalcämie zu entwickeln, als gering einzustufen ist. Weiterhin wird durch den Einsatz von Calcidiol als Vitamin D-Rezeptoragonist der Vitamin D₃-Rezeptor nicht herunterreguliert.

Mit diesem erfindungsgemäßen Konzept einer gezielten Kombination von Vitamin D und 5-ASA kann im Rahmen der vorliegenden Erfindung eine verbesserte Behandlung insbesondere auch der endoluminalen Entzündung bei chronisch-entzündlichen Darmerkrankungen bereitgestellt werden. Im Rahmen einer derartigen Behandlung wird zudem ein positiver Systemeffekt, welcher eine Prophylaxe, Stabilisierung, Remissionserhaltung und Steroideinsparung bzw. Intensivierung der Steroidwirkung bewirkt, erzielt. Insbesondere bei frühzeitigem Therapieeinsatz kann somit durch das erfindungsgemäße Therapiekonzept eine wirkeffiziente Alternative und Prophylaxe der Resistenzentwicklung gegenüber TNF-alpha-Inhibitoren bereitgestellt werden, insbesondere auf Basis einer Wirkungsverstärkung der zur Behandlung von chronisch-entzündlichen Darmerkrankungen eingesetzten Leitlinientherapeutika. Somit sind die erfindungsgemäßen Ergebnisse eine zentrale Voraussetzung für ein neues Therapiekonzept zur Verbesserung der oftmals nicht ausreichenden entzündungshemmenden, lokalen und systemischen Wirkungen von insbesondere 5-ASA und Budesonid zur Behandlung von chronisch-entzündlichen Darmerkrankungen.

Diese Wirkung gilt auch für die Prophylaxe und Therapie des Reizdarmsyndroms. Im Zusammenhang mit der Behandlung des Reizdarmsyndroms stellt die Co-Medikation von Vitamin D mit 5-ASA und gegebenenfalls Budesonid eine neue Therapieform zur Erhöhung der Entzündungshemmung dar.

### d) Anwendung- und Wirksamkeitsstudien

Im Rahmen eines ersten Untersuchungskomplexes wurden 11 Patienten im Alter von 32 bis 65 Jahren mit persistierendem Asthma bronchiale (GINA II), mit einer Kombinationstherapie aus inhalativem Glukokortikoid (Beclometason, 2 x 200 µg/die inhalativ) und inhalativem langwirksamen beta-2-Sympathomimetika (LABA, Salmeterol) sowie peroral Theophyllin behandelt. Nach einwöchiger Therapie konnte bei 5 der 11 Probanden eine leichte Verbesserung der Lungenfunktion erreicht werden. Nach fortgesetzter zwölfwöchiger Dauertherapie hatte sich das persistierende Bronchialasthma weiter stabilisiert, so dass bei 7 der 11 Patienten der inhalative Glukokortikoidbedarf um 35 % reduziert werden konnte. Bei zwei der behandelten Patienten konnte auch der Betamimetikabedarf um 10 % reduziert werden. Eine weitere Gruppe auf Basis von 9 Patienten im Alter von 39 bis 70 Jahren mit identischer Indikation, d. h. persistierendem Asthma bonchiale (GINA II), welche gleichermaßen die oben angeführten Kombinationstherapie aus inhalativem Glukokortikoid und inhalativem langwirksamem beta-2-Sympathomimetika sowie Theophyllin verabreicht bekamen, erhielten eine Woche zudem Vitamin D in einer Tagesdosis von 80 µg. Nach einwöchiger Therapie mit der vorgenannten Dosis konnte bei 6 der 9 Probanden eine leicht- bis mittelgradige Verbesserung der Lungenfunktion erreicht werden. Nach fortgesetzter zwölfwöchiger Dauertherapie hatte sich das persistierende Bonchialasthma insofern stabilisiert, als bei 7 der 9 Patienten der inhalative Glukokortikoidbedarf um bis zu 60 % reduziert werden konnte und bei 1 der 9 Patienten die inhalativen Glukokortikoide zeitweise sogar ganz abgesetzt werden konnten. Die Therapie wurde ohne Nebenwirkungen gut vertragen. Bei 4 der behandelten Patienten konnte auch der Betamimetikabedarf um bis zu 40 % reduziert werden.

### e) Weitere Abwendung- und Wirksamkeitsstudien:

Eine Vitamin D und 5-Aminosalicylsäure als Wirkstoffe enthaltende Darreichungsform nach der vorliegenden Erfindung wurde gleichermaßen im Rahmen der Wirksamkeitsuntersuchung bei der Behandlung von *Morbus Crohn* und *Colitis ulcerosa* in Co-Medikation zu systemischen Kortikosteroiden eingesetzt.

Als Vergleich dient ein Monopräparat mit selber Wirkstoffmenge auf Basis von 5-Aminosalicylsäure als alleinige Wirksubstanz.

Im Rahmen der klinischen Anwendungsbeobachtungen wurden jeweils 8 Probanden (zum einen im Alter von 32 bis 59 Jahren und zum anderen im Alter von 26 bis 60 Jahren) einerseits mit dem Monopräparat auf Basis von 5-Aminosalicylsäure und andererseits mit der zuvor beschriebenen erfindungsgemäßen Darreichungsform behandelt.

Mit der erfindungsgemäßen Darreichungsform auf Basis der Kombination von 5-Aminosalicylsäure und Vitamin D konnte ein deutlich verbesserter klinischer Effekt hervorgerufen werden, welcher sich dadurch manifestiert, dass die Stuhlfrequenz im Vergleich zu der Gruppe mit dem Monopräparat, bei welcher die Frequenz um etwa 40 % nachgelassen hat, nochmals deutlich auf eine Gesamtreduktion von über 55 % verbessert werden konnte. Ebenfalls konnte auch im Vergleich zu der Behandlung mit dem Monopräparat die systemische Kortikosteroidgabe nochmals deutlich reduziert werden.

Die vorliegenden Wirksamkeitsstudien belegen insgesamt die hervorragende und synergistische Wirkung der erfindungsgemäß angeführten Kombination eines Vitamin D-Rezeptoragonisten (VDA) einerseits und eines Monoterpens andererseits.

### Zusammenfassung:

Die im Rahmen der vorliegenden Erfindung durchgeführten Untersuchungen zeigen, dass die Stimulation des Vitamin D-Rezeptors mit den Agonisten Calcitriol und Calcidiol eine eigenständige, antientzündliche Wirkung auch an der unteren Vitamin D-Normgrenze von 30 ng/ml besitzt. Insbesondere vermitteln Calcitriol bzw. Calcidiol im Vergleich zur Kontrolle, d. h. von lediglich mit LPS-stimulierten humanen Monozyten ohne Inkubation von Calcitriol bzw. Calcidiol, eine Hemmung der LPS-stimulierten Produktion von TNF-alpha von 30 bzw. 82 %.

Infolge von Interaktionen von Vitamin D bzw. dessen Metaboliten mit verschiedenen, überwiegend entzündungshemmenden Leitlinientherapeutika, wird im Rahmen der vorliegenden Erfindung die Entwicklung spezieller entzündungshemmender Kombinationen, bevorzugt auf Basis von Calcidiol mit mindestens einem Leilinientherapeutikum, ermöglicht. Insbesondere sind diese neuartigen Kombinationen zur Intensivierung der Leitlinientherapie geeignet. Dieser Effekt ist insbesondere auf die überraschend festgestellte, synergistisch zunehmende Entzündungshemmung durch den zusätzlichen Einsatz von Vitamin D-Metaboliten zurückzuführen. Insgesamt erlauben die erfindungsgemäßen Therapiekonzepte die Behandlung unterschiedlicher, mit oder ohne Vitamin D-Mangel assoziierter chronisch-entzündlicher Darmerkrankungen und Atemwegserkrankungen.

Die vorliegende Erfindung wird durch die folgenden Aspekte bzw. Erfindungsgegenstände näher beschrieben, wobei für sämtliche Aspekte gilt, dass die beschriebenen Kombinationstherapeutika und Zusammensetzungen jeweils keinen Inhalts- und/oder Wirkstoff aus der Gruppe von Terpenen, Monoterpenen, Vasokonstriktoren, alpha-Sympathomimetika und/oder deren physiologisch unbedenklichen Salzen oder Derivaten enthalten. Dies gilt gleichermaßen für solche Zusammensetzungen, welche im Rahmen einer Co-Medikation und/oder Zusatztherapie mit den in Rede stehenden Kombinationstherapeutika bzw. Zusammensetzungen erfindunggemäß eingesetzt werden:

### Aspekt 1:

Vitamin D-Rezeptoragonisten (VDA), insbesondere 25-OH D₃ (Calcidiol) oder Analoga zur Prophylaxe und Therapie der Entzündungshemmung bei allergischen, chronischen, infektiösen, exazerbierten Lokal- und Systemerkrankungen, mit dem Ziel der Reduktion des Therapiebedarfs bzw. Intensivierung der Hauptwirkung von anerkannten Leitlinientherapeutika unabhängig von einem Vit. D-Mangelzustand.

### Aspekt 2:

Vitamin D-Rezeptoragonisten (VDA), insbesondere 25-OH D₃ (Calcidiol) oder Analoga bevorzugt zur prophylaktischen, lokalen, topischen und inhalativen Therapie von oberen und unteren Atemwegserkrankungen sowie zur lokalen Therapie chronisch entzündlicher Darmerkrankungen, insbesondere gelöst in dünndarmlöslichen Kapseln oder in Form von Pellets unabhängig von einem Vit. D-Mangelzustand.

### Aspekt 3:

Vitamin D-Rezeptoragonisten (VDA), insbesondere 25-OH D₃ (Calcidiol), 1,25-DOH D₃ (Calcitriol) oder deren Analoga, bevorzugt in lokaler, topischer Form bei Erkältungskrankheiten, chronisch obstruktiven Atemwegserkrankungen und chronisch entzündlichen Darmerkrankungen zur Inaktiverung oder Hemmung der Vermehrung von viralen und bakteriellen Infekterregern, insbesondere mit dem Ziel, die Virulenz, Infektiosität, chronische persistierende Entzündungsreaktion und somit den Therapiebedarf von Antibiotika, Kortikosteroiden und anderen Immunsupressiva unabhängig von Vit. D-Mangelzuständen zu reduzieren,

### Aspekt 4:

25-OH D₃ (Calcidiol) in Kombination von 1,25-DOH D₃ (Calcitriol) zur Intensivierung der Wirkungen nach Aspekt 1 bis 3 durch eine Zunahme der Entzündungshemmung und einer Wirkverstärkung durch Reduktion der 1,25-DOH D₃ vermittelten Downregulation des Vit.D3-Rezeptors.

### Aspekt 5:

Vitamin D₃ wird eingesetzt als Tablette und/oder dünndarmlösliche Kapsel (2x täglich 800 IE bis 2.000 IE), zur topisch-nasalen Therapie (50-100 µg/Hub, 1-2x täglich) oder zur Inhalation als Suspension- oder Pulverform (250 µg/Hub bzw. 250-500 µg/Pulver).

### Aspekt 6:

Verwendung von 25-OH D₃ und 1,25-DOH D₃ oder deren Analoga zur lokalen-nasalen, inhalativen oder systemischen Therapie zur Prophylaxe und Therapie von viralen und bakteriellen Erkältungskrankheiten, insbesondere auch in Kombination Vitamin C, Vitamin E und Acetylsalicylsäure oder einem anderen nicht-steroidalem Antiphlogistikum (aus der Gruppe der NSAIDS) oder von Paracetamol zur Verstärkung deren Wirksamkeit mit Besserung und Reduktion des Krankheitsverlaufs von Erkältungskrankheiten.

### Aspekt 7:

Verwendung von 25-OH D₃ oder Analoga, als Kombinationstherapeutikum, insbesondere in Form eines Kits als Nasenspray, mit nasal-topischen Glukokortikosteroiden zur Intensivierung der Steroidwirkung bei oberen Atemwegserkrankungen, insbesondere der akuten, allergischen, chronischen und chronisch rezidivierenden, polypoiden Rhinitis bzw. Rhinosinusitis.

### Aspekt 8:

Verwendung von 1,25-DOH D₃, insbesondere von 25-OH D₃, oder Analoga als Kombinationstherapeutikum, insbesondere in Form eines Kits als Tablette oder Kapsel, in Verbindung mit Leukotrienrezeptorantagonisten, insbesondere Montelukast, Pranlukast, Zaforlulkast, 5-Lipoxygenase-Hemmstoffen oder Antiallergika bzw. Antihistaminika zur Intensivierung der Leitlinientherapieempfehlungen bei allergischen Erkrankungen, insbesondere des allergischen Asthmas, bzw. der allergischen Rhinitis und Konjunktivitis, Neurodermitis und von Nahrungsmittelallergien.

### Aspekt 9:

Verwendung von 1,25-DOH D₃, insbesondere von 25-OH D₃, oder deren Analoga als Kombinationstherapeutikum, insbesondere in Form eines Kits zur Inhalation, in Verbindung mit ICS, LABAs, LAMAs, LABA+ICS sowie LA-BA+ICS+LAMA, insbesondere mit dem Ziel der Verbesserung des Therapie-effekts der topisch eingesetzten Leitlinientherapie für untere Atemwegserkrankungen.

### Aspekt 10:

Verwendung von 1,25-DOH D₃, insbesondere von 25-OH D₃, oder deren Analoga als Kombinationstherapeutikum, insbesondere in Form eines Kits als Zusatztherapie (+Vitamin D₃ 20-30.000 IE / 2 Wochen), zusammen mit einer Anti-IgE-Therapie, insbesondere mit Omalizumab zur Hemmung der Produktion von IgE-Antikörpern, vorzugsweise zum Schutz vor allergischen Sensibilisierungen bei gleichzeitiger Wirkverstärkung und besserer Verträglichkeit mit eventueller Option zur Dosisreduktion kostenaufwendiger Therapien mit Anti-IgE.

### Aspekt 11:

Verwendung nach einem der oben genannten Aspekte zur Hemmung oder Modulation COPD-assoziierter und COPD-unabhängiger Alterungsprozesse, insbesondere mit dem Ziel, die Erkrankungshäufigkeit und -schwere zu reduzieren und die Lebenserwartung und -qualität zu bessern und den gesamten Alterungsprozess zu retardieren.

### Aspekt 12:

Verwendung von 1,25-DOH D₃, insbesondere von 25-OH D₃, oder deren Analoga als Kombinationstherapeutikum, insbesondere in Form eines Kits als dünndarmlösliche Kapsel, in Verbindung mit 5-Aminosalicylsäure (Vit.D3+5-ASA), einem Glukokortikosteroid, insbesondere Budesonid (Vit.D3+Budesonid), und einem Zytokininhibitor- oder Rezeptorantagonisten, oder anderen Biologika zur Intensivierung der Leitlinientherapie von chronisch entzündlichen Darmerkrankungen (IBD) mit Befall des Dünn- und Dickdarms, insbesondere von *Morbus Crohn* (CD) und *Colitis ulcerosa* (CU), hepatischen Autoimmunerkrankungen, wie bei primärbilärer Zirrhose, zur Prophylaxe und Erhöhung der Steroidwirkung bei IBD.

### Aspekt 13:

Prophylaxe chronisch entzündlicher Darmerkrankungen durch antioxidative Agenzien, insbesondere eine Kombination von Vitamin D₃, Vitamin C, und/oder Vitamin E mit Budesonid in dünndarmlöslichen Kapseln und in Form als Nahrungsmittelzusatz.

### Aspekt 14:

Verwendung von 1,25-DOH D₃, insbesondere von 25-OH D₃, oder deren Analoga als Kombinationstherapeutikum, insbesondere in Form eines Kits als dünndarmlösliche Kapsel, in Verbindung mit 5-ASA oder Derivaten, Budesonid oder anderen Glukokortikosteroiden, zur lokalen, systemischen und synergistischen Verstärkung der Leitlinientherapie der unter Aspekt 12 genannten Darmerkrankungen mit besonderem Fokus auf die klinische Überlegenheit durch Reduktion akuter, infektiöser, allergisch oder chronisch bedingter Rezidivhäufungen mit Besserung der Therapierefraktärität durch einen neuen kausalen Ansatz zur kombinierten Behandlung der Lokal- und Systementzündung bei IBD.

## Patentansprüche

1. Kombinationstherapeutikum, insbesondere pharmazeutisches Kombinationstherapeutikum, vorzugsweise zur Verwendung bei der prophylaktischen und/oder therapeutischen Behandlung von insbesondere entzündlichen Erkrankungen des Verdauungstraktes, insbesondere des Darms,
wobei das Kombinationstherapeutikum - insbesondere in jeweils wirksamen, vorzugsweise pharmazeutisch wirksamen Mengen - umfasst:
(a) mindestens einen Vitamin D-Rezeptoragonisten (VDA) und
(b) mindestens einen weiteren Wirkstoff, wobei der weitere Wirkstoff 5-Aminosalicylsäure oder deren physiologisch unbedenkliche Salze oder Ester und gegebenenfalls mindestens ein insbesondere systemisches Kortikosteroid im Fall der prophylaktischen und/oder therapeutischen Behandlung von entzündlichen Darmerkrankungen ist;
jedoch jeweils mit der Maßgabe, dass das Kombinationstherapeutikum keinen Inhalts- und/oder Wirkstoff aus der Gruppe von Terpenen, Monoterpenen, Vasokonstriktoren, alpha-Sympathomimetika und/oder deren physiologisch unbedenklichen Salzen oder Derivaten enthält.

2. Kombinationstherapeutikum nach Anspruch 1,
wobei (a) der Vitamin D-Rezeptoragonist (VDA) ausgewählt ist aus der Gruppe von Verbindungen der Vitamin D-Gruppe, insbesondere Vitamin D₃ (Cholecalciferol) und/oder Vitamin D₂ (Ergocalciferol), vorzugsweise Vitamin D₃ (Cholecalciferol), und/oder wobei (a) der Vitamin D-Rezeptoragonist (VDA) 25-Hydroxy-Vitamin D₃ (Calcidiol) ist; und/oder
wobei (a) der Vitamin D-Rezeptoragonist (VDA) ausgewählt ist aus der Gruppe von insbesondere physiologischen Vitamin D₃-Metaboliten und/oder insbesondere physiologischen Vitamin D₃-Präkursoren, vorzugsweise aus der Gruppe von insbesondere physiologischen Vitamin D₃-Metaboliten, und/oder wobei (a) der Vitamin D-Rezeptoragonist (VDA) ausgewählt ist aus der Gruppe von insbesondere synthetischen Vitamin D₃-Analoga, insbesondere Tacalcitol; und/oder
wobei (a) der Vitamin D-Rezeptoragonist (VDA) ausgewählt ist aus der Gruppe von 25-Hydroxy-Vitamin D₃ (Calcidiol) und/oder 1,25-Dihydroxy-Vitamin D₃ (Calcitriol), vorzugsweise 25-Hydroxy-Vitamin D₃ (Calcidiol).

3. Kombinationstherapeutikum nach einem der vorangehenden Ansprüche,
wobei (a) der Vitamin D-Rezeptoragonist (VDA) eine Kombination von 25-Hydroxy-Vitamin D₃ (Calcidiol) und 1,25-Dihydroxy-Vitamin D₃ (Calcitriol) ist, insbesondere wobei das Kombinationstherapeutikum (i) 25-Hydroxy-Vitamin D₃ und (ii) 1,25-Di-hydroxy-Vitamin D₃ in einem gewichtsbezogenen Mengenverhältnis von [(i) : (ii)] im Bereich von 1.000: 1 bis 1 : 100, insbesondere im Bereich von 500 : 1 bis 1 : 10, vorzugsweise im Bereich von 100 : 1 bis 1 : 5, bevorzugt im Bereich von 50 : 1 bis 1 : 1, besonders bevorzugt im Bereich von 10 : 1 bis 1 : 1, aufweist; und/oder wobei der Vitamin D-Rezeptoragonist (VDA) 25-Hydroxy-Vitamin D₃ (Calcidiol) ist;
und/oder
wobei das Kombinationstherapeutikum (a) den Vitamin D-Rezeptoragonisten (VDA), insbesondere 25-Hydroxy-Vitamin D₃ (Calcidiol) und/oder 1,25-Dihydroxy-Vitamin D₃ (Calcitriol), in einer Menge im Bereich von 0,0001 Gew.-% bis 5 Gew.-%, insbesondere im Bereich von 0,0005 Gew.-% bis 2 Gew.-%, vorzugsweise im Bereich von 0,001 Gew.-% bis 1,5 Gew.-%, bevorzugt im Bereich von 0,005 Gew.-% bis 1 Gew.-%, besonders bevorzugt im Bereich von 0,01 Gew.-% bis 0,75 Gew.-%, ganz besonders bevorzugt im Bereich von 0,05 Gew.-% bis 0,5 Gew.-%, bezogen auf das Kombinationstherapeutikum, enthält; und/oder
wobei das Kombinationstherapeutikum (a) den Vitamin D-Rezeptoragonisten (VDA), insbesondere 25-Hydroxy-Vitamin D₃ (Calcidiol) und/oder 1,25-Dihydroxy-Vitamin D₃ (Calcitriol), in einer Menge im Bereich von 0,025 µg bis 400 µg, insbesondere im Bereich von 0,05 µg bis 500 µg, vorzugsweise im Bereich von 0,1 µg bis 300 µg, bevorzugt im Bereich von 1 µg bis 200 ug, besonders bevorzugt im Bereich von 10 µg bis 100 µg, bezogen auf eine Dosier- und/oder Applikationseinheit des Kombinationstherapeutikums, enthält; und/oder
wobei das Kombinationstherapeutikum mindestens einen mit (a) dem Vitamin D-Rezeptoragonisten (VDA) mischbaren und/oder hierin löslichen, insbesondere bei 20 °C und Atmosphärendruck flüssigen, physiologisch unbedenklichen Träger (Exzipienten) enthält.

4. Kombinationstherapeutikum nach einem der vorangehenden Ansprüche,
wobei (b) 5-Aminosalicylsäure in Form ihrer physiologisch unbedenklichen Alkali- und/oder Erdalkalisalze, vorzugsweise in Form des Natriumsalzes von 5-Aminosalicysäure und/oder Natrium-5-Aminosalicylat, eingesetzt ist; und/oder
wobei das Kombinationstherapeutikum (b) 5-Aminosalicylsäure in einer Menge im Bereich von 0,001 Gew.-% bis 50 Gew.-%, insbesondere im Bereich von 0,01 Gew.-% bis 20 Gew.-%, vorzugsweise im Bereich von 0,05 Gew.-% bis 10 Gew.-%, bevorzugt im Bereich von 0,1 Gew.-% bis 5 Gew.-%, bezogen auf das Kombinationstherapeutikum, enthält.

5. Kombinationstherapeutikum nach einem der vorangehenden Ansprüche, wobei das Kombinationstherapeutikum mindestens einen weiteren pharmakologisch wirksamen Inhaltsstoff und/oder Wirkstoff umfasst, wobei der pharmakologisch wirksame Inhaltsstoff und/oder Wirkstoff ausgewählt ist aus der Gruppe von Antiphlogistika, insbesondere nichtsteroidalen Antiphlogistika, vorzugsweise Acetylsalicylsäure und/oder Paracetamol; beta-Sympathomimetika, vorzugsweise beta-2-Sympathomimetika; Parasympatholytika und/oder Vagolytika; und Anticholinergika; sowie Mischungen und Kombinationen der vorgenannten Verbindungen.

6. Kombinationstherapeutikum nach einem der vorangehenden Ansprüche,
wobei (b) das systemische Kortikosteroid ein Glukokortikoid, insbesondere Glukokortikosteroid, ist, vorzugsweise ausgewählt aus der Gruppe von Dexamethason, Prednison, Prednisolon, Betamethason, Rimexolon, Paramethason, Hydrocortison und deren physiologisch verträglichen Derivaten, insbesondere Salzen und Estern, sowie Mischungen und Kombinationen der vorgenannten Verbindungen; und/oder
wobei das Kombinationstherapeutikum (b) das systemische Kortikosteroid in einer Menge im Bereich von 0,001 Gew.-% bis 10 Gew.-%, insbesondere im Bereich von 0,01 Gew.-% bis 5 Gew.-%, vorzugsweise im Bereich von 0,1 Gew.-% bis 2 Gew.-%, bezogen auf das Kombinationstherapeutikum, enthält; und/oder
wobei das Kombinationstherapeutikum mindestens ein weiteres Vitamin, insbesondere ausgewählt aus der Gruppe von Vitamin A, Vitamin C und Vitamin E, insbesondere Vitamin C und Vitamin E, enthält; und/oder
wobei das Kombinationstherapeutikum mindestens einen weiteren Inhaltsstoff aufweist, insbesondere ausgewählt aus der Gruppe von Verarbeitungshilfsstoffen, Stabilisatoren, insbesondere organischen Lösemitteln, Emulgatoren, Antioxidantien, Feuchthaltemitteln, Verdickungsmitteln, Antiseptika, Farbstoffen, Aromastoffen, Riechstoffen, Duftstoffen, Streckmitteln, Bindemitteln, Netzmitteln, Vitaminen, Spurenelementen, Mineralstoffen, Mikronährstoffen und/oder ätherischen Ölen sowie deren Kombinationen.

7. Kombinationstherapeutikum nach einem der vorangehenden Ansprüche, wobei das Kombinationstherapeutikum topisch applizierbar ist und/oder appliziert wird oder wobei das Kombinationstherapeutikum systemisch, insbesondere peroral applizierbar ist und/oder appliziert wird; und/oder
wobei das Kombinationstherapeutikum in Tagesdosen von (a) 10 µg bis 1.000 µg, insbesondere 20 µg bis 800 µg, vorzugsweise 30 µg bis 500 µg, Vitamin D-Rezeptoragonist (VDA) verabreicht wird und/oder wobei das Kombinationstherapeutikum zur Verabreichung in einer Tagesdosis von (a) 10 µg bis 1.000 µg, insbesondere 20 µg bis 800 µg, vorzugsweise 30 µg bis 500 µg, Vitamin D-Rezeptoragonist (VDA) hergerichtet ist; und/oder
wobei das Kombinationstherapeutikum in Tagesdosen von (b) 50 bis 10.000 mg, insbesondere 75 bis 6.000 mg, besonders bevorzugt 100 bis 2.000 mg, 5-Aminosalicylsäure verabreicht wird und/oder insbesondere wobei das Kombinationstherapeutikums zur Verabreichung einer Tagesdosis von (b) 50 bis 10.000 mg, insbesondere 75 bis 6.000 mg, besonders bevorzugt 100 bis 2.000 mg, 5-Aminosalicylsäure hergerichtet ist; und/oder
wobei das Kombinationstherapeutikum in Tagesdosen von (b) 50 bis 1.500 µg, insbesondere 75 bis 1.000 µg, besonders bevorzugt 100 bis 800 µg, insbesondere systemisches Kortikosteroid verabreicht wird und/oder wobei das Kombinationstherapeutikum zur Verabreichung in einer Tagesdosis von (b) 50 bis 1.500 µg, insbesondere 75 bis 1.000 µg, besonders bevorzugt 100 bis 800 µg, insbesondere systemisches Kortikosteroid hergerichtet ist.

8. Kombinationstherapeutikum nach einem der vorangehenden Ansprüche,
wobei das Kombinationstherapeutikum in einer peroral, insbesondere inhalativ applizierbaren Form, vorzugsweise als Suspension und/oder in Pulverform, vorliegt;
oder
wobei das Kombinationstherapeutikum in einer peroral applizierbaren Darreichungsform, insbesondere in Form von Kapseln, Pellets und/oder Tabletten, vorzugsweise in Form von Kapseln oder Pellets, bevorzugt Kapseln, vorliegt und/oder wobei das Kombinationstherapeutikum in Form einer peroralen, magensaftresistenten, aber dünndarmlöslichen Darreichungsform, insbesondere Kapseln und/oder Pellets, bevorzugt Kapseln, vorliegt.

9. Kombinationstherapeutikum nach einem der vorangehenden Ansprüche zur prophylaktischen und/oder therapeutischen Behandlung, insbesondere Kombinationstherapie und/oder Co-Medikation, von insbesondere entzündlichen Erkrankungen des Verdauungstrakts, insbesondere des Darms.

10. Kombinationstherapeutikum nach einem der vorangehenden Ansprüche,
wobei das Kombinationstherapeutikum (a) den Vitamin D-Rezeptoragonisten (VDA) einerseits und (b) den weiteren Wirkstoff andererseits, gegebenenfalls gemeinsam mit dem weiteren pharmakologisch wirksamen Inhaltsstoff und/oder Wirkstoff, wie in Anspruch 5 definiert, und/oder dem weiteren Vitamin und/oder dem weiteren Inhaltsstoff, in einer gemeinsamen und/oder einzigen Zusammensetzung aufweist;
und/oder
wobei das Kombinationstherapeutikum, bevorzugt zur Verwendung bei der prophylaktischen und/oder therapeutischen Behandlung von entzündlichen Darmerkrankungen, umfassend - insbesondere in jeweils wirksamen, vorzugsweise pharmazeutisch wirksamen Mengen - mindestens einen Vitamin D-Rezeptoragonisten (VDA) einerseits und mindestens einen weiteren Wirkstoff andererseits, wobei der weitere Wirkstoff 5-Aminosalicylsäure oder deren physiologisch unbedenkliche Salze oder Ester und gegebenenfalls mindestens ein insbesondere systemisches Kortikosteroid ist, wobei das Kombinationstherapeutikum in Form eines Kits ("*Kit of Parts*") vorliegt und/oder wobei das Kombinationstherapeutikum (a) den Vitamin D-Rezeptoragonisten (VDA) einerseits und (b) den weiteren Wirkstoff andererseits räumlich voneinander getrennt, insbesondere in voneinander verschiedenen Zusammensetzungen, aufweist,
jedoch mit der Maßgabe, dass das Kombinationstherapeutikum keinen Inhalts- und/oder Wirkstoff aus der Gruppe von Terpenen, Monoterpenen, Vasokonstriktoren, alpha-Sympathomimetika und/oder deren physiologisch unbedenklichen Salzen oder Derivaten enthält.

11. Verwendung eines Kombinationstherapeutikums, insbesondere nach einem der vorangehenden Ansprüche, zur prophylaktischen und/oder therapeutischen Behandlung von insbesondere entzündlichen Erkrankungen des Verdauungstraktes, insbesondere des Darms, und/oder Verwendung eines Kombinationstherapeutikums nach einem der vorangehenden Ansprüche zur Herstellung eines Arzneimittels zur prophylaktischen und/oder therapeutischen Behandlung von insbesondere entzündlichen Erkrankungen des Verdauungstraktes, insbesondere des Darms,
wobei das Kombinationstherapeutikum - insbesondere in jeweils wirksamen, vorzugsweise pharmazeutisch wirksamen Mengen - mindestens einen Vitamin D-Rezeptoragonisten (VDA) einerseits und mindestens einen weiteren Wirkstoff andererseits, wobei der weitere Wirkstoff 5-Aminosalicylsäure oder deren physiologisch unbedenkliche Salze oder Ester und gegebenenfalls mindestens ein insbesondere systemisches Kortikosteroid ist, umfasst,
jedoch mit der Maßgabe, dass das Kombinationstherapeutikum keinen Inhalts- und/oder Wirkstoff aus der Gruppe von Terpenen, Monoterpenen, Vasokonstriktoren, alpha-Sympathomimetika und/oder deren physiologisch unbedenklichen Salzen oder Derivaten enthält.

12. Verwendung eines Vitamin D-Rezeptoragonisten (VDA) zur Herstellung eines Arzneimittels zur prophylaktischen und/oder therapeutischen Behandlung von insbesondere entzündlichen Erkrankungen des Verdauungstraktes, insbesondere des Darms, insbesondere wobei der Vitamin D-Rezeptoragonist (VDA) zusammen mit mindestens einem weiteren Wirkstoff eingesetzt wird, wobei der weitere Wirkstoff 5-Aminosalicylsäure oder deren physiologisch unbedenkliche Salze oder Ester und gegebenenfalls mindestens ein insbesondere systemisches Kortikosteroid im Fall der prophylaktischen und/oder therapeutischen Behandlung von (B) entzündlichen Darmerkrankungen ist,
jedoch mit der Maßgabe, dass das Kombinationstherapeutikum keinen Inhalts- und/oder Wirkstoff aus der Gruppe von Terpenen, Monoterpenen, Vasokonstriktoren, alpha-Sympathomimetika und/oder deren physiologisch unbedenklichen Salzen oder Derivaten enthält.

13. Verwendung eines Kombinationstherapeutikums, insbesondere nach einem der vorangehenden Ansprüche, und mindestens eines weiteren Therapeutikums zur prophylaktischen und/oder therapeutischen Behandlung, insbesondere Kombinationstherapie und/oder Co-Medikation, von insbesondere entzündlichen Erkrankungen des Verdauungstraktes, insbesondere des Darms,
wobei das Kombinationstherapeutikum - insbesondere in jeweils wirksamen, vorzugsweise pharmazeutisch wirksamen Mengen - umfasst:
(a) mindestens einen Vitamin D-Rezeptoragonisten (VDA) und
(b) mindestens einen weiteren Wirkstoff, wobei der weitere Wirkstoff 5-Aminosalicylsäure oder deren physiologisch unbedenkliche Salze oder Ester und gegebenenfalls mindestens ein insbesondere systemisches Kortikosteroid im Fall der prophylaktischen und/oder therapeutischen Behandlung von entzündlichen Darmerkrankungen ist;
jedoch jeweils mit der Maßgabe, dass das Kombinationstherapeutikum keinen Inhalts- und/oder Wirkstoff aus der Gruppe von Terpenen, Monoterpenen, Vasokonstriktoren, alpha-Sympathomimetika und/oder deren physiologisch unbedenklichen Salzen oder Derivaten enthält.

14. Verwendung nach einem der Ansprüche 11 bis 13,
wobei zusätzlich mindestens ein weiterer systemischer, insbesondere peroraler Wirkstoff appliziert wird, insbesondere ausgewählt aus der Gruppe von systemischen Phosphodiesterasehemmern, insbesondere Theophyllin; systemischen Leukotrienrezeptorantagonisten, insbesondere Montelukast, Zaforlukast und Pranlukast; systemischen Kortikosteroiden; Antiphlogistika, insbesondere nichtsteroidalen Antiphlogistika, vorzugsweise Acetylsalicylsäure und/oder Paracetamol; sowie deren Mischungen und Kombinationen; und/oder
wobei das weitere Therapeutikum, insbesondere im Fall der prophylaktischen und/oder therapeutischen Behandlung von entzündlichen Darmerkrankungen, ein systemisch applizierbares Therapeutikum, insbesondere ein peroral applizierbares Therapeutikum, ist, insbesondere wobei das Therapeutikum mindestens ein systemisch applizierbares, vorzugsweise peroral applizierbares Kortikosteroid, insbesondere Glukokortikosteroid, aufweist, insbesondere wobei das Glukokortikosteroid ausgewählt ist aus der Gruppe von Dexamethason, Prednison, Prednisolon, Betamethason, Rimexolon, Paramethason, Hydrocortison und deren physiologisch verträglichen Derivaten, insbesondere Salzen und Estern, sowie Mischungen und Kombinationen der vorgenannten Verbindungen.

15. Kombinationstherapeutikum nach einem der Ansprüche 1 bis 10 oder Verwendung nach einem der Ansprüche 11 bis 14, wobei die insbesondere entzündliche Erkrankung des Verdauungstraktes, insbesondere des Darms, *Morhus Crohn* und/oder *Colitis ulcerosa* und/oder eine chronisch entzündliche Darmerkrankung (*Inflammatory Bowel Disease,* IBD) ist.
